# EUROPEAN PATENT SPECIFICATION

(11) **EP 2 877 455 B1**
(45) Date of publication and mention of the grant of the patent: **17.08.2016**
(21) Application number: 13747989.5
(22) Date of filing: 23.07.2013
(51) Int. Cl.: C07D 221/18, C07D 231/12, C07D 231/56, C07D 239/26, C07D 239/72, C07D 311/82, C07D 333/10, A61K 31/473, A61K 31/517, A61K 31/352, A61K 31/381, A61K 31/4433, A61K 31/506, A61K 31/416, A61K 31/4155

(54) **FUSED QUINONIC COMPOUNDS**
KONDENSIERTE CHINONVERBINDUNGEN
COMPOSÉS QUINONIQUES FUSIONNÉS

(30) Priority: 24.07.2012 EP 12177591; 24.07.2012 US 201261675073 P
(43) Date of publication of application: 03.06.2015
(73) Proprietor: Centro Atlantico del Medicamento S.A (Ceamed, S.A), 38204 Santa Cruz de Tenerife (ES)
(72) Inventor: McNAUGHTON SMITH, Grant, E-38204 Santa Cruz de Tenerife (ES); ESTÉVEZ BRAUN, Ana, E-38206 Santa Cruz de Tenerife (ES); JIMÉNEZ ALONSO, Sandra, E-38206 Santa Cruz de Tenerife (ES); GUTIÉRREZ RAVELO, Ángel Domingo, E-38206 Santa Cruz de Tenerife (ES); FERNÁNDEZ PÉREZ, Leandro, E-35016 Las Palmas de Gran Canaria (ES); DÍAZ CHICO, Bonifacio Nicolás, E-35016 Las Palmas de Gran Canaria (ES)
(74) Representative: Illescas, Manuel
(86) International application number: PCT/EP2013/065552
(87) International publication number: WO 2014/016314

(56) References cited:
- CN-A- 102 276 564
- HAMDAN, ABDULLA J. ET AL: "A novel synthetic route to heterocyclic quinones", JOURNAL OF ORGANIC CHEMISTRY , 50(18), 3427-8 CODEN: JOCEAH; ISSN: 0022-3263, 1985, XP002688262,
- DATABASE WPI Week 201114 Thomson Scientific, London, GB; AN 2010-N13491 XP002688263, & CN 101 829 108 A (CHINESE ACAD SCI SHANGHAI INST MATERIA) 15 September 2010 (2010-09-15)
- DU, BAIXIANG ET AL: "Efficient one-pot three-component synthesis of 3,4-dihydro-12-phenyl-2H-benzo[b] xanthene-1,6,11(12H)-trione derivatives in ionic liquid", RESEARCH ON CHEMICAL INTERMEDIATES AHEAD OF PRINT CODEN: RCINEE; ISSN: 0922-6168, 29 June 2012 (2012-06-29), XP002688261,
- MINOO DABIRI ET AL: "An efficient three-component synthesis of benzoxanthenes in water", JOURNAL OF HETEROCYCLIC CHEMISTRY, vol. 47, no. 5, 1 September 2010 (2010-09-01), pages 1062-1065, XP055045904, ISSN: 0022-152X, DOI: 10.1002/jhet.420

## Description

### Field of the Invention

The present invention relates to fused quinionic compounds. Additionally, the present invention relates to fused quinonic compounds for use in treating diseases. Furthermore, the present invention relates to a composition comprising a fused quinonic compound and at least one pharmaceutically acceptable carrier, as well as the use of the compounds in a method of modulating a Janus Kinase-Signal Transduction and Activators of Transcription (JAK-STAT) signaling pathway. JAK-STAT signaling pathwaysare associated with diseases such as immunological and inflammatory diseases, hyperproliferative disorders including cancer, and myeloproliferative diseases.

### Background of the Invention

The JAK-STAT signaling cascades are one of several kinase-mediated pathways by which an extracellular stimulus can be transferred into a nuclear response (for a more detailed discussion of the JAK-STAT pathway, see Murray PJ., "The JAK-STAT signaling pathway: input and output integration" J. Immunol, 178:2623, 2007). In brief, the extracellular stimulus (commonly known as the ligand) for the JAK-STAT pathway, which is normally a cytokine, growth factor or hormone, first binds to a specific receptor to which is bound one of the JAK's in an inactivated state. Once the extracellular stimulus is bound, the receptor forms a dimeric structure which leads to the activation of the bound JAK's. This activation leads to the site-specific phosphorylation of the cytoplasmic part of each receptor, which then attracts, and binds specific STAT molecules. Once bound to the receptor, the STAT becomes site-specifically phosphorylated leading to its release from the receptor. Cytoplasmic phosphorylated STAT molecules dimerize and migrate to the nucleus whereupon they bind to specific DNA regions that promote gene transcription. In the case of the JAK-STAT pathway this promotion of gene transcription leads to, amongst other things, cellular proliferation (Scott, Godshall et al., "JAKs STATs, Cytokines, and Sepsis." Clin.Diagn. Lab. Immunol., 9(6):1153-9, 2002).

Currently, there are four known mammalian JAK family members: JAK1 (also known as Janus kinase-1), JAK2 (also known as Janus kinase-2), JAK3 (also known as Janus kinase-3, L-JAK and JAKL) and TYK2 (also known as protein-tyrosine kinase 2). For a brief review of the Janus kinase family see Yamaoka, K. et al., "The Janus kinases", Genome Biology, 5:253, 2004. The JAK proteins range from 120 to 140 kDa and share a complex multi-domain structure, unique among protein tyrosine kinases, characterized by seven distinct domains termed the JAK homology (JH1-JH7) domains. A unique feature of the JAKs is the presence of two similar but non-identical domains (JH1 and JH2) at the C-terminus. While the JH1 domian comprises the highly conserved protein tyrosine kinase (PTK) domain that is critically important for its physiological function, the JH2 domain, also called the pseudokinase domain or kinase-like domain, has no catalytic activity but plays a crucial role in the regulation of the PTK domain. Although the JH3-JH4 region shares some homology with Src homology 2 domains, it has no phosphotyrosine-binding capability and seems to play a structural role in stabilizing the conformation of the JAKs. The N-terminus (JH5-JH7) comprises an FERM domain, which is known to be critical for receptor binding and appears to be essential for the overall regulation of the JAK proteins (see Williams. N. K., et al., J. Mol. Biol, 387, 219-232, 2009 and references therein).While JAK1, JAK2 and TYK2 are ubiquitously expressed; JAK3 is reported to be preferentially expressed in lymphocytes.

The family of Janus kinases plays a central role in certain cytokine dependent pathways that regulate cellular proliferation. Table 1 highlights the relationship between several cytokines and their ability to selectively activate certain JAK-STAT pathways.

**Table 1: Cytokine mediated activation of JAKs and STATs.**

| **Cytokine** | **Activated Janus Kinase** | **Activated STAT** |
|---|---|---|
| IL-2 | JAK1, JAK2, JAK3 | STAT3, STAT5 |
| IL-3 | JAK2 | STAT3, STAT5 |
| IL-4 | JAK1, JAK3 | STAT6 |
| IL-5 | JAK2 | STAT1, STAT3, STAT5, |
| IL-6 | JAK1, JAK2, TYK2 | STAT1, STAT3 |
| IL-7 | JAK1, JAK3 | STAT3, STAT5 |
| IL-9 | JAK3 | STAT3, STAT5 |
| IL-10 | JAK1, TYK2 | STAT1, STAT3 |
| IL-11 | JAK1, JAK2, TYK2 | STAT3 |
| IL-12 | JAK2, TYK2 | STAT4 |
| IL-13 | JAK1, JAK2, TYK2 | STAT6 |
| IL-15 | JAK1, JAK3 | STAT3, STAT5 |
| IFNalpha/beta | JAK1, TYK2 | STAT1, STAT2 |
| IFNgamma | JAK1, JAK2 | STAT1 |
| EPO | JAK2 | STAT5 |
| TPO | JAK2, TYK2 | STAT5 |
| G-CSF | JAK2, JAK3 | STAT3 |
| GH | JAK2 | STAT3, STAT5 |
| PRL | JAK2 | STAT5 |
| Leptin | JAK2 | STAT3, STAT5, STAT6 |
| GM-CSF | JAK2 | STAT5 |
| CNTF | JAK1, JAK2, TYK2 | STAT3 |
| CT-1 | JAK1, JAK2, TYK2 | STAT3 |
| LIF | JAK1, JAK2, TYK2 | STAT3 |
| OSM | JAK1, JAK2, TYK2 | STAT3 |
| EGF | JAK1 | STAT1, STAT3, STAT5 |
| PDGF | JAK1 | STAT1, STAT3, STAT5 |
| Insulin | JAK2 | STAT1, STAT5 |

A review of the JAK-STAT literature offers strong support to the hypothesis that this pathway is important for the recruitment and marshalling of the host immune response to environmental insults, such as viral and bacterial infection. Information accumulated from gene knock-out experiments have highlighted the importance of members of the JAK family to the intracellular signaling triggered by a number of important immune regulatory cytokines (Igaz, P., Toth, S. and Falus, A. "Biological and clinical significance of the JAK-STAT pathway; lessons from knock-out mice." Inflam.Res. 50, 435-441, 2001.)

Mutations and translocations of the JAK genes that result in constitutively active JAK proteins are associated with a variety of hematopoietic malignancies, including auto-immune diseases, myeloproliferative syndromes, leukemias and lymphomas, as well as cardiovascular disease. Moreover, a single mutation in the kinase-like (JH2) domain of JAK2 (V617F) seems to underpin a range of myleoproliferative diseases, such as polycythemia vera (PV), essential thromobocythemia (ET) and chronic idiopatic myleofibrosis. For a detailed discussion about the JAK2 (V617F) mutation see Staerk, J., et al., Path. Biol., 55(2), 88-91, 2007.Similarly, several JAK1 mutations have been associated with T-cell precursor acute lymphoblastic leukemia and acute myeloid leukemia.

**Table 2: Reported involvement of JAKs in human diseases (disorders).**

| **Disease or disorder** | **Janus Kinase involved** | **Literature reference** |
|---|---|---|
| Serve combined immunodeficiency (SCID) | JAK3 | Candotti, F., Oakes, S. A., et al., Blood, 90(19): 3996-4003, 1997. |
| Asthma | JAK1 | Perris, A. B and Rothman, P. B. J Clin Invest.109(10):1279-1283, 2002. Seto, Y., Nakalima, H. et al., J Immunol. 170(2):1077-83, 2003. Malaviya, et al., JPET., 295, 912, 2000. Kudlacz, et al., European J. Pharm., 582, 154, 2008. |
| | JAK2 | |
| | JAK3 | |
| | TYK2 | |
| Endometrial cancer Cervical Cancer | JAK2 | Chen, C.L. et al., British Journal of Cancer, 96: 591-599,2007. |
| | JAK3 | |
| Prostate cancer | JAK1 | Chang, Y-M., Kung, H-J., Evans, C. P., Neoplasia, 9(2), 90-100, 2007. |
| | JAK2 | |
| Adult T cell leukemia/lymphoma | JAK3 | Takemoto, S., Mulloy, J. C., et al., Proc. Natl. Acad. Sci., USA 94(25): 13897-902, 1997. |
| Mutliple Myeloma | JAK2 | De Vos, J., Jourdan, M., et al., Br J Haematol.109(4): 823-828, 2000; Li, J., et al., Neoplasia, 12(1), 28-38, 2010. |
| Graft versus host disease (GVHD) | JAK3 | Saemann, M. D., Diakos, C., et al., Am J Transplant, 3(11): 1341-9, 2003; Changelian, P. S., et al., Science, 302, 875-878, 2003. |
| Myeloproliferative disorder (including Polycythemia Vera, Essential thromobocythemia and chronic idiopatic myleofibrosis) | JAK2 | Levine, et al., Cancer Cell, (7):387-397, 2005; Levine, et al., Nat. Rev. Cancer, 7, 673-683, 2007; Scott, L. M., et al., N. Engl. J. Med. 356: 459-468, 2007; Kralovics, R., et al., Eng. J. Med. 352, 1779, 2005; James C., et al., Nature, 434, 1144-1148, 2005; Baxter, E. J., et al., Lancet, 365, 1054, 2005; Zhoa, R., J. Bio. Chem. 280, 22788, 2005; Tono, C., et al., Leukemia, 19, 1843, 2005; Tefferi, A., et al., Br. J. Haematol., 131, 320, 2005; Shannon, K and Van Etten, R. A., Cancer Cell, 7, 291-293, 2005. James C., et al., Trends Mol. Med. 11, 546-554, 2005; Hood, J. et al., J. Clin. Oncol, 25:18S, 7031, 2007. Pardanani, A. Leukemia, 22: 23-30, 2008. |
| Lymphoblastic leukemia | JAK1 | Flex, E., et al., J. Exp. Med., 205, 751-758, 2008; Meydan, et al., Nature, 379, 645, 1996. |
| | JAK2 | |
| Myeloid leukemia | JAK1 | Tefferi, A., Leukemia & Lymphoma, 49(3), 388-397, 2008; Jeong, E. G., et al., Clin.Cancer Res., 14, 3716-3721, 2008; Xiang, Z., et al., Blood, 111, 4809-4812, 2008. |
| | JAK2 | |
| Rheumatoid arthritis | JAK3 | Deon, D., et al. J. Immunol. 167, 5395-5403, 2001; Walker, J. G and Smith, M. D., J. Rheumatol. Sep:32(9), 1650-1653, 2005; Walker, J. G., et al., Ann. Rheum. Dis., 66, 992-999, 2007. |
| Cardiovascular disease | JAK1 | Booz, G. W., Mol. Cell. Biochem. 240, 39-46, 2002. |
| | JAK2 | |
| | JAK3 | |
| | TYK2 | |
| Multiple Sclerosis | JAK1 | Imitola, J., Chitnis, T and Khoury, S. J., Pharmacol. Ther. 106(2), 163-177, 2005. |
| | JAK2 | |
| | JAK3 | |
| Psoriasis | JAK3 | Chang, et al., JAK3 inhibition significantly attenuates psoriasiform skin inflammation on CD18 mutant PL/J mice. J. Immunol, 183, 2183, 2009. |

Currently there are seven known members of the STAT family, namely, STAT1, STAT2, STAT3, STAT4, STAT5a, STAT5b and STAT6 (for more information about the STAT family see, Darnell, JE., Science, 277, 1630-1635, 1997). In normal cells, STAT signaling is usually transient and tightly regulated by extracellular stimuli such as cytokines, growth factors and hormones predominantly via kinase mediated phosphorylation mechanisms. However, constitutive activation of some STATs, including STAT3 and STAT5, has been detected in a large number of diverse human cancer cells and primary tumor tissues, including blood malignancies and solid tumors (Yu, H., et al., "The STATs of cancer-new molecular targets come of age", Nat. Rev. Cancer, 4, 97-105, 2004; Buettner, R., "Activated STAT signaling in human tumors provides novel molecular targets for therapeutic intervention." Clin. Cancer Res., 8, 945-954, 2002). Overwhelming evidence indicates that hyperactivation of STAT3 signaling leads to enhanced tumor cell proliferation and survival (Bowman, T., "STATs in oncogenesis." Oncogene, 19, 2474-2488, 2000).Moreover, STAT3 itself has been identified as an oncoprotein (Bromberg, JF., "STAT3 as an oncogene" Cell, 98, 295-303, 1999).Constitutive STAT3 has also been shown to mediate the abnormal growth and survival of rheumatoid arthritis (RA) synoviocytes (Ivashkiv and Hu, "The JAK-STAT pathway in rheumatoid arthritis: pathogenic or protective?" Arth & Rheum., 48, 2092, 2003).Activation of STAT3 has been reported for endometrial and cervical cancers (Chen, C.L. et al., Br. J. Cancer, 96: 591-599, 2007).

**Table 3: Reported involvement of STATs in human diseases (disorders).**

| **Disease or disorder** | **STAT involved** | **Literature reference** |
|---|---|---|
| Rheumatoid Arthritis | STAT3 | Ivashkiv and Hu, Arth & Rheum., 48, 2092, 2003. |
| Crohn's disease | STAT3 | Lovato, et al., J.Biol. Chem., 278, 16777, 2003. |
| Encephalomyelitis | STAT1 | Skarica, et al., J. Immunol., 182, 4192, 2009. |
| | STAT3 | |
| | STAT5 | |
| Solid and Haematological tumors | STAT3 | Siddiquee, et al., Cell Res., 18, 254, 2008; Yu, H., et al., Nat. Rev. Cancer, 4, 97-105, 2004; Buettner, R., et al., Clin. Cancer Res., 8, 945-954, 2002 |

Cytokines of the interleukin 6 (IL-6) family, which activate the signal transducer gp130, are major survival and growth factors for human multiple myeloma (MM) cells. The signal transduction of gp130 is believed to involve JAK1, JAK2 and TYK2 and the downstream effectors STAT3 and the mitogen-activated protein kinase (MAPK) pathways.

JAK-STAT pathways have also been implicated in leukemia and lymphomas. An association between JAK3 and STAT1, STAT3, and STAT5 activation and cell-cycle progression was demonstrated in four patients with adult T cell leukemia/lymphoma (ATLL). These results imply that JAK-STAT activation is associated with replication of leukemic cells (Takemoto, S., Mulloy, J. C., et al., "Proliferation of adult T cell leukemia/lymphoma cells is associated with the constitutive activation of JAK-STAT proteins." Proc Natl Acad Sci USA 94(25): 13897-902, 1997.)

JAK2-mediated JAK-STAT signaling pathways have, in addition to the above, also been implicated in vascular diseases such as hypertension, hypertrophy, cardiac ischemia, heart failure, migrane, Raynaud's disease, pulmonary arterial hypertension. The literature also supports that JAK2-mediated JAK-STAT signalingis implicated in myeloproliferative disorders (MPDs) such as polycythemia Vera (PV), essential thrombocythemia (ET), myeloid metaplasia with myelofibrosis (MMM), hyperosinophile syndrome (HES), idiopathic myelofibrosis (IMF), or systemic mast cell disease (SMCD). Both JAK1- and JAK2-mediated JAK-STAT signaling pathways have been implicated in cancers including multiple myeloma,prostate, breast and lung cancers, gastric cancer, Hodgkin's Lymphoma, B-cell Chronic Lymphocytic Leukemia, gliomas and hepatomas.

Activation of one or more JAK-STAT pathways in cancers may occur by multiple mechanisms including cytokine stimulation or by a reduction in the endogenous suppressors of JAK signaling, such as SOCS (suppressors of cytokine signaling) or PIAS (protein inhibitor of activated STAT) (Boudny, V and Kovarik, J., Neoplasm. 49:349-355, 2002). Furthermore, activation of STAT signaling, as well as other pathways downstream of JAKs, has been correlated with poor prognosis in many types of cancers (Bowman, T., et al., Oncogene 19;2474-2488, 2000).

### Summary of the Invention

Departing from the prior art, it is the problem of the present invention to provide a compound which exhibits activity in modulating the JAK, the STAT and/or the JAK-STAT signaling pathways.

It is a further problem of the present invention to provide a compound which exhibits inhibition of said JAK, STAT and/or JAK-STAT signaling pathways by:
- inhibiting the phosphorylation of one or more JAKs;
- inhibiting the phosphorylation of one or more STATs;
- inhibiting the transcription of one or more STATs;
- inhibiting the dimerization of one or more STATs;
- inhibiting the translocation of a STAT dimer to the nucleus;
- inhibiting the binding of a STAT dimer to nuclear DNA;
- reducing the cytosolic concentrations of one or more JAKs; or
- reducing the cytosolic concentrations of one or more STATs.

It is a further problem of the present invention to provide a compound which exhibits a reduction in viability (survival and proliferation) and/or an increase in apoptosis of cells associated with a disease mediated by a JAK, STAT and/or JAK-STAT signaling pathway, such that said compound provides a therapeutic benefit to a patient.

These problems are solved according to the present invention by a compound having the formula (I): 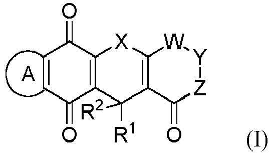 or a pharmaceutically acceptable salt thereof, wherein:
Ring A is independently selected from the group consisting of substituted or unsubstituted (C₃-C₈)cycloalkyl, substituted or unsubstituted aryl, substituted or unsubstituted heteroaryl, and substituted or unsubstituted heterocyclyl having up to 3 heteroatoms selected from N, O, S, SO and SO₂;
X is independently selected from the group consisting of O, NR³, S and SO₂; Wherein,
   R³ is independently selected from the group consisting of H, CN, SO₂R⁴, C(=O)OR⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂, wherein;
   R⁴ is independently selected from the group consisting of substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂;
   R⁵ and R⁶ are independently selected from the group consisting of H, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂;
      or R⁵ and R⁶, when both connected to the same N, form a 4-, 5-, 6- or 7-membered heterocycloalkyl group or heteroaryl group, each optionally substituted with 0, 1, 2 or 3 substitutents independently selected from OH, CN, halogen, CF₃, (C₁-C₆)alkyl, (C₂-C₆)alkenyl, (C₂-C₆)alkynyl, (C₁-C₆)alkoxy, (C₃-C₈)cycloalkyl, aryl or heteroaryl;
      R⁷ is independently selected from the group consisting of H, CN, substituted or unsubstituted (C₁-C₆)alkyl, substituted or unsubstituted (C₁-C₆)alkoxy, substituted or unsubstituted (C₃-C₈)cycloalkyl, substituted or unsubstituted aryl, substituted or unsubstituted heteroaryl;
   W and Z are independently selected from the group consisting of (CR⁸R⁹)ₙ, O and NR³;
   Y is independently selected from the group consisting of (CR⁸R⁹)ₙ and C(=O), wherein;
      n is an integer from 0 to 2; and
      R⁸ and R⁹ are independently selected from the group consisting of H, CN, substituted or unsubstituted (C₁-C₆)alkyl, substituted or unsubstituted (C₂-C₆)alkenyl, substituted or unsubstituted (C₂-C₆)alkynyl, substituted or unsubstituted C₁-C₆alkoxy, substituted or unsubstituted (C₃-C₈)cycloalkyl, substituted or unsubstituted aryl, substituted or unsubstituted heteroaryl;
      R¹ is independently selected from the group consisting of unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂;
      R² is H;
      with the provisos that when ring A is unsubstituted phenyl, X = O, W and Z = CH₂, and Y = C(CH₃)₂ then R¹ is other than phenyl, 4-nitrophenyl, 4-chlorophenyl, 3-chlorophenyl, 3-bromophenyl, 3-nitrophenyl, 2-chlorophenyl , 3-fluorophenyl, benzo[d][1,3]dioxol-5-yl, 3,4-dimethylphenyl, 2-bromophenyl, 4-fluorophenyl, 3-hydroxyphenyl, 2,3-dichlorophenyl, 4-bromophenyl, 4-methylphenyl, 3-methoxyphenyl or 3,4-dimethoxyphenyl, and
      when ring A is unsubstituted phenyl, X = O, and Y and Z = CH₂, then R¹ is other than
      benzo[d][1,3]dioxol-5-yl, 4-fluorophenyl, 3-hydroxyphenyl, 4-chlorophenyl, 2-methoxyphenyl, 3-methoxyphenyl or 2-thienyl.

The present invention additionally provides a composition comprising a compound, as defined in the foregoing, and at least one pharmaceutically acceptable carrier.

Additionally provided by the present invention is a compound or a composition, as defined in the foregoing, for use as medicament.

Furthermore, the present invention provides the use of a compound, as defined in the foregoing, for treatment of a disease mediated by of one or more kinase-based signaling pathways.

In addition, the present disclosure provides a method for treating a disease characterized by administering an effective amount of a compound, as defined in the foregoing, to an individual.

Moreover, the present disclosure provides a method of modulating a JAK-STAT signaling pathway, comprising contacting said JAK-STAT signaling pathway with a compound, as defined in the foregoing.

### Figures Description

Figure 1.
   A) Overnight serum deprived HEL cells were stimulated with Epo (10 units/ml) for 5, 10, 20, 30, 45 and 60 min. JAK2, pYJAK2, STAT5 and pYSTAT5 were detected using Western immunoblotting as described in methods. B) Overnight serum deprived HEL cells were pre-incubated with compound 4 (C4) (5 µM) for 0.5, 1, 2, and 3 h, and subsequently stimulated with 10 units/ml Epo for 5 min. JAK2, pYJAK2, STAT5 and pYSTAT5 were detected using Western immunoblotting as described in methods. C) Overnight serum deprived HEL cells were pre-incubated with C4 at increasing concentrations (0.5-10 µM) for 3h, and subsequently stimulated with 10 units/ml Epo for 5 min. JAK2, pJAK2, STAT5 and pSTAT5 were detected using Western immunoblotting as described in methods. Equal protein loading was confirmed using a specific human anti-β-actin antibody. C- Veh: HEL cells exposed to either the vehicle for C4 or Epo (0.1% DMSO and PBS, respectively) for the indicated times. C+: HEL cells exposed to C4 vehicle (0.1% DMSO) and stimulated with Epo (10 units/ml) for the indicated time.

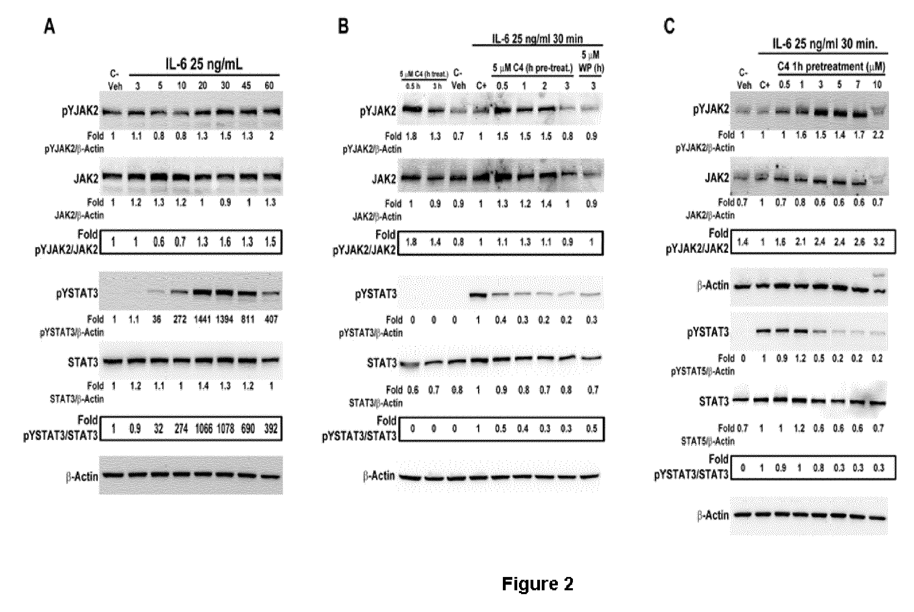

Figure 2. Overnight serum deprived T47D cells were stimulated with IL-6 (25 ng/ml) for 3, 5, 10, 20, 30, 45 and 60 min. JAK2, pYJAK2, STAT3 and pYSTAT3 were detected using Western immunoblotting as described in methods. B) Overnight serum derived T47D cells were pre-incubated with compound 4 (C4) (5 µM) for 0.5, 1, 2, and 3 h and stimulated with 25 ng/ml for 30 min. JAK2, pYJAK2, STAT3 and pYSTAT3 were detected using Western immunoblotting as described in methods. C) Overnight serum derived T47D cells were preincubated with C4 at increasing concentrations (0.5-10 µM) for 3h and stimulated with 25 ng/ml IL-6 for 30 min. JAK2, pJAK2, STAT5 and pSTAT5 were detected using Western immunoblotting as described above. Equal protein loading was confirmed using a specific human anti-β-actin antibody. C- Veh: T47D cells exposed to C4 and IL-6 vehicle (0.1% DMSO and PBS, respectively) for the indicated times. C+: T47D cells exposed to C4 vehicle (0.1% DMSO) and stimulated with IL-6 (25 ng/ml) for the indicated time.

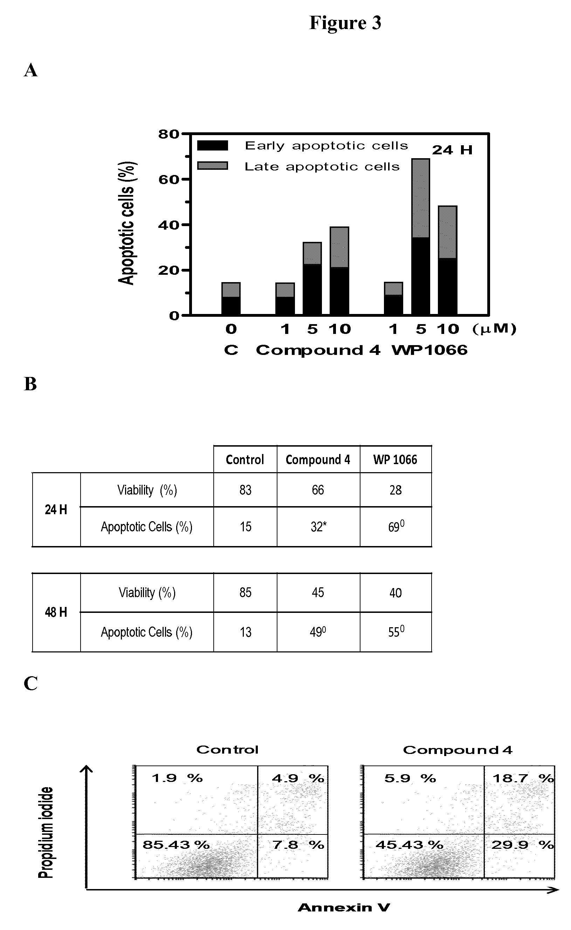

Figure 3. Analysis of phosphatidylserine externalization in human erythroleukemia cells (HEL) treated with compound 4 or the reported JAK-STAT inhibitor WP 1066. A) At different concentrations for 24 hours. B) Representation of the data obtained after treatment of cells with 5 µM of compound 4 for 48 hours compared to vehicle. (C) Data obtained after treatment of the cells with a concentration of 5 µM of compound 4 for 24 or 48 hours. Data are representative of three experiments. * Signifies a P <0.01 difference from control. ⁰ Signifies a P <0.001 difference from control.

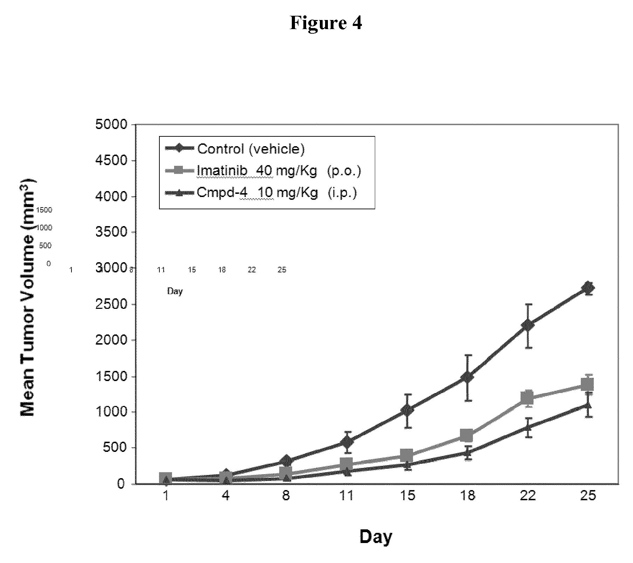

Figure 4: *In vivo* effects of compound 4 and Imatinib on K562 tumor growth in NOD-SCID mice. NOD-SCID mice bearing K562 tumors were treated with vehicle, compound 4 (10 mg/kg i.p) or Imatinib (40 mg/kg, oral) daily except Saturdays and Sundays (Day 1 being a Monday). Mean tumor volumes (mean ± standard deviation [SD]; n = 8) as determined by caliper measurements are shown over the course of the study.

### Detailed description of the Invention

### A. Definitions and general terminology.

Where substitutent groups are specified by their conventional chemical formulae, written left to right, they equally encompass the chemically identical substituents which would result from writing the structure right to left, *e.g*., -CH₂O- is intended to also recite -OCH₂-; -NHS(O)₂- is also intended to represent -S(O)₂NH-, *etc.*

The term "(C₁-C₆)alkyl" refers to straight chain or branched chain hydrocarbon groups having from 1 to 6 carbon atoms. Examples include methyl, ethyl, propyl, isopropyl, butyl, isobutyl, sec-butyl, tert-butyl, pentyl, neopentyl and hexyl, preferably ethyl, propyl, isopropyl, n-butyl, isobutyl, sec-butyl, tert-butyl, pentyl, neopentyl and hexyl, more preferably ethyl, propyl, isopropyl, n-butyl, tert-butyl or pentyl.

The term "(C₂-C₆)alkenyl" refers to straight chain or branched chain hydrocarbon groups having at least one double bond of either E or Z stereochemistry where applicable and 2 to 6 carbon atoms. Examples include vinyl, 1-propenyl, 1- and 2-butenyl and 2-methyl-2-propenyl.

The term "(C₂-C₆)alkynyl" refers to straight chain or branched chain hydrocarbon groups having at least one triple bond and 2 to 6 carbon atoms. Examples include ethynyl, 1- or 2-propynyl, 1-, 2-, or 3-butynyl and methyl-2-propynyl.

The term "alkoxy", "alkylamino" and "alkylthio" are used in their conventional sense, and refer to those alkyl groups attached to the remainder of the molecule via an oxygen atom, an amino group, or a sulfur atom, respectively.

In particular, the term "alkoxyl" or "(C₁-C₆)alkoxyl" refers to an O-alkyl group, wherein alkyl is defined as a (C₁-C₆)alkyl group. Examples of such a substituent include methoxy (CH₃-O-), ethoxy, n-propoxy, isopropoxy, n-butoxy, sec-butoxy, tert-butoxy and the like, preferably ethoxy, n-propoxy, isopropoxy, n-butoxy, sec-butoxy and tert-butoxy groups.

In the case of the term "alkylamino", more than one (C₁-C₆)alkyl may be attached to the nitrogen to afford [(C₁-C₆)alkyl]HN- or [(C₁-C₆)alkyl]₂N- groups. Examples of such substituents include, dipropylamino, isopropylamino, diisopropylamino, butylamino, dibutylamino, pentylamino and dipentylamino groups, preferably methylamino, dimethylamino, ethylamino, diethylamino, and propylamino groups.

The term "alkylthio" or "(C₁-C₆)alkylthio" refers to an S-alkyl group, wherein alkyl is defined as above. Examples of such substituents include methylthio, ethylthio, n-propylthio, isopropylthio, n-butylthio, sec-butylthio and tert-butylthio groups, preferably methylthio and ethylthio groups.

The term "carboxy" refers to a (C₁-C₆)alkyl or aryl group, as disclosed herein, attached to the remainder of the molecule via a carboxy moiety, -O-C(=O)-, to form an ester.

The term "acyl" refers to a (C₁-C₆)alkyl or aryl group attached to the remainder of the molecule via an acyl moiety, -C(=O)-. Examples of such a substituent include methanoyl, ethanoyl, n-propanoyl, isopropanoyl, n-butanoyl, benzoyl and nitrobenzoyl groups, preferably methanoyl, ethanoyl and benzoyl groups.

The term "amido" refers to a (C₁-C₆)alkyl group attached to the remainder of the molecule via an amide moiety, -N-C(=O)-. Examples of such a substituent include methylamide, ethylamide, n-propylamide, isopropylamide and n-butylamide, preferably methylamide and ethylamide groups.

The term "sulphonamide" refers to a (C₁-C₆)alkyl or aryl group attached to the remainder of the molecule via a sulphonamide moiety, -N-S(=O)₂. Examples of such a substituent include methylsulfonamide, ethylsulfonamide, trifluoromethanesulfonamide, phenylsulfonamide and (chlorophenyl)sulfonamide groups, preferably methylsulfonamide and phenylsulfonamide groups.

The term "urea" refers to a H₂N-C(=O)-NH-, [(C₁-C₆)alkyl]HN-C(=O)-NH-, [(C₁-C₆)alkyl]₂N-C(=O)-NH-, H₂N-C(=O)-N[(C₁-C₆)alkyl]-, [(C₁-C₆)alkyl]HN-C(=O)-N[(C₁-C₆)alkyl]- or [(C₁-C₆)alkyl]₂N-C(=O)-N[(C₁-C₆)alkyl]- group. Preferably, the term urea refers to a H₂N-C(=O)-NH-, [(C₁-C₆)alkyl]HN-C(=O)-NH- or H₂N-C(=O)-N[(C₁-C₆)alkyl]- group, more preferably a H₂N-C(=O)-NH- group.

The term "sulfone" refers to a (C₁-C₆)alkyl or aryl group attached to the remainder of the molecule via a sulphone moiety, -S(=O)₂-. Examples of such a substituent include methylsulfonyl, ethylsulfonyl, trifluoromethanesulfonyl, phenylsulfonyl and (chlorophenyl)sulfonyl groups, preferably methylsulfonyl and phenylsulfonyl groups.

The term "(C₃-C₈)cycloalkyl" refers to non-aromatic cyclic hydrocarbon groups having from 3 to 8 carbon atoms. Examples include cyclopropyl, cyclobutyl, cyclopentyl, cyclohexyl, cycloheptyl, norbornyl and adamantyl, preferably cyclopropyl, cyclobutyl, cyclopentyl, cyclohexyl, cycloheptyl and norbornyl.

The term "aryl" refers to single, polynuclear, conjugated or fused residues of aromatic hydrocarbons and substituted or unsusbstituted versions thereof. Examples of unsubstituted aryls include phenyl, biphenyl, naphthyl, tetrahydronaphthyl, anthracenyl, benzanthracenyl and phenylanthrenyl. Examples of substituted aryls include nitrophenyl, chlorophenyl, bromophenyl, fluorophenyl, iodophenyl, benzo[d][1,3]dioxol-5-yl, alkylphenyls, alkoxyphenyl, hydroxyphenyl, aminophenyl, cyanophenyl and mercaptophenyl, preferably 2-nitrophenyl, 2-fluorophenyl, 2-iodophenyl, 3-iodophenyl, 4-iodophenyl, ethylphenyl, isopropylphenyl ethoxyphenyl, aminophenyl, cyanophenyl and mercaptophenyl, more preferably ethylphenyl, isopropylphenyl, ethoxyphenyl, aminophenyl and cyanophenyl.

The term "heterocyclyl" refers to non-aromatic heterocycles having one or more ring forming heteroatoms such as N, O and S atoms. Heterocyclyl groups include monocyclic and polycyclic (e.g., having 2, 3 or 4 fused rings) systems as well as spirocycles. Example groups include morpholino, thiomorpholino, piperazinyl, tetrahydrofuranyl, tetrahydrothienyl, 2,3-dihydrobenzofuryl, piperidinyl and pyrrolidinyl. Ring forming carbon atoms and heteroatoms of a heterocyclyl group can be optionally substituted by oxo or sulfido. Also included in the definition of heterocyclyl group are moieties that have one or more aromatic rings fused (i.e., having a bond in common with) to the non-aromatic heterocyclic ring. Examples of heterocyclyl groups include without limitation, phthalimidyl, naphthalimidyl, and benzo derivatives of heterocycles. The heterocyclyl group can be attached through a ring-forming carbon atom or a ring-forming heteroatom. The number of carbon atoms present in the heterocyclyl group can range from 1 to 20 and may include double or triple bonds.

The term "heteroaryl" refers to an aromatic heterocycle having at least one heteroatom ring member such as sulfur, oxygen or nitrogen. Heteroaryl groups include monocyclic and polycyclic systems. Examples of heteroaryl groups include without limitation, pyridyl, pyrimidinyl, pyrazinyl, pyridazinyl, triazinyl, furyl, quinolyl, isoquinolyl, thienyl, imidazolyl, thiazolyl, indolyl, pyrolyl, benzofuryl, benzothienyl, isoxazolyl, pyrazolyl, triazolyl, tetrazolyl, indazolyl, carbazolyl, benzimidazolyl and indolinyl, preferably pyridyl, pyrimidinyl, pyrazinyl, pyridazinyl, triazinyl, furyl, quinolyl, isoquinolyl, imidazolyl, thiazolyl, indolyl, pyrolyl, benzofuryl, benzothienyl, isoxazolyl, pyrazolyl, triazolyl, tetrazolyl, indazolyl, carbazolyl, benzimidazolyl and indolinyl. In some embodiments, any ring forming N can be substituted by an oxo, to form an N-oxide.

The term "halogen" refers to fluorine, chlorine, bromine and iodine. The terms "amino", "azido", "nitro", "cyano" and "hydroxyl" respectively refer to -NH₂, -N₃, - NO₂, -CN and -OH groups attached to the remainder of the molecule.

The term "substituted or unsubstituted" refers to a group that may or may not be further substituted with one or more groups selected from (C₁-C₆)alkyl, Si[(C₁-C₆)alkyl]₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, amido, azido, nitro, cyano, carboxy, sulphonamide, urea, sulphone, acyl, mercapto, alkylamino, alkylthio and hydroxyl. Preferably, said term "substituted or unsubstituted" refers to a group that may or may not be further substituted with one or more groups selected from (C₁-C₆)alkyl, Si(C₁-C₆)alkyl)₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto and hydroxyl. In one embodiment said term "substituted or unsubstituted" refers to a group that may or may not be further substituted with one or more groups selected from (C₁-C₆)alkyl, Si(C₁-C₆)alkyl)₃, (C₁-C₆)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto, and the like. In an alternative embodiment "substituted or unsubstituted" refers to a group that may or may not be further substituted with one or more groups selected from (C₂-C₆)alkyl, Si(C₁-C₆)alkyl)₃, (C₃-C₈)cycloalkyl, (C₂-C₆)alkenyl, (C₂-C₆)alkynyl, aryl, heteraryl, heterocyclyl, (C₂-C₆)alkoxyl, amino, azido, cyano, carboxy, sulphonamide, urea, sulphone, acyl and mercapto groups. In another embodiment "substituted or unsubstituted" refers to a group that may or may not be further substituted with one or more groups selected from (C₂-C₆)alkyl, (C₂-C₆)alkenyl, (C₂-C₆)alkoxyl, amino, cyano, carboxy, acyl, mercapto and hydroxyl groups, further preferably (C₂-C₆)alkyl, (C₂-C₆)alkenyl, (C₂-C₆)alkoxyl, amino, cyano, carboxy, acyl and mercapto groups.

The phrase "treating" or "treatment" means an alleviation of symptoms associated with a disease or condition, or halt of further progression or worsening of those symptoms. Depending on the disease or condition of the patient, the term "treatment" as used herein may include one or more curative, palliative and prophylactic treatment. Treatment may also include administering a pharmaceutical formulation of the present invention in combination with other therapies. The compounds of the invention can also be administered in conjunction with other drugs and therapies.

### B. Description of the compound(s) of the invention.

The present invention relates to, inter alia, a quinonic compound that modulates one or more of the JAK-STAT pathways and/or is useful, for example, in the treatment of disease associated with signal transduction through this pathway, as mentioned above. The quinonic compound is a polycyclic compound comprising a quinone moiety fused into said polycyclic structure. Among its many embodiments, the quinonic compound of the present invention includes, in one embodiment, a compound having the structure according to formula I: 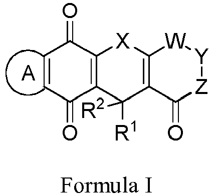 or a pharmaceutically acceptable salt thereof, wherein:
Ring A is independently selected from the group consisting of substituted or unsubstituted (C₃-C₇)cycloalkyl, substituted or unsubstituted aryl, substituted or unsubstituted heteroaryl, and substituted or unsubstituted heterocyclyl having up to 3 heteroatoms selected from N, O, S, SO and SO₂;
X is independently selected from the group consisting of O, NR³, S and SO₂,
   wherein R³ is independently selected from the group consisting of H, CN, SO₂R⁴, C(=O)OR⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂, wherein;
   R⁴ is independently selected from the group consisting of substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂;
   R⁵ and R⁶ are independently selected from the group consisting of H, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂;
   or R⁵ and R⁶, when both connected to the same N, form a 4-, 5-, 6- or 7-membered heterocycloalkyl group or heteroaryl group, each optionally substituted with 0, 1, 2 or 3 substitutents independently selected from OH, CN, halogen, CF₃, (C₁-C₆)alkyl, (C₂-C₆)alkenyl, (C₂-C₆)alkynyl, (C₁-C₆)alkoxy, (C₃-C₇)cycloalkyl, aryl or heteroaryl;
   R⁷ is independently selected from the group consisting of H, CN, substituted or unsubstituted (C₁-C₆)alkyl, substituted or unsubstituted (C₁-C₆)alkoxy, substituted or unsubstituted (C₃-C₇)cycloalkyl, substituted or unsubstituted aryl, substituted or unsubstituted heteroaryl;
W and Z are independently selected from the group consisting of (CR⁸R⁹)ₙ, O and NR³;
Y is independently selected from the group consisting of (CR⁸R⁹)ₙ and C(=O), wherein;
   n is an integer from 0 to 2; and
   R⁸ and R⁹ are independently selected from the group consisting of H, CN, substituted or unsubstituted (C₁-C₆)alkyl, substituted or unsubstituted (C₂-C₆)alkenyl, substituted or unsubstituted (C₂-C₆)alkynyl, substituted or unsubstituted C₁-C₆alkoxy, substituted or unsubstituted (C₃-C₇)cycloalkyl, substituted or unsubstituted aryl, substituted or unsubstituted heteroaryl;
R¹ is independently selected from the group consisting of unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂;
R² is H; with the provisos that when ring A is unsubstituted phenyl, X = O, W and Z = CH₂, and Y = C(CH₃)₂
then R¹ is other than phenyl, 4-nitrophenyl, 4-chlorophenyl, 3-chlorophenyl, 3-bromophenyl, 3-nitrophenyl, 2-chlorophenyl, 3-fluorophenyl, benzo[d][1,3]dioxol-5-yl, 3,4-dimethylphenyl, 2-bromophenyl, 4-fluorophenyl, 3-hydroxyphenyl, 2,3-dichlorophenyl, 4-bromophenyl, 4-methylphenyl, 3-methoxyphenyl or 3,4-dimethoxyphenyl; and
   when ring A is unsubstituted phenyl, X = O, and Y and Z = CH₂, then R¹ is other than benzo[d][1,3]dioxol-5-yl, 4-fluorophenyl, 3-hydroxyphenyl, 4-chlorophenyl, 2-methoxyphenyl, 3-methoxyphenyl or 2-thienyl.

The present invention refers thus to a compound having the formula (Ia): 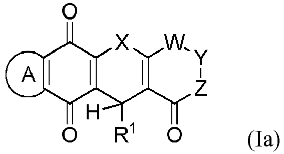
wherein X is independently selected from the group consisting of O, NR³, S and SO₂, wherein;
   (a) R³ is independently selected from the group consisting of H, CN, SO₂R⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂; and
   (b) A, W, Y, Z, R¹, R⁴, R⁵, R⁶, R⁷, R⁸, R⁹ and n are as previously defined. In the aforementioned embodiments symbol A represents a ring system (ring A) which is independently selected from the group consisting of substituted or unsubstituted (C₃-C₈)cycloalkyl, substituted or unsubstituted aryl, substituted or unsubstituted heteroaryl, and substituted or unsubstituted heterocyclyl having up to 3 heteroatoms selected from N, O, S, SO and SO₂. In a preferred embodiment, ring A may be independently selected from the group consisting of a five- or six-membered substituted or unsubstituted aryl, a five- or six-membered substituted or unsubstituted heteroaryl, a substituted or unsubstituted (C₃-C₇)cycloalkyl or a substituted or unsubstituted 5-8 membered heterocyclyl having up to 3 heteroatoms selected from N, O, S, SO and SO₂.

In one embodiment, ring A is a member selected from a substituted or unsubstituted aryl, preferably a substituted or unsubstituted phenyl. Exemplary substituted phenyl moieties include those that are substituted with one or two groups that are independently selected from halogen, nitrile, hydroxyl, amine, nitro, C(=O)OR⁴, COR⁴, CONR⁵R⁶, NR⁵COR⁴, NR⁵CONR⁵R⁶, C(=NR⁷)NR⁵R⁶, SO₂R⁴, SO₂NR⁵R⁶, halogen, CF₃, OR⁵, SR⁵, NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂. Ring A may be a member selected from a phenyl moiety substituted with at least one group independently selected from halogen, nitrile, hydroxyl, amine, nitro, C(=O)OR⁴, COR⁴, CONR⁵R⁶, NR⁵COR⁴, NR⁵CONR⁵R⁶, C(=NR⁷)NR⁵R⁶, SO₂R⁴, SO₂NR⁵R⁶, CF₃, SR⁵, NR⁵R⁶, substituted or unsubstituted (C₁-C₆)alkyl, substituted or unsubstituted (C₂-C₆)alkenyl, substituted or unsubstituted (C₁-C₆)alkoxy, substituted or unsubstituted (C₃-C₇)cycloalkyl.

In another embodiment, ring A is a member selected from a substituted or unsubstituted heteroaryl, preferably a substituted or unsubstituted 5- or 6-membered heteroaryl, more preferably a substituted or unsubstituted 6-membered heteroaryl.

In a further embodiment, ring A is a member selected from a 5-, 6- or 7-membered substituted or unsubstituted heterocyclyl having up to 3 heteroatoms selected from N, O, S, SO and SO₂.

In an alternative second preferred embodiment, the invention provides a compound having the formula Ib: 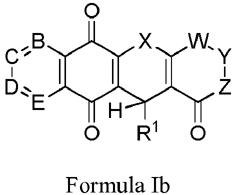 wherein:
(i) the symbols B, C, D and E independently represent a moiety selected from N or CR¹⁰, withthe proviso that B, C, D and E cannot all be N; wherein R¹⁰ represents a group independently selected from H, CN, NO₂, C(=O)OR⁴, COR⁴, CONR⁵R⁶, NR⁵COR⁴, NR⁵CONR⁵R⁶, C(=NR⁷)NR⁵R⁶, SO₂R⁴, SO₂NR⁵R⁶, halogen, CF₃, OR⁵, SR⁵, NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂; and
(ii) W, X, Y, Z, R¹, R³, R⁴, R⁵, R⁶, R⁷, R⁸, R⁹ and n are as previously defined.

In a preferred embodiment of the foregoing, the symbols B and D represent CR¹⁰ and C and E represent N; the symbols C and E represent CR¹⁰ and B and D represent N; the symbols C and E represent CR¹⁰ and D represents N; the symbols B, D and E represent CR¹⁰ and C represents N; or the symbols C, D and E represent CR¹⁰ and B represents N.

In the foregoing, R¹⁰ represents a group independently selected from H, CN, NO₂, C(=O)OR⁴, COR⁴, CONR⁵R⁶, NR⁵COR⁴, NR⁵CONR⁵R⁶, C(=NR⁷)NR⁵R⁶, SO₂R⁴, SO₂NR⁵R⁶, halogen, CF₃, OR⁵, SR⁵, NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₈)cycloalkyl, substituted or unsubstituted aryl, substituted or unsubstituted heteroaryl, and substituted or unsubstituted heterocyclyl having up to 3 heteroatoms selected from N, O, S, SO and SO₂. Preferably, R¹⁰ represents a group independently selected from H, halogen, CF₃, OR⁵, SR⁵, NR⁵R⁶, substituted or unsubstituted (C₁-C₆)alkyl, substituted or unsubstituted (C₁-C₆)alkoxy, substituted or unsubstituted (C₃-C₈)cycloalkyl, substituted or unsubstituted aryl, substituted or unsubstituted heteroaryl, and substituted or unsubstituted heterocyclyl having up to 3 heteroatoms selected from N, O, S, SO and SO₂. More preferably, R¹⁰ represents a group independently selected from H, halogen, CF₃, OR⁵, NR⁵R⁶, substituted or unsubstituted (C₁-C₆)alkyl, substituted or unsubstituted (C₁-C₆)alkoxy, substituted or unsubstituted (C₃-C₈)cycloalkyl, and substituted or unsubstituted aryl. R¹⁰ may also represent a member selected from halogen, nitrile, hydroxyl, amine, nitro, C(=O)OR⁴, COR⁴, CONR⁵R⁶, NR⁵COR⁴, NR⁵CONR⁵R⁶, C(=NR⁷)NR⁵R⁶, SO₂R⁴, SO₂NR⁵R⁶, CF₃, SR⁵, NR⁵R⁶, substituted or unsubstituted (C₁-C₆)alkyl, substituted or unsubstituted (C₂-C₆)alkenyl, substituted or unsubstituted (C₁-C₆)alkoxy, substituted or unsubstituted (C₃-C₇)cycloalkyl. Furthermore, R¹⁰ may be selected from halogen, nitrile, hydroxyl, amine, nitro, C(=O)OR⁴, COR⁴, CONR⁵R⁶, NR⁵COR⁴, C(=NR⁷)NR⁵R⁶, SO₂NR⁵R⁶, CF₃, NR⁵R⁶, substituted or unsubstituted (C₁-C₆)alkyl, substituted or unsubstituted (C₁-C₆)alkoxy.

In the foregoing X is a moiety selected from O, NR³, S and SO₂. In one embodiment, X is a moiety selected from O and NR³. In a preferred embodiment, X is selected from O and NH. More preferably, X is O.

In the foregoing definitions of X, R³ is either independently selected from the group consisting of H, CN, SO₂R⁴, C(=O)OR⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₈)cycloalkyl, substituted or unsubstituted aryl, substituted or unsubstituted heteroaryl or a substituted or unsubstituted heterocyclyl having up to 3 heteroatoms selected from N, O, S, SO and SO₂, or R³ is independently selected from the group consisting of H, CN, SO₂R⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂. Alternatively, R³ is independently selected from the group consisting of H, CN, SO₂R⁴, C(=O)OR⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, and substituted or unsubstituted (C₃-C₇)cycloalkyl;

In the foregoing, R⁴ is a member selected from the group consisting of substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂. Alternatively, R⁴ is independently selected from the group consisting of substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl.

In the foregoing, the symbols R⁵ and R⁶ represent groups that are independently selected from H, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂;or R⁵ and R⁶, when connected to the same N, form a 4-, 5-, 6- or 7-membered heterocycloalkyl group or heteroaryl group, each optionally substituted with 0, 1, 2 or 3 substitutents independently selected from OH, CN, halogen, CF₃, (C₁-C₆)alkyl, (C₂-C₆)alkenyl, (C₂-C₆)alkynyl, (C₁-C₆)alkoxy, (C₃-C₈)cycloalkyl, aryl or heteroaryl. Alternatively, R⁵ and R⁶ are independently selected from the group consisting of H, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl; or R⁵ and R⁶, when both connected to the same N, form a 4-, 5-, 6- or 7-membered heterocycloalkyl group or heteroaryl group, each optionally substituted with 1, 2 or 3 substitutents independently selected from OH, CN, halogen, CF₃, (C₁-C₆)alkyl, (C₂-C₆)alkenyl, (C₂-C₆)alkynyl, (C₁-C₆)alkoxy, (C₃-C₇)cycloalkyl, aryl or heteroaryl;

In the foregoing, R⁷ represents a group independently selected from H, CN, substituted or unsubstituted (C₁-C₆)alkyl, substituted or unsubstituted (C₁-C₆)alkoxy, substituted or unsubstituted (C₃-C₈)cycloalkyl, substituted or unsubstituted aryl, and substituted or unsubstituted heteroaryl. Alternatively, R⁷ represents a group independently selected from H, substituted or unsubstituted (C₁-C₆)alkyl, substituted or unsubstituted (C₁-C₆)alkoxy, substituted or unsubstituted aryl, and substituted or unsubstituted heteroaryl.

In the foregoing, the symbols W and Z represent groups that are independently selected from (CR⁸R⁹)ₙ, O and NR³, while the symbol Y is independently selected from the group consisting of (CR⁸R⁹)ₙ and C(=O). Alternatively, W, Y and Z are independently selected from the group consisting of (CR⁸R⁹)ₙ.

In the foregoing definition of (CR⁸R⁹)ₙ, the symbol n is an integer from 0 to 2. Alternatively, n is an integer of from 0 to 1. Moreover, symbols R⁸ and R⁹ represent groups that are independently selected from H, CN, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₈)cycloalkyl, substituted or unsubstituted aryl, substituted or unsubstituted heteroaryl. Alternatively, R⁸ and R⁹ are independently selected from the group consisting of H, substituted or unsubstituted (C₁-C₆)alkyl, substituted or unsubstituted (C₁-C₆)alkoxy, substituted or unsubstituted (C₃-C₇)cycloalkyl, substituted or unsubstituted aryl, substituted or unsubstituted heteroaryl. Preferably, R⁸ and R⁹ are independently selected from the group consisting of H, and substituted or unsubstituted (C₁-C₆)alkyl, Even more preferably, R⁸ and R⁹ are selected so that W and Z are each CH₂ and Y is CR⁸R⁹, wherein R⁸ and R⁹ represent a (C₁-C₆)alkyl moiety, as hereinbefore defined.

In the foregoing, the symbol R¹ represents a group independently selected from unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂. In a preferred embodiment, R¹ is independently selected from the group consisting of substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂.Preferably, R¹ is independently selected from the group consisting of substituted or unsubstituted aryl, substituted or unsubstituted heteroaryl, or substituted or unsubstituted heterocyclyl having up to 3 heteroatoms selected from N, O, S, SO and SO₂. More preferably, R¹ is independently selected from the group consisting of substituted aryl, substituted or unsubstituted heteroaryl, or substituted or unsubstituted heterocyclyl having up to 3 heteroatoms selected from N, O, S, SO and SO₂. Even more preferably, R¹ is independently selected from the group consisting of substituted or unsubstituted heteroaryl, or substituted or unsubstituted heterocyclyl having up to 3 heteroatoms selected from N, O, S, SO and SO₂. Furthermore, said heteroaryl or heterocyclyl preferably consists of a single ring.

In the foregoing, when R¹ is a substituted aryl, R¹ is a phenyl moiety substituted with at least one group selected from (C₁-C₆)alkyl, Si[(C₁-C₆)alkyl]₃, (C₃-C₈)cycloalkyl, (C₂-C₆)alkenyl, (C₂-C₆)alkynyl, aryl, heteraryl, heterocyclyl, halogen, (C₁-C₆)alkoxyl, amino, amido, azido, nitro, cyano, carboxy, sulphonamide, urea, sulphone, acyl, mercapto, alkylamino, alkylthio and hydroxyl. Alternatively, when R¹ is a substituted aryl, R¹ is a phenyl moiety substituted with at least one group selected from (C₁-C₆)alkyl, (C₃-C₈)cycloalkyl, (C₂-C₆)alkenyl, (C₂-C₆)alkynyl, aryl, heteraryl, heterocyclyl, halogen, (C₁-C₆)alkoxyl, amino, azido, nitro, cyano, carboxy, sulphonamide, urea, sulphone, acyl, mercapto and hydroxyl or R¹ is a phenyl moiety substituted with at least one group selected from (C₁-C₆)alkyl, (C₂-C₆)alkenyl, halogen, (C₁-C₆)alkoxyl, amino, nitro, cyano, carboxy, sulphonamide, sulphone or acyl. Alternatively, when R¹ is a substituted aryl, R¹ is a phenyl moiety substituted with at least one group selected from (C₂-C₆)alkyl, (C₃-C₈)cycloalkyl, (C₂-C₆)alkenyl, (C₂-C₆)alkynyl, aryl, heteraryl, heterocyclyl, (C₂-C₆)alkoxyl, amino, azido, cyano, carboxy, sulphonamide, urea, sulphone, acyl and mercapto groups. When R¹ is a substituted aryl, R¹ may also refer to a phenyl moiety that is substituted with one or more groups selected from (C₂-C₆)alkyl, (C₂-C₆)alkenyl, (C₂-C₆)alkoxyl, amino, cyano, carboxy, acyl and hydroxyl groups, or a phenyl moiety substituted with one or more groups selected from (C₂-C₆)alkyl, (C₂-C₆)alkenyl, (C₂-C₆)alkoxyl, amino, cyano, carboxy and acyl groups.

In a preferred embodiment of the invention relating to the hereinbefore defined compounds of formulae (I), (Ia) and (Ib), X is independently selected from the group consisting of O or NR³, and W, Y and Z are independently selected from the group consisting of (CR⁸R⁹)ₙ. In another preferred embodiment, X is independently selected from the group consisting of O or NH, and W, Y and Z are independently selected from the group consisting of (CR⁸R⁹)ₙ. More preferably, X is independently selected from the group consisting of O or NH and W, Y and Z are independently selected from the group consisting of (CR⁸R⁹)ₙ, wherein the symbol n is an integer from 0 to 1. Even more preferably, X is independently selected from the group consisting of O or NH and W, Y and Z are independently selected from the group comprised of (CR⁸R⁹)ₙ, wherein the symbol n is an integer from 0 to 1 and R⁸ and R⁹ are independently selected from H and a (C₁-C₆)alkyl moiety. Yet more preferably, when X is O, and W and Z are CH₂, then Y is CR⁸R⁹, wherein R⁸ and R⁹ are independently selected from a (C₁-C₆)alkyl moiety. In an even more preferred compound, when X is O, and W and Z are CH₂, then Y is CR⁸R⁹, wherein R⁸ and R⁹ are independently selected from a (C₁-C₆)alkyl moiety, and R¹ is independently selected from the group consisting of substituted aryl, substituted or unsubstituted heteroaryl, or substituted or unsubstituted heterocyclyl having up to 3 heteroatoms selected from N, O, S, SO and SO₂.

In another preferred embodiment of the invention relating to compounds defined by formulae (I), (Ia) and (Ib), R³ is independently selected from the group consisting of H, CN, SO₂R⁴, C(=O)OR⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₇)cycloalkyl; wherein
- R⁴ is independently selected from the group consisting of substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl;
- R⁵ and R⁶ are independently selected from the group consisting of H, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl;
   or R⁵ and R⁶, when both connected to the same N, form a 4-, 5-, 6- or 7-membered heterocycloalkyl group or heteroaryl group, each optionally substituted with 1, 2 or 3 substitutents independently selected from OH, CN, halogen, CF₃, (C₁-C₆)alkyl, (C₂-C₆)alkenyl, (C₂-C₆)alkynyl, (C₁-C₆)alkoxy, (C₃-C₇)cycloalkyl, aryl or heteroaryl;
- n is an integer from 0 to 1; and
- R⁸ and R⁹ are independently selected from the group consisting of H, substituted or unsubstituted (C₁-C₆)alkyl, substituted or unsubstituted (C₁-C₆)alkoxy, substituted or unsubstituted (C₃-C₇)cycloalkyl, substituted or unsubstituted aryl, substituted or unsubstituted heteroaryl.

In the foregoing definition when ring A is unsubstituted phenyl or when B, C, D and E are all CR¹⁰ and R¹⁰ = H, and X = O, W and Z = CH₂ and Y = C(CH₃)₂, then R¹ is other than phenyl, 4-nitrophenyl, 4-chlorophenyl, 3-chlorophenyl, 3-bromophenyl, 3-nitrophenyl, 2-chlorophenyl, 3-fluorophenyl, benzo[d][1,3]dioxol-5-yl, 3,4-dimethylphenyl, 2-bromophenyl, 4-fluorophenyl, 3-hydroxyphenyl, 2,3 dichlorophenyl, 4-bromophenyl, 4-methylphenyl, 3-methoxyphenyl or 3,4-dimethoxyphenyl; and when ring A is unsubstituted phenyl, X = O, W, Y and Z = CH₂, then R¹ is other than benzo[d][1,3]dioxol-5-yl, 4-fluorophenyl, 3-hydroxyphenyl, 4-chlorophenyl, 2-methoxyphenyl, 3 methoxyphenyl or 2-thienyl. Thus, when X = O, W and Z = CH₂ and Y = C(CH₃)₂, then for a compound of formula (I), when ring A is unsubstituted phenyl or for a compound of formula (Ib) when B, C, D and E are all CR¹⁰ and R¹⁰ = H, then R¹ is other than phenyl, 4-nitrophenyl, 4-chlorophenyl, 3-chlorophenyl, 3-bromophenyl, 3-nitrophenyl, 2-chlorophenyl, 3-fluorophenyl, benzo[d][1,3]dioxol-5-yl, 3,4-dimethylphenyl, 2-bromophenyl, 4-fluorophenyl, 3-hydroxyphenyl, 2,3 dichlorophenyl, 4-bromophenyl, 4-methylphenyl, 3-methoxyphenyl or 3,4-dimethoxyphenyl; and when ring A is unsubstituted phenyl, X = O, W, Y and Z = CH₂, then R¹ is other than benzo[d][1,3]dioxol-5-yl, 4-fluorophenyl, 3-hydroxyphenyl, 4-chlorophenyl, 2-methoxyphenyl, 3 methoxyphenyl or 2-thienyl. Similarly, for a compound of formula (Ia), when ring A is unsubstituted phenyl, X = O, W and Z = CH₂ and Y = C(CH₃)₂, then R¹ is other than phenyl, 4-nitrophenyl, 4-chlorophenyl, 3-chlorophenyl, 3-bromophenyl, 3-nitrophenyl, 2-chlorophenyl, 3-fluorophenyl, benzo[d][1,3]dioxol-5-yl, 3,4-dimethylphenyl, 2-bromophenyl, 4-fluorophenyl, 3-hydroxyphenyl, 2,3 dichlorophenyl, 4-bromophenyl, 4-methylphenyl, 3-methoxyphenyl or 3,4-dimethoxyphenyl; and when ring A is unsubstituted phenyl, X = O, W, Y and Z = CH₂, then R¹ is other than benzo[d][1,3]dioxol-5-yl, 4-fluorophenyl, 3-hydroxyphenyl, 4-chlorophenyl, 2-methoxyphenyl, 3 methoxyphenyl or 2-thienyl.

The present invention includes, in a preferred embodiment, a compound having the formula (I), as defined above, with the provisos that when ring A is unsubstituted phenyl, X = O, W and Z = CH₂, Y = C(CH₃)₂ then R¹ is other than phenyl, 4-nitrophenyl, 4-chlorophenyl, 3-chlorophenyl, 3-bromophenyl, 3-nitrophenyl, 2-chlorophenyl, 3-fluorophenyl, benzo[d][1,3]dioxol-5-yl, 3,4-dimethylphenyl, 2-bromophenyl, 4-fluorophenyl, 3-hydroxyphenyl, 2,3-dichlorophenyl, 4-bromophenyl, 4-methylphenyl, 3-methoxyphenyl or 3,4-dimethoxyphenyl; and when ring A is unsubstituted phenyl, X = O, W, Y and Z = CH₂, then R¹ is other than benzo[d][1,3]dioxol-5-yl, 4-fluorophenyl, 3-hydroxyphenyl, 4-chlorophenyl, 2-methoxyphenyl, 3-methoxyphenyl or 2-thienyl.

In addition, the compound of the formula (I) may be defined as above, with the provisos that when ring A is unsubstituted phenyl, X = O, W and Z = CH₂, Y = C(CH₃)₂ then R¹ is other than phenyl, benzo[d][1,3]dioxol-5-yl, 3,4-dimethylphenyl, 2,3-dichlorophenyl, 3,4-dimethoxyphenyl, or a phenyl mono-substituted with a halogen, methyl, methoxy or nitro group; and when ring A is unsubstituted phenyl, X = O, W, Y and Z = CH₂, then R¹ is other than benzo[d][1,3]dioxol-5-yl, 2-thienyl, or a phenyl mono-substituted with a halogen or methoxy group. Furthermore, the compound of the formula (I) may be defined as above, with the provisos that when ring A is unsubstituted phenyl, X = O, W and Z = CH₂, Y = C(CH₃)₂ then R¹ is other than phenyl, benzo[d][1,3]dioxol-5-yl, 3,4-dimethylphenyl, 2,3-dichlorophenyl, 3,4-dimethoxyphenyl, 3-hydroxyphenyl, or a phenyl mono-substituted with a halogen, methyl, methoxy or nitro group; and when ring A is unsubstituted phenyl, X = O, W, Y and Z = CH₂, then R¹ is other than benzo[d][1,3]dioxol-5-yl, 2-thienyl, 3-hydroxyphenyl, or a phenyl mono-substituted with a halogen or methoxy group.

Alternatively, a compound having the formula (I) is as defined above, with the provisos that when ring A is unsubstituted phenyl, X = O, W and Z = CH₂, Y = C(CH₃)₂ then R¹ is other than phenyl, benzo[d][1,3]dioxol-5-yl, 3,4-dimethylphenyl, 2,3-dichlorophenyl, or 3,4-dimethoxyphenyl, or a phenyl mono-substituted with a halogen, methyl, methoxy or nitro group; and when ring A is unsubstituted phenyl, X = O, W, Y and Z = CH₂, then R¹ is other than benzo[d][1,3]dioxol-5-yl, 2-thienyl, or a phenyl mono-substituted with a halogen or methoxy group. In a preferred embodiment, a compound having the formula (I) is as defined above, with the provisos that when ring A is unsubstituted phenyl, X = O, W and Z = CH₂, Y = C(CH₃)₂ then R¹ is other than phenyl, benzo[d][1,3]dioxol-5-yl, 3,4-dimethylphenyl, 2,3-dichlorophenyl, or 3,4-dimethoxyphenyl, or a phenyl mono-substituted with a halogen, methyl, hydroxy, methoxy or nitro group; and when ring A is unsubstituted phenyl, X = O, W, Y and Z = CH₂, then R¹ is other than benzo[d][1,3]dioxol-5-yl, 2-thienyl, or a phenyl mono-substituted with a halogen, hydroxy or methoxy group.

Moreover, a compound having the formula (I) is as defined above, with the proviso that when ring A is unsubstituted phenyl, X = O, W and Z = CH₂, Y = CH₂ or C(CH₃)₂ then R¹ is other than 2-thienyl, phenyl, benzo[d][1,3]dioxol-5-yl, 3,4-dimethylphenyl, 2,3-dichlorophenyl, or 3,4-dimethoxyphenyl, or a phenyl mono-substituted with a halogen, methyl, methoxy or nitro group. In another preferred embodiment, a compound having the formula (I) is as defined above, with the proviso that when ring A is unsubstituted phenyl, X = O, W and Z = CH₂, Y = CH₂ or C(CH₃)₂ then R¹ is other than 2-thienyl, phenyl, benzo[d][1,3]dioxol-5-yl, 3,4-dimethylphenyl, 2,3-dichlorophenyl, or 3,4-dimethoxyphenyl, or a phenyl mono-substituted with a halogen, methyl, hydroxy, methoxy or nitro group. In addition, the compound of the formula (I) may be defined as above, with the proviso that when ring A is unsubstituted phenyl, X = O, W and Z = CH₂, Y = CH₂ or C(CH₃)₂ then R¹ is other than 2-thienyl, phenyl, or a phenyl substituted with at least one halogen, methyl, methoxy, nitro or [1,3]dioxole group. Furthermore, the compound of the formula (I) may be defined as above, with the proviso that when ring A is unsubstituted phenyl, X = O, W and Z = CH₂, Y = CH₂ or C(CH₃)₂ then R¹ is other than 2-thienyl, phenyl, or a phenyl substituted with at least one halogen, methyl, hydroxy, methoxy, nitro or [1,3]dioxole group.

Illustrative examples of compounds of the present invention are shown in formula 1 to 131.

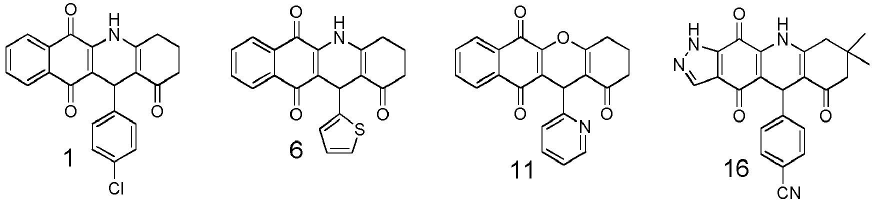

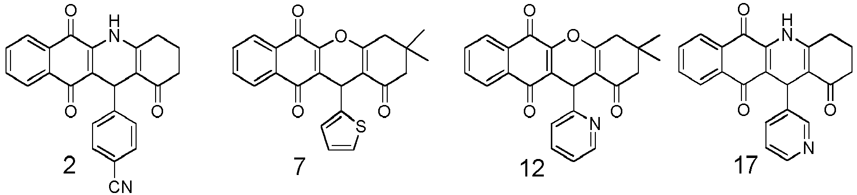

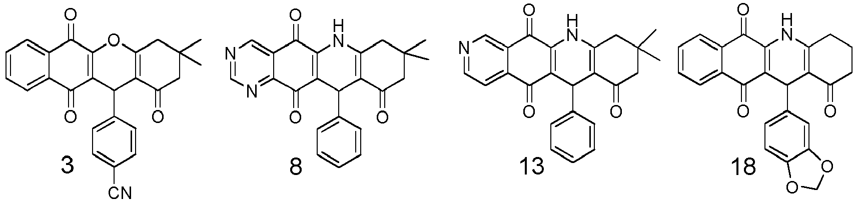

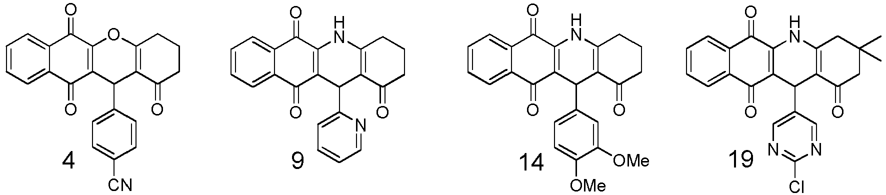

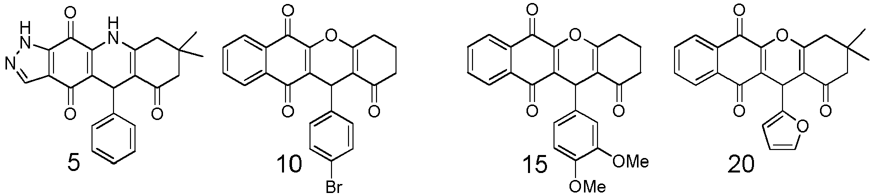

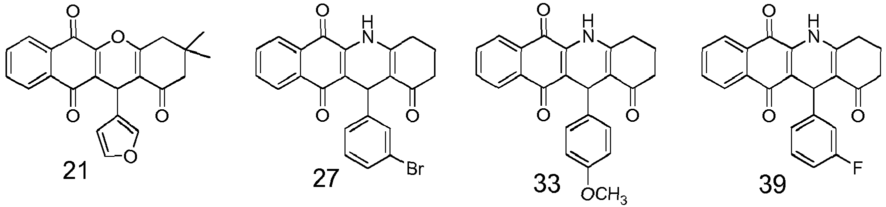

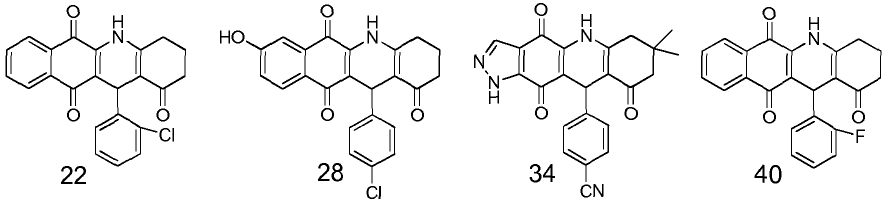

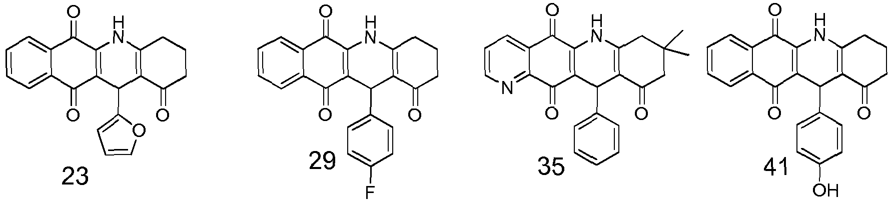

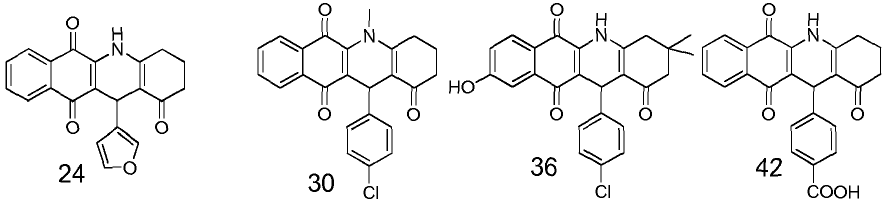

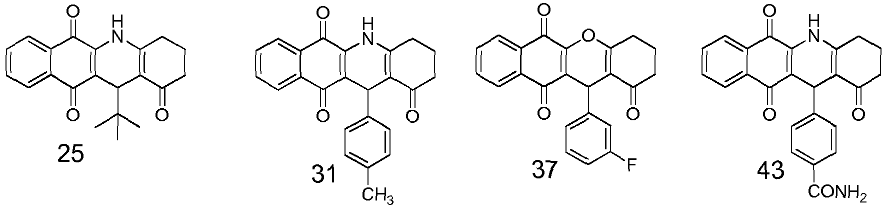

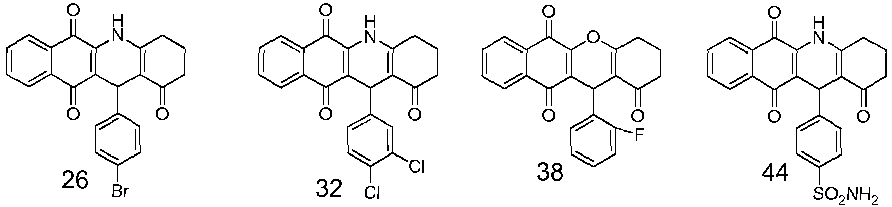

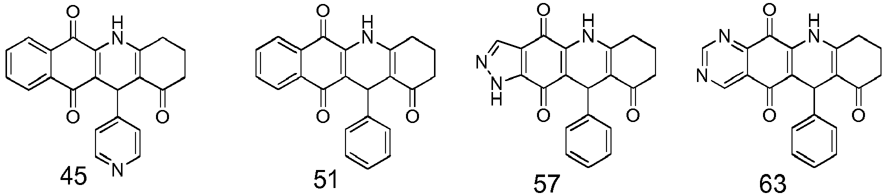

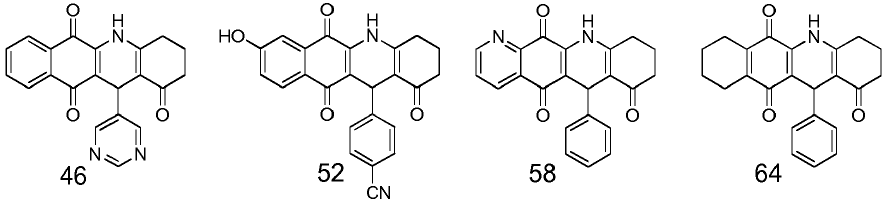

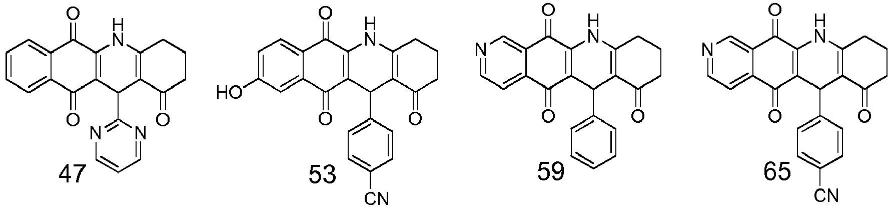

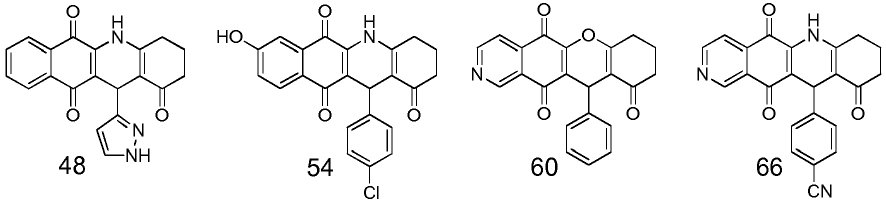

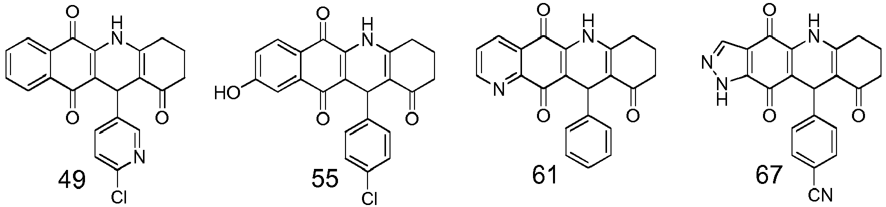

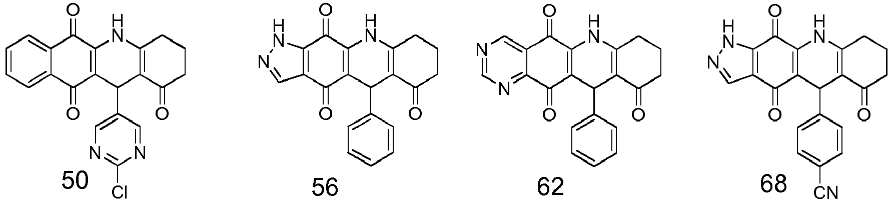

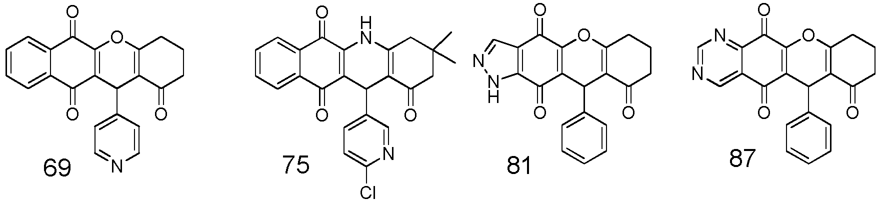

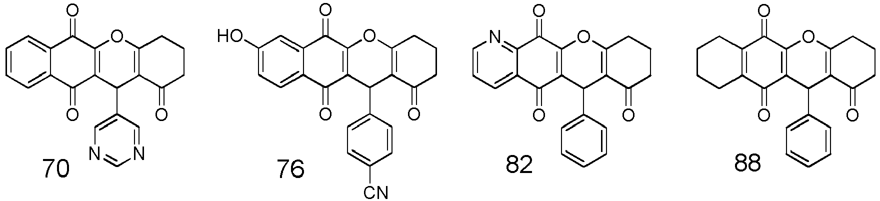

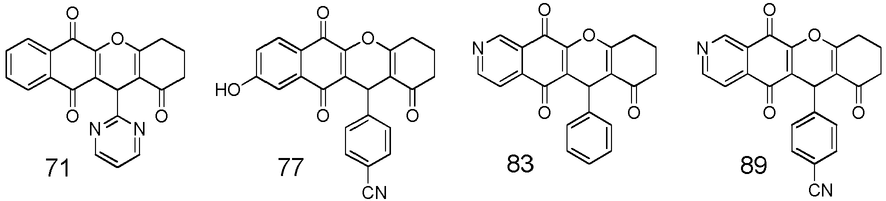

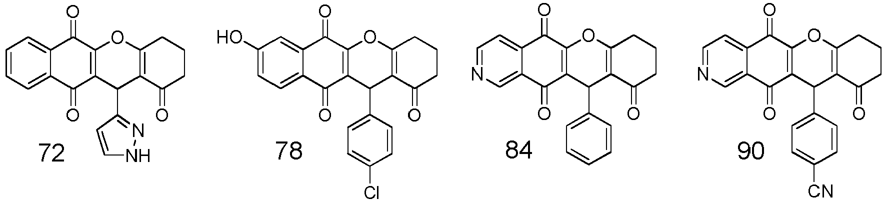

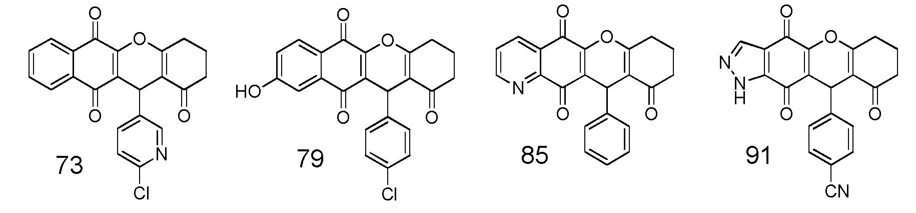

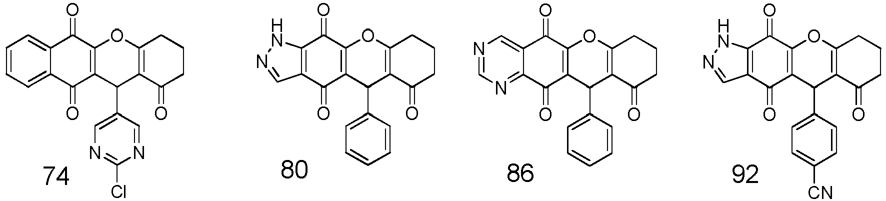

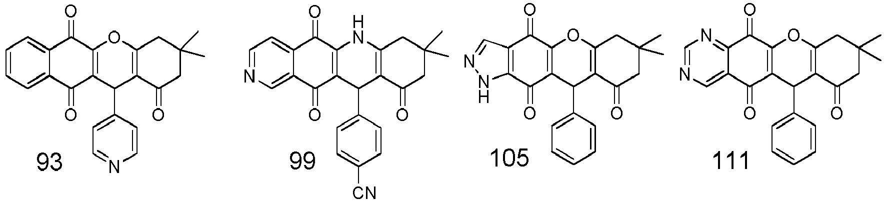

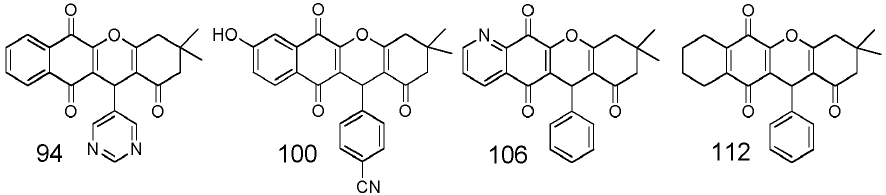

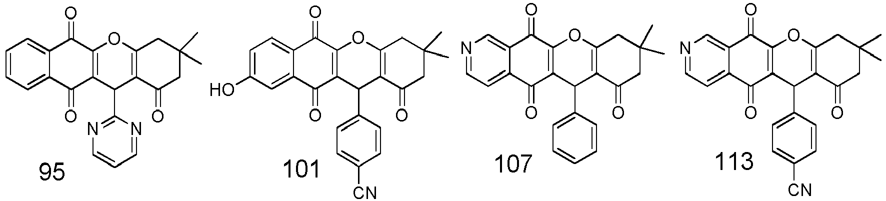

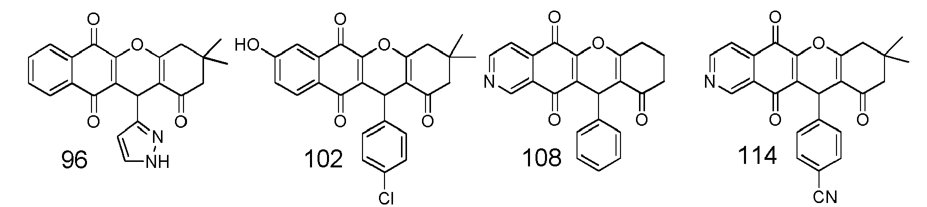

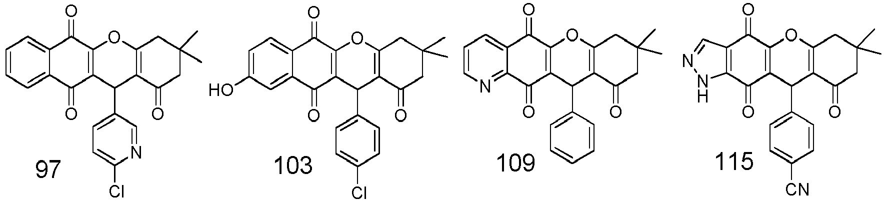

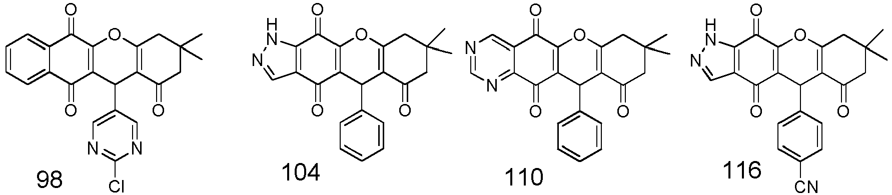

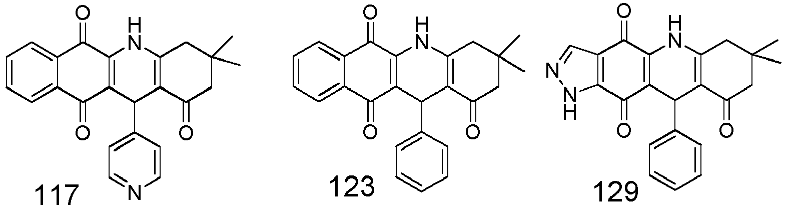

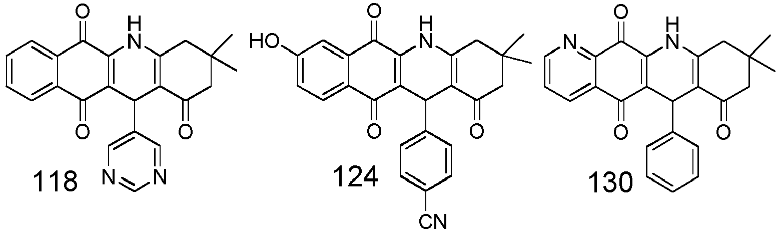

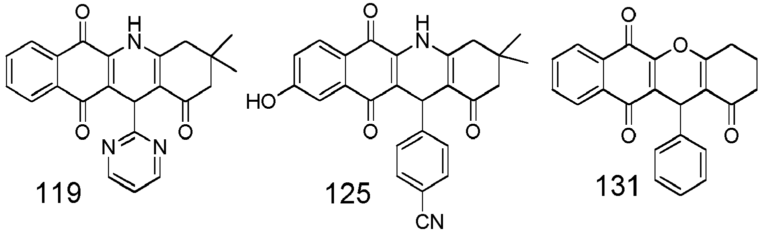

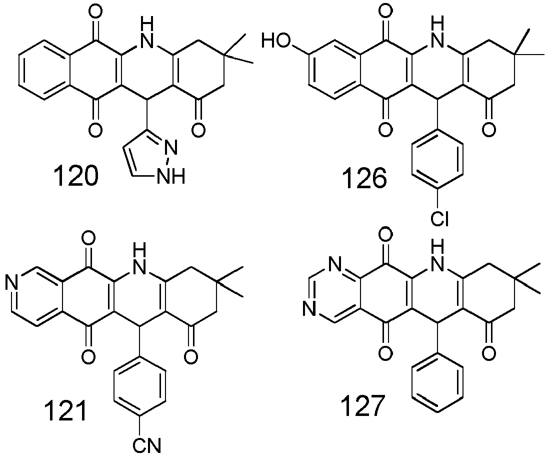

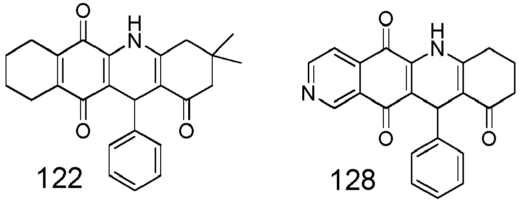

### Uses, formulations and administration.

### Pharmaceutically acceptable compositions.

The term "pharmaceutical acceptable" is employed herein to refer to those compounds, materials, compositions, and/or dosage forms which, when contacted with the tissue of human beings and animals, do not exhibit problems or complications such as toxicity, irritation, or an allergic response, wherein said problems or complications do not outweigh the benefits to the health of the patient, commensurate with a benefit/risk ratio greater than 1.

The present invention also includes pharmaceutically acceptable salts of the compounds described herein. Preferably, the present invention includes pharmaceutically acceptable salts of the compounds of the formulae (I), (Ia) or (Ib), as hereinbefore described.

As used herein "pharmaceutically acceptable salts" refers to derivatives of the disclosed compounds wherein the parent compound is modified by converting an existing acid or base moiety to its salt form. Examples of pharmaceutically acceptable salts include, but are not limited to;
(i) mineral or organic acid salts of basic residues such as amines. Examples of mineral acid salts of basic residues include hydrochloride, hydrobromide, hydroiodide, phosphate and sulfatesalts. Examples of organic acid salts of basic residues include tartrate (e.g. (+)-L-tartrate), maleate, citrate, acetate, lactate, mesylate, methylsulfate and fumarate salts;
(ii) alkali or organic salts of acidic residues such as carboxylic acids. Examples of alkali salts of acidic residues include sodium, potassium, calcium, magnesium and ammonium salts. Examples of organic salts of acidic residues include L-argininate, N-methylglucaminate, cholinate, lysinate, benethaminate, diethylaminate, 2-diethylaminoethanol base, 4-(2-hydroxyethyl)morpholine base, 1-(2-hydroxyethyl)pyrrolidine base and tromethaminate base salts;.

The salts may be formed by conventional means, such as by reacting the free base form of the compound with one or more equivalents of the appropriate acid in a solvent or medium in which the salt is insoluble, or in a solvent such as water which can be removed in vacuo or by freeze drying or by exchanging the anions of an existing salt for another anion using a suitable ion exchange resin.

This disclosure also optionally encompasses prodrugs of the compounds of formula I. For example, compounds of compounds of formula I having free amino, amido, hydroxy, phenolic, or carboxylic acid groups can be converted into prodrugs. Prodrugs of the compounds described herein are those compounds that readily undergo chemical changes under physiological conditions to provide the compounds of the present invention. Said prodrugs are, for example, ester prodrugs or amide prodrugs. Ester prodrugs include derivatives of the hereinbefore described compounds, wherein the parent compound is modified by converting an existing acid or alcohol moiety into an ester. Examples of esters include, but are not limited to, (C₁-C₆)alkyl esters of carboxylic acid moieties, or (C₁-C₆)alkanoyl esters of alcohol moieties. A (C₁-C₆)alkyl ester of a carboxylic acid moiety may be formed by condensing, for example, a (C₁-C₆)alkyl alcohol such as ethanol, with said carboxylic acid moiety. A (C₁-C₆)alkanoyl ester of an alcohol moiety may be formed by condensing, for example a (C₁-C₆)carboxylic acid such as acetic acid, with said alcohol moiety, Amide prodrugs include derivatives of the hereinbefore described compounds, wherein the parent compound is modified by converting an existing acid or amino moiety into an amide. Examples of amides include, but are not limited to, (C₁-C₆)alkyl amides of carboxylic acid moieties, or (C₁-C₆)alkanoyl amides of amine moieties. A (C₁-C₆)alkyl amide of a carboxylic acid moiety may be formed by condensing, for example, a (C₁-C₆)alkyl amine such as ethyl amine, with said carboxylic acid moiety. A (C₁-C₆)alkanoyl amide of an amine moiety may be formed by condensing, for example a (C₁-C₆)carboxylic acid such as acetic acid, with said amine moiety,

Certain compounds of the present invention can exist in unsolvated forms as well as solvated forms, including hydrate forms, and are encompassed within the scope of the present invention. Certain compounds of the present invention may exist in multiple crystalline or amorphous forms and are encompassed within the scope of the present invention.

Also included in the scope of the disclosure are the metabolites of the compounds of formula (I), (Ia) and (Ib), as hereinbefore defined, that is, compounds formed *in vivo* upon administration of the drug. Some examples of metabolites in accordance with the invention include:
(i) where the compound of formula I contains a methyl group, an hydroxymethyl derivative thereof

   (-CH₃→ -CH₂OH);
(ii) where the compound of formula I contains an alkoxy group, an hydroxyl derivative thereof

   (-OR →-OH);
(iii) where the compound of formula I contains a tertiary amino group,a secondary amino derivative thereof (-NR¹R²→ -NHR¹ or -NHR²);
(iv) where the compound of formula I contains a secondary amino group, a primary amino derivative thereof (-NHR¹→ -NN₂); and
(v) where the compound of formula I contains a phenyl group, a phenol derivative thereof

   (-Ph→ -PhOH).

Compounds of formulae (I), (Ia) and (Ib) containing one or more asymmetric carbon atoms can exist as two or more stereoisomers. Where a compound of formula (I), (Ia) or (Ib) contains an alkene or alkenylene group, geometric cis/trans (E/Z) isomers are possible. Where structural isomers are interconvertible via a low energy barrier, tautomeric isomerism ("tautomerism") can occur. This can take the form of proton tautomerism in compounds of formulae (I), (Ia) and (Ib) containing, for example, an imino, keto, or oxime group, or so called valence tautomerismin compounds which contain an aromatic moiety. It follows that a single compound may exhibit more than one type of isomerism. Thus, also included in the scope of the present invention are all stereoisomers, geometric isomers and tautomeric forms of the compounds of formula (I), (Ia) or (Ib), as hereinbefore defined, including compounds that exhibit more than one type of isomerism, and mixtures of one or more thereof. Also included are acid or base salts wherein the counter ion is optically active, for example, d-lactate or 1-lysine, or racemic, for example, dl-tartrate or dl-arginine.

Cis/trans isomers may be separated by conventional techniques well known to those skilled in the art, for example, chromatography or fractional crystallization.

Where a compound possesses a chiral center the compound can be used as a purified enantiomer or diastereomer, or as a mixture of any ratio of stereoisomers. It is however preferred that the mixture comprises at least 70%, 80%, 90%, 95%, 97.5% or 99% of the preferred isomer. Conventional techniques for the preparation/isolation of individual enantiomers include chiral synthesis from a suitable optically pure precursor or enantiomeric enrichment by resolution of the racemate (or the racemate of a salt or derivative) using for example, chiral high pressure liquid chromatography (HPLC), namely using HPLC with an asymmetric resin and a mobile phase.Alternatively, the racemate (or a racemate precursor) may be reacted with a suitable optically active compound, for example, an alcohol, or in the case where the compounds of formula I contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to those skilled in the art.

The present invention includes all pharmaceutically acceptable isotopically-labelled compounds of formulae(I), (Ia) and (Ib), as hereinbefore defined, wherein one or more atoms are replaced by atoms having the same atomic number, but an atomic mass or mass number different from the atomic mass or mass number which predominates in nature. Example of isotopes acceptable for inclusion in the compounds of the invention include isotopes of hydrogen such as ²H and ³H, carbons such as ¹¹C, ¹³C and ¹⁴C, chlorine such as ³⁶Cl, fluorine such as ¹⁸F, iodine such as ¹²³I and ¹²⁵I, nitrogen such as ¹³N and ¹⁵N, oxygen such as ¹⁵O, ¹⁷O and ¹⁸O, phosphorus such as ³²P, and sulphur such as ³⁵S. Certain isotopically-labelled compounds of formula I, for example those incorporating a radioactive isotope, are useful in drug and/or substrate tissue distribution studies. The radioactive isotopes tritium, i.e. ³H, and carbon-14, i.e. ¹⁴C are particularly useful for this purpose in view of their ease of incorporation and ready means of detection. Substitution with heavier isotopes such as deuterium i.e. ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C and ¹⁸F, ¹⁵O and ¹³N, can be useful in Positron Emission Topography (PET) studies for examining substrate receptor occupancy. Isotopically-labelled compounds of formula I can generally be prepared by conventional techniques known to those skilled in the art or by processes analogous to those described in the accompanying Examples and preparations section, using an appropriate isotopically-labelled reagent in place of the non-labelled reagent previously employed. Thus, all references to the hereinbefore described compounds of formula I include references to salts, multicomponent complexes and liquid crystals thereof and to solvates, multicomponent complexes and liquid crystals thereof. Furthermore, the compounds of the invention include compounds of formula I as hereinbefore defined, including all polymorphs, and crystal habitats thereof, and isomers thereof (including optical, geometric and tautomeric isomers) as hereinafter defined and isotopically-labeled compounds of formula I.

### Formulation of the Compounds (Compositions)

The compounds of the present invention can be prepared and administered in a wide variety of oral, parenteral and topical dosage forms. Thus, the compounds of the present invention can be administered by injection, that is, intravenously, intramuscularly, intracutaneously, subcutaneously, intraduodenally, or intraperitoneally. Also, the compounds described herein can be administered by inhalation, for example, intranasally. Additionally, the compounds of the present invention can be administered transdermally. Accordingly, in one embodiment the present invention also relates to a pharmaceutical composition comprising a pharmaceutically acceptable carrier (excipient) and a compound of the present invention, namely a compound of formula (I), (Ia) or (Ib), or a pharmaceutically acceptable salt or prodrug thereof, as hereinbefore defined.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration. A solid carrier can be one or more substances, which may also act as diluents, flavouring agents, binders, preservatives, tablet disintegrating agents, or an encapsulating material.

In powders and tablets, preferably containing from 5% to 80% of the active compound, suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized moulds, allowed to cool, and thereby to solidify.

Liquid preparations include solutions, suspensions, and emulsions, for example, water or water/propylene glycol solutions. For peritoneal injection, liquid preparations can be formulated in solution in aqueous polyethylene glycol solution.

Aqueous solutions suitable for oral use can be prepared by dissolving the active component in water and adding suitable colorants, flavours, stabilizers, and thickening agents as desired. Aqueous suspensions suitable for oral use can be prepared by dispersing the finely divided active component in water with viscous material, such as natural or synthetic gums, resins, methylcellulose, sodium carboxymethylcellulose, and other well-known suspending agents.

Thus, a pharmaceutically acceptable carrier may comprise magnesium carbonate, magnesium stearate, talc, sugar, lactose, pectin, dextrin, starch, gelatin, tragacanth, methylcellulose, sodium carboxymethylcellulose, low melting fatty acid glycerides, cocoa butter, water, propylene glycol, natural or synthetic gums, resins, methylcellulose, and sodium carboxymethylcellulose.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 10000 mg, more typically 1.0 mg to 1000 mg, most typically 10 mg to 500 mg, according to the particular application and the potency of the active component. The composition can, if desired, also contain other compatible therapeutic agents.

### Effective Dosages

The compounds of the present invention, or their pharmaceutically acceptable salts or prodrugs, are administered in a therapeutically effective amount which will vary depending upon a variety of factors including the activity of the specific compound employed, the metabolic stability and length of action of the compound, age, body weight, general health, sex, diet, mode and time of administration, rate of excretion, drug combination effects, the severity of the particular disease, and the patients side effect profile towards the compound. Determination of a therapeutically effective amount of a compound of the invention is well within the capabilities of those skilled in the art.

Patient doses for oral administration of the compounds described herein, which is the preferred mode of administration for prophylaxis treatment of an exemplary disease such as cancer, typically range from about 0.1 mg/day to about 10000 mg/day, more typically from about 1 mg/day to about 1000 mg/day, and most typically 1 mg/day to 500 mg/day. Stated in terms of patient body weight, typical dosages range from about 0.01 to about 150 mg/kg/day, more typically from about 0.1 to about 15 mg/kg/day, and most typically from about 0.5 to about 10 mg/kg/day.

For other modes of administration, dosage amount and interval, can be adjusted individually to provide plasma levels of the administered compound, that are effective for the particular clinical indication being treated.

### C. Use of the compound(s) of the present invention

The present invention also relates to a hereinbefore described compound of formula (I) for use as a medicament. Preferably, said compound is for use as a medicament in the treatment of a disease. Thus, the present invention also relates to the hereinbefore described compounds for use in the treatment of disease. Preferably, it relates to the hereinbefore described compound of formula (I) for use in treating a disease in a patient wherein said patient is a human or other animal species and wherein said disease is mediated by one or more kinase-based signaling pathways. Thus, the present disclosure also relates to a method for treating a disease characterized by administering an effective amount of a hereinbefore described compound of formula (I) to an individual. Preferably, said disease is mediated by one or more kinase-based signaling pathways. Moreover, the present invention also relates to the use of a compound of formula (I) for the manufacture of a medicament for use in the treatment of a disease, preferably wherein said disease is mediated by of one or more kinase-based signaling pathways. The manufacture of a medicament is described in the foregoing sections entitled "Formulation of the Compounds (Compositions)" and "Effective Dosages".

In a preferred embodiment of the foregoing, said disease is mediated by a JAK, a STAT and/or a JAK-STAT signaling pathway.

One embodiment of the present invention includes a compound having the formula I: 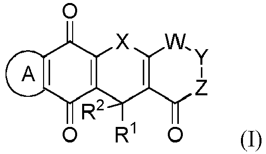 or a pharmaceutically acceptable salt thereof, wherein:
Ring A is independently selected from the group consisting of substituted or unsubstituted (C₃-C₇)cycloalkyl, substituted or unsubstituted aryl, substituted or unsubstituted heteroaryl, and substituted or unsubstituted heterocyclyl having up to 3 heteroatoms selected from N, O, S, SO and SO₂;
X is independently selected from the group consisting of O, NR³, S and SO₂,
   wherein R³ is independently selected from the group consisting of H, CN, SO₂R⁴, C(=O)OR⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂, wherein;
   R⁴ is independently selected from the group consisting of substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂;
   R⁵ and R⁶ are independently selected from the group consisting of H, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂;
   or R⁵ and R⁶, when both connected to the same N, form a 4-, 5-, 6- or 7-membered heterocycloalkyl group or heteroaryl group, each optionally substituted with 0, 1, 2 or 3 substitutents independently selected from OH, CN, halogen, CF₃, (C₁-C₆)alkyl, (C₂-C₆)alkenyl, (C₂-C₆)alkynyl, (C₁-C₆)alkoxy, (C₃-C₇)cycloalkyl, aryl or heteroaryl;
   R⁷ is independently selected from the group consisting of H, CN, substituted or unsubstituted (C₁-C₆)alkyl, substituted or unsubstituted (C₁-C₆)alkoxy, substituted or unsubstituted (C₃-C₇)cycloalkyl, substituted or unsubstituted aryl, substituted or unsubstituted heteroaryl;
W and Z are independently selected from the group consisting of (CR⁸R⁹)ₙ, O and NR³;
Y is independently selected from the group consisting of (CR⁸R⁹)ₙ and C(=O), wherein; n is an integer from 0 to 2; and
   R⁸ and R⁹ are independently selected from the group consisting of H, CN, substituted or unsubstituted (C₁-C₆)alkyl, substituted or unsubstituted (C₂-C₆)alkenyl, substituted or unsubstituted (C₂-C₆)alkynyl, substituted or unsubstituted C₁-C₆alkoxy, substituted or unsubstituted (C₃-C₇)cycloalkyl, substituted or unsubstituted aryl, substituted or unsubstituted heteroaryl;
R¹ is independently selected from the group consisting of unsubstituted (C₁-C₆)alkyl, substituted or unsubstituted (C₂-C₆)alkenyl, substituted or unsubstituted (C₂-C₆)alkynyl, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂;
R² H; with the proviso that when ring A is unsubstituted phenyl, X = O, W and Z = CH₂, Y = C(CH₃)₂ then R¹ is other than phenyl, 4-nitrophenyl, 4-chlorophenyl, 3-chlorophenyl, 3-bromophenyl, 3-nitrophenyl, 2-chlorophenyl, 3-fluorophenyl, benzo[d][1,3]dioxol-5-yl, 3,4-dimethylphenyl, 2-bromophenyl, 4-fluorophenyl, 3-hydroxyphenyl, 2,3-dichlorophenyl, 4-bromophenyl, 4-methylphenyl, 3-methoxyphenyl or 3,4-dimethoxyphenyl, and
when ring A is unsubstituted phenyl, X = O, W, Y and Z = CH₂, then R¹ is other than benzo[d][1,3]dioxol-5-yl, 4-fluorophenyl, 3-hydroxyphenyl, 4-chlorophenyl, 2-methoxyphenyl, 3-methoxyphenyl or 2-thienyl
or a composition comprising said compound and at least one pharmaceutically acceptable carrier, for use as medicament in the treatment of a disease mediated by a JAK or STAT signaling pathway.

However, a compound having the formula I, as defined in the foregoing section B "Description of the compound(s) of the invention", or a composition comprising said compound and at least one pharmaceutically acceptable carrier, is preferred for use as medicament in the treatment of a disease mediated by activity of a JAK or STAT signaling pathway. More preferably, the disease is mediated by a JAK, a STAT or a JAK-STAT pathway and contains a mutant JAK and/or mutant STAT.

In a further preferred embodiment of the foregoing, said disease is an immunological or inflammatory disease such as an autoimmune disease, a hyperproliferative disorder such as cancer or a myeloproliferative disorder (MPD). Preferably, said immunological or inflammatory disease is an autoimmune disease such as a skin disorder, severe combined immunodeficiency (SCID), asthma and chronic obstructive pulmonary disease (COPD), multiple sclerosis, scleroderma, rheumatoid arthritis, juvenile arthritis, type I diabetes, lupus, inflammatory bowel disease, Crohn's disease, immunoglobulin nephropathies, myocarditis or autoimmune thyroid disorder. In addition, said hyperproliferative disease is preferably:
(i) prostate cancer, renal cancer, hepatic cancer, breast cancer, lung cancer, thyroid cancer, pancreatic cancer or glioma;
(ii) lymphoma such as Hodgkin's lymphoma, leukemia such as B-cell Chronic Lymphocytic Leukemia, or multiple myeloma; or
(iii) acute leukemia (ALL), chronic myelomonocytic leukemia (CMML), chronic myeloid leukemia (CML), atypical CML or atypical CMML.

Moreover, said myeloproliferative disorder (MPD) is preferably polycythemia vera (PV), essential thrombocythemia (ET), myeloid metaplasia with myelofibrosis (MMM), hyperosinophile syndrome (HES), idiopathic myclofibrsis (IMF), or systemic mast cell disease (SMCD). The compound of the present invention may also serve as immunosuppressive agents for, amongst others, organ transplants, severe combined immunodeficiency (SCID), asthma and chronic obstructive pulmonary disease (COPD), scleroderma, multiple sclerosis, rheumatoid arthritis, Crohn's disease, Type I diabetes and autoimmune disorders in general. Moreover, the compound of the invention may serve to treat vascular diseases such as hypertension, hypertrophy, cardiac ischemia, heart failure, migraine, Raynaud's disease, pulmonary arterial hypertension.

The present disclosure also relates to a method of modulating at least one kinase-based signaling pathway comprising contacting the kinase signaling pathway with any of the hereinbefore described compounds. In particular, the present disclosure relates to a method of modulating a

JAK-STAT signaling pathway, wherein said method comprises contacting said JAK-STAT signaling pathway with a compound of any of the hereinbefore described formulae (I), (Ia) or (Ib). The method may also serve to simultaneously modulate more than one JAK-STAT signaling pathway, or to modulate a JAK or STAT signaling pathway , wherein said method comprises contacting said JAK, STAT and/or JAK-STAT signaling pathway(s) with a compound of any of the hereinbefore described formulae (I), (Ia) or (Ib). Contacting a JAK, STAT and/or JAK-STAT signaling pathway with said compound means said compound interacts with at least one component of a JAK, STAT and/or JAK-STAT signaling pathway. Components of a JAK, STAT and/or JAK-STAT signaling pathway with which said compound interacts include a protein such as a receptor, a JAK, a STAT, a nucleic acid, or a messenger such as a cytokine, an interferon, an interleukin, a growth factor or a hormone.

In one embodiment of the foregoing method, said modulation is inhibition. Thus, contacting a JAK, STAT and/or JAK-STAT signaling pathway with a compound of formula (I) means preventing transduction on one or several parts of the signaling cascade, such that inhibition of said STAT signaling pathway occurs. Preferably, inhibition involves:
- inhibiting the phosphorylation of one or more Janus Kinases;
- inhibiting the phosphorylation of one or more STATs;
- Inhibiting the transcription of one or more STATs;
- impeding the dimerization of one or more STATs;
- impeding the translocation of one or more STAT dimers to the nucleus;
- impeding the binding of one or more STAT dimers to nuclear DNA;
- reducing the cytosolic concentrations of one or more JAKs; or
- reducing the cytosolic concentrations of one or more STATs.

More preferably, inhibition involves:
- inhibiting the phosphorylation of one or more Janus Kinases;
- inhibiting the phosphorylation of one or more STATs; or
- Inhibiting the transcription of one or more STATs.

In the foregoing method, said JAK is preferably mutant. In a preferred embodiment of the foregoing method, the JAK part of the signaling pathway is JAK1, JAK2, JAK3, TYK2 or a mutant form thereof. In another preferred embodiment thereof, the STAT part of the signaling pathway is a STAT activated by a JAK or another kinase, preferably STAT1, STAT2, STAT3, STAT4, STAT5a, STAT5b, STAT6 or a mutant form thereof. In a further preferred embodiment, the JAK part of the signaling pathway is JAK1, JAK2, JAK3 or TYK2, and the STAT part of the signaling pathway is STAT1, STAT2, STAT3, STAT4, STAT5a, STAT5b or STAT6. Preferably the JAK-STAT receptor, the JAK or the STAT is mutant.

In still another embodiment of present disclosure a method for treating a disease is disclosed. The
method of treatment being characterized by administering an effective amount of a compound of formula I to an individual, particularly, suffering from a disease mediated by activity of a JAK, STAT and/or JAK-STAT signaling pathway and, more particularly, wherein that disease is characterized by being an immunological or inflammatory disease, a hyperproliferative disease or a myeloproliferative disorder (MPD).

### Experimental section

### Methodology for synthesis and characterization of compounds of the invention.

The method by which the compounds of the present invention are prepared is not critical; compounds of the invention, including salts and prodrugs thereof, can be prepared using known organic synthesis techniques and can be synthesized according to any of numerous possible synthetic routes.

The reactions for preparing compounds of the invention can be carried out in suitable solvents which can be readily selected by one skilled in the art of organic synthesis. A given reaction can be carried out in one solvent or a mixture of more than one solvent and at temperatures that can range from the solvent's freezing temperature to the solvent's boiling temperature. In cases where sealed-tube assisted heating is used then the solvent temperature may exceed the boiling temperature of the solvent.

Preparation of compounds of the invention can involve protection and deprotection of various chemical groups. The need for protection and deprotection, and the selection of appropriate protecting groups, can be readily determined by one skilled in the art. The chemistry of protecting groups can be found, for example, in T. W. Greene and P. G.M. Wuts, "Protective Groups in Organic Synthesis", 3rd Ed., Wiley & Sons, Inc, New York (1999), which is incorporated herein by reference in its entirety.

### General analytical methods

Reactions can be monitored according to any suitable method known in the art. For example, product formation can be monitored by chromatographic methods such as thin layer chromatography (TLC) or high performance liquid chromatography (HPLC), or by spectroscopic means, such as nuclear magnetic resonance spectroscopy (e.g., ¹H, ¹³C or ¹⁹F), infrared spectroscopy, spectrophotometry (e.g., UV-visible) or mass spectroscopy.

### Examples

The following examples are offered to illustrate, but not to limit, the claimed invention.

In the examples below, unless otherwise stated, temperatures are given in degrees Celsius (°C); operations were carried out at room, or ambient temperature (typically a range from 15-25°C); evaporation of solvent was carried out using a rotary evaporator under reduced pressure (typically 4-30mmHg/ with a bath temperature of up to 60°C; and the following conventional abbreviations are also used: mp (melting point), L (liter(s)), mL (millitres), mmol (millimoles), g (grams), mg (milligrams), min (minutes), and h (hours).

In an exemplary reaction pathway, fused quinonic compounds of the invention can be prepared utilizing a one-pot, three component cyclisationreaction sequence between 2-hydroxy-1,4-quinones (a), an aldehyde or ketone (b) and a 1,3-dicarbonyl derivative (c), following the method outlined in scheme 1.

Compounds of the type (a) in scheme 1, including substituted hydroxynaphthaquinones, hydroxyisoquinoline-5,8-diones, hydroxy-1H-indazole-4,7-diones, and the like, are commercially available or can be prepared using known literature procedures.Literature examples for the preparation of compounds of the type (a) include Yang, R-Y., et al., Bioorg. Med. Chem., 16, 5635-5643, 2008; Marlerich, J. P., et al., J. Am. Chem. Soc., 127, 6276, 2005.

The desired aldehydes or ketones, designated (b) in scheme 1, were either commercially available or were prepared following standard chemical transformation techniques. General examples for the preparation of aldehydes or ketones, of the type (b) can be found in M.B. Smith and J. March "March's Advanced Organic Chemistry: Reactions, Mechanisms and Structure", 6th edition, Wiley-Interscience; 2007;L.M. Harwood, C.J. Moody and J.M. Percy, "Experimental Organic Chemistry: Standard and Microscale" Wiley-Blackwell; 2 edition, 1999.

The desired 1,3-dicarbonyl derivatives, designated as component (c) in scheme 1, were eithercommercially available or were prepared following standard chemical transformation techniques.

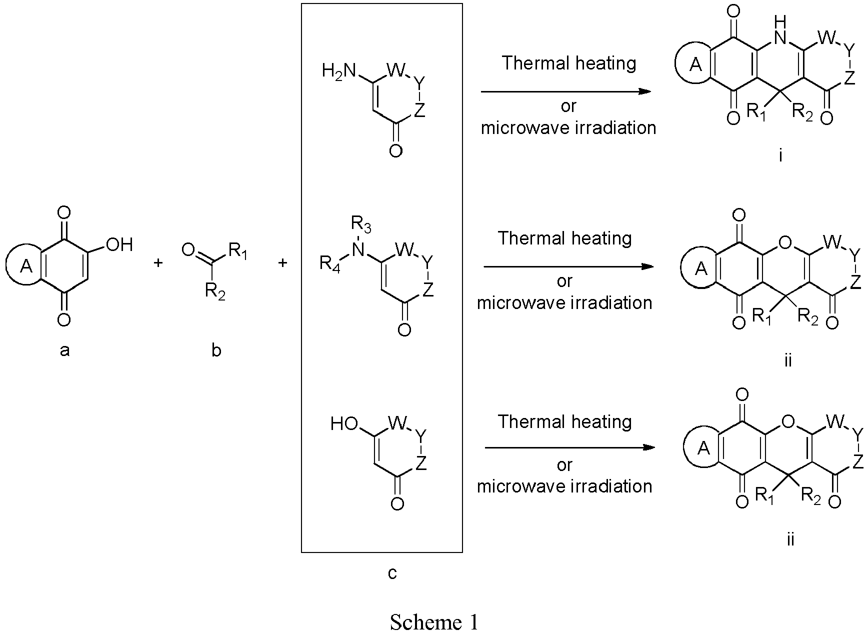

Although the reaction sequence can be done stepwise, as illustrated in scheme 2, the reaction is typically carried out as a one-pot procedure in which the three components (a), (b) and (c) are charged into a vessel and heated, either thermally or by microwave irradiation, in a solvent such as toluene, dioxane, acetonitrile, ethanol, ethylacetate, THF, DMF, NMP or water. Reaction temperatures range from 50°C to 200 °C. Alternatively a catalytic amount of a tertiary base can be added to the reaction system to facilitate product formation. A typical amine for this type of reaction is ethylenediamine diacetate (EDDA).

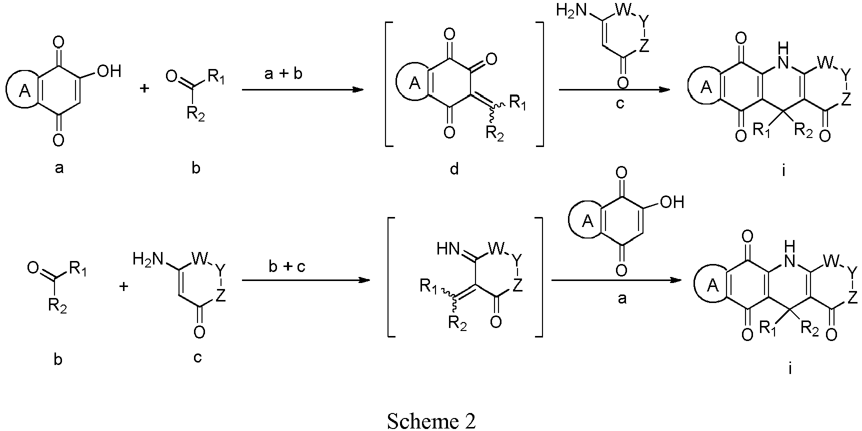

### General preparative method for formation of the dihydrobenzo[b]acridinetrione based fused quinonic systems.

### EXAMPLE 1

Example 1 sets forth a general method for preparing dihydrobenzo[b]acridine based quinonic systems.

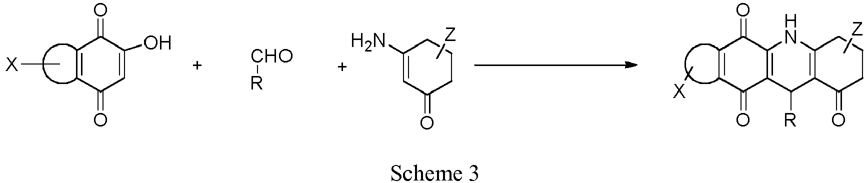

According to scheme 3, a mixture of a 2-hydroxy-1,4-quinone derivative (1equivalent), an aldehyde (1-2 equivalents) and a 3-aminocyclohex-2-enone derivative (1-2 equivalents) was heated at reflux (or in the case of microwave irradiation at a set temperature or time) until TLC analysis indicated that all the 2-hydroxynaphthaquinone had been consumed (typically between 0.1-24 h). Typical solvents include toluene, ethanol, acetonitrile, dioxane, N,N-dimethylformamide and acetic acid. The solvent was removed under reduced pressure, and the desired product was isolated using either column chromatography or preparatie TLC (using either DCM, or EtOAc/hexanes as the eluents). The resulting solid could be crystallized from either DCM/hexanes or Et₂O/hexanes.

### 1.1 Preparation of 12-(4-chlorophenyl)-3,4-dihydrobenzo[b]acridine-1,6,11(2H,SH,12H)-trione (1).

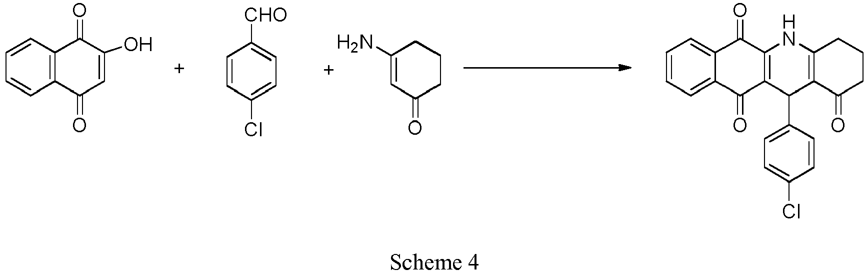

A stirring mixture of 2-hydroxynaphthaquinone (209 mg, 1.2 mmol), 4-chlorobenzaldehyde (190 mg, 1.35 mmol) and 3-aminocyclohex-2-enone (150 mg, 1.35 mmol) in ethanol (5mL) was heated at 160 °C for 2 x 15 mins (Biotage initiator microwave). After cooling to room temperature, the solvent was removed under reduced pressure and the crude material was purified by column chromatography (hexanes:EtOAc; 3:1 to 2:1).Crystallization from DCM/hexanes gave the desired product as a red solid (158 mg, 34%).
¹H NMR (400 MHz, CDCl₃): delta2.03 -2.13 (2H, m), 2.37-2.47 (2H, m), 2.63-2.67 (2H, m), 5.30 (1H, s), 5.41 (1H, s), 7.18 (2H, d, J = 8.4 Hz), 7.27 (2H, d, J = 8.4 Hz), 7.65 (1H, t, J = 8.5 Hz), 7.71 (1H, t, J = 8.5 Hz), 8.02 (1H, d, J = 7.5 Hz) and 8.06 (1H, d, J = 7.5 Hz).
HRMS (EI+) Calcd for C₂₃H₁₆ClNO₃ (M⁺, ³⁷Cl): 391.0789, Found: 391.0807; (M⁺, ³⁵Cl): 389.0819, Found: 389.0827.

### 1.2 Preparation of 12-(thiophen-2-yl)-3,4-dihydrobenzo[b]acridine-1,6,11(2H,5H,12H)-trione (6)

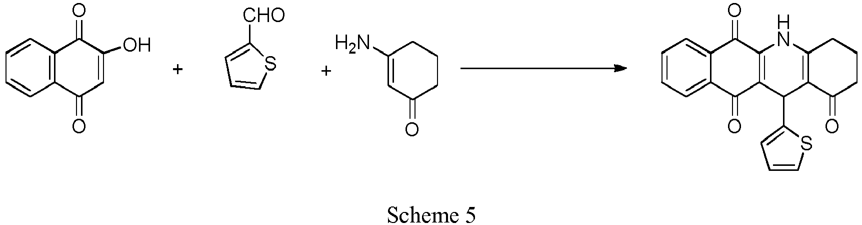

A stirring mixture of 2-hydroxynaphthaquinone (150 mg, 0.86 mmol), thiophene-2-carbaldehyde (112 mg, 1 mmol) and 3-aminocyclohex-2-enone (111 mg, 1 mmol) in ethanol (10 mIL was heated in a sealed tube at 100 °C for 4 h. After cooling to room temperature, the solvent was removed under reduced pressure. Column chromatography (hexanes:EtOAc; 3:1 to 1:1) followed by crystallization from DCM/hexanes gave the desired product as a red solid (27 mg, 8%).
¹H NMR (400 MHz, CDCl₃): delta2.11 -2.17 (2H, m), 2.43 (1H, ddd, J = 16.6, 8.8 & 6.8 Hz), 2.55 (1H, dt, J = 16.8 & 5.1 Hz), 2.61-2.73 (2H, m), 5.83 (1H, s), 6.86 (1H, dd, J = 5.0 & 3.6 Hz), 6.95 (1H, d, J = 3.4 Hz), 7.08 (1H, dd, J = 5.1 &1.1 Hz), 7.37 (1H, brs), 7.69 (1H, dt, J = 7.5 & 1.2 Hz), 7.76 (1H, dt, J = 7.5 & 1.3 Hz), 8.08 (1H, dd, J = 7.6 & 1.1 Hz) and 8.13 (1H, dd, J = 7.6 & 1.1 Hz).
HRMS (EI+) Calcd for C₂₁H₁₅NO₃S (M⁺): 361.0773, Found: 361.0779.

### 1.3 Preparation of 12-(3,4-dimethoxyphenyl)-3,4-dihydrobenzo[b]acridine-1,6,11(2H,5H,12H)-trione (14)

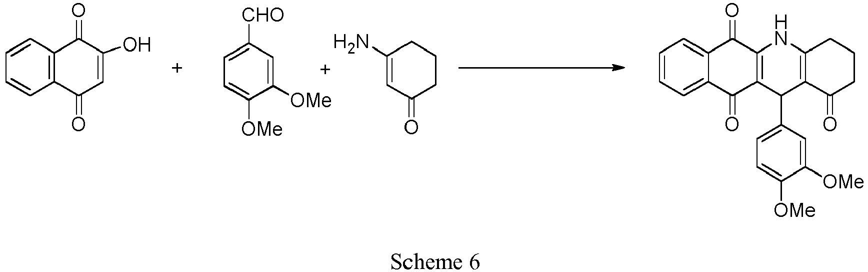

A stirring mixture of 2-hydroxynaphthaquinone (150 mg, 0.86 mmol), 3,4-dimethoxybenzaldehyde (166 mg, 1 mmol) and 3-aminocyclohex-enone (111 mg, 1 mmol) in ethanol (15 mL) was heated at reflux overnight. After cooling to room temperature, the solvent was removed under reduced pressure. Column chromatography (EtOAc) followed by crystallization from DCM/hexanes gave the desired product as a dark red solid (48 mg, 13%).
¹H NMR (400 MHz, CDCl₃): delta2.06 -2.17 (2H, m), 2.40 (1H, ddd, J = 16.6, 10.4 & 5.3 Hz), 2.49 (1H, dt, J = 16.8 & 5.2 Hz), 2.65-2.72 (2H, m), 3.80 (3H, s), 3.90 (3H, s), 5.42 (1H, s), 6.72 (1H, d, J = 8.2 Hz), 6.80 (1H, dd, J = 8.3& 2.0 Hz), 7.10 (1H, d, J = 2.0 Hz), 7.26 (1H, brs), 7.67 (1H, t, J = 7.6 Hz), 7.74 (1H, t, J = 7.6 Hz) and 8.07 (2H, t, J = 6.6 Hz).
HRMS (EI+) Calcd for C₂₅H₂₁NO₅ (M⁺): 415.1420, Found: 415.1420.

### 1.4 Preparation of 12-(4-bromophenyl)-3,4-dihydrobenzo[b]acridine 1,6,11(2H,5H,12H)-trione (26).

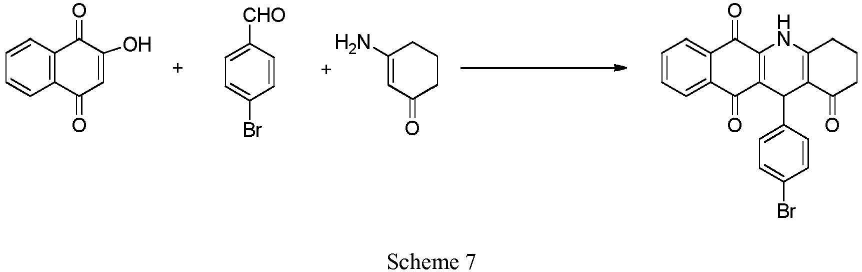

A stirring solution of 2-hydroxynaphthaquinone (522 mg, 3 mmol), 4-bromobenzaldehyde (614 mg, 3.3 mmol) and 3-aminocyclohex-enone (400 mg, 3.6 mmol) in ethanol (15 mL) was heated in a conventional microwave oven (700W) for 3 mins. After cooling to room temperature, the solvent was removed under reduced pressure. Column chromatography (hexanes:EtOAc; 2:1 to 1:1) gave the desired product as a red solid (255 mg, 20%).
¹H NMR (400 MHz, CDCl₃): delta2.06 -2.17 (2H, m), 2.36-2.48 (2H, m), 2.61-2.73 (2H, m), 5.41 (1H, s), 7.28 (2H, d, J = 8.3 Hz), 7.34 (2H, d, J = 8.3 Hz), 7.39 (1H, brs), 7.66 (1H, t, J = 7.4 Hz), 7.72 (1H, t, J = 7.4 Hz), 8.02 (1H, d, J = 7.5 Hz) and 8.06 (1H, t, J = 7.5 Hz).

### 1.5 Preparation of 12-(4-methoxyphenyl)-3,4-dihydrobenzo[b]acridine-1,6,11(2H,SH,12H)-trione (33).

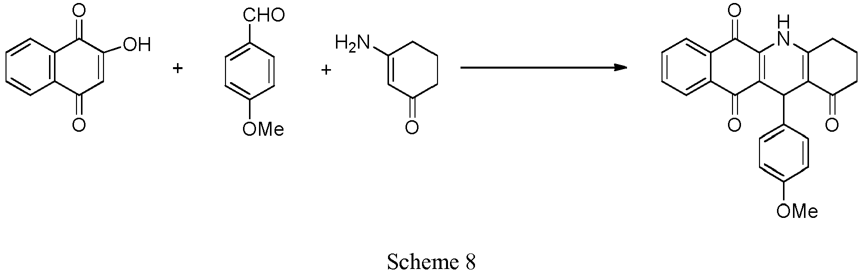

A stirring solution of 2-hydroxynaphthaquinone (174 mg, 1 mmol), 4-methoxybenzaldehyde (204 mg, 1.5 mmol) and 3-aminocyclohex-enone (166 mg, 1.5 mmol) in ethanol (5 mL) was heated at 160 °C for 20 mins (Biotage initiator microwave). After cooling to room temperature, the solvent was removed under reduced pressure. Column chromatography (hexanes:EtOAc; 2:1 to 1:1) gave the desired product as a purple solid (115 mg, 30%).
¹H NMR (400 MHz, CDCl₃): delta2.03 -2.17 (2H, m), 2.33-2.47 (2H, m), 2.58-2.68 (2H, m), 3.72 (3H, s), 5.39 (1H, s), 6.75 (2H, d, J = 8.8 Hz), 7.24 (1H, brs), 7.30 (2H, d, J = 8.8 Hz), 7.64 (1H, dt, J = 7.4& 1.4 Hz), 7.69 (1H, dt, J = 7.6 & 1.6 Hz) and 8.01-8.04 (2H, m).
HRMS (EI+) Calcd for C₂₄H₁₉NO₄ (M⁺): 385.1314, Found: 385.1328.

### 1.6 Preparation of 12-(tert-butyl)-3,4-dihydrobenzo[b]acridine-1,6,11(2H,5H,12H)-trione (25).

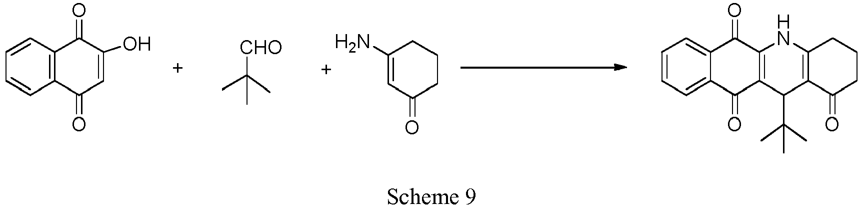

A stirring solution of 2-hydroxynaphthaquinone (150 mg, 1 mmol), pivaladehyde (86 mg, 1 mmol) and 3-aminocyclohex-enone (111 mg, 1 mmol) in DMF (3 mL) was heated in a conventional microwave oven (700W) for 3 x 30 s. After cooling to room temperature, the solvent was removed under reduced pressure. Column chromatography (hexanes:EtOAc; 3:2) followed by crystallization from DCM/hexanes gave the desired product as a red solid (22 mg, 8%).
¹H NMR (400 MHz, CDCl₃): delta0.77 (9H, s), 2.12 -2.16 (2H, m), 2.29-2.34 (1H, m), 2.60-2.70 (3H, m), 4.42 (1H, s), 7.41 (1H, brs), 7.70 (1H, dt, J = 7.5& 1.1 Hz), 7.77 (1H, dt, J = 7.5 & 1.1 Hz), 8.09 (1H, d, J = 7.6 Hz) and 8.15 (1H, d, J = 7.6 Hz).

### 1.7 Preparation of 4-(1,6,11-trioxo-1,2,3,4,5,6,11,12-octahydrobenzo[b]acridin-12-yl)benzonitrile (2).

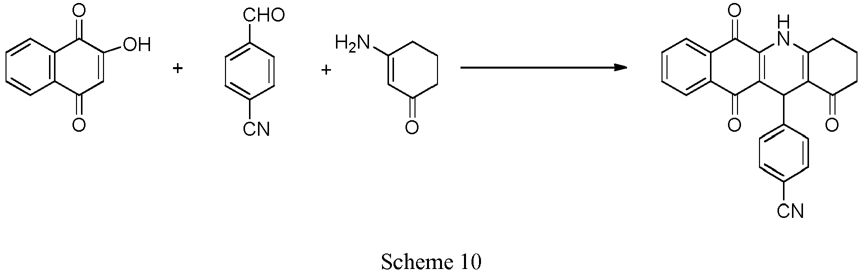

A stirring mixture of 2-hydroxynaphthaquinone (174 mg, 1 mmol), 4-formylbenzonitrile (144 mg, 1.1 mmol), 3-aminocyclohex-2-enone (122 mg, 1.1 mmol) in ethanol (5mL) was heated at 160 °C for 2 x 15 mins (Biotage initiator microwave). After cooling to room temperature, the solvent was removed under reduced pressure and the crude material was purified by column chromatography (hexanes: EtOAc; 3:1 to 1:1). Crystallization from DCM/hexanes gave the desired product as a red solid (53 mg, 14%).
¹H NMR (400 MHz, CDCl₃): delta2.03 -2.17 (2H, m), 2.36-2.48 (2H, m), 2.65-2.71 (2H, m), 5.50 (1H, s), 7.32 (1H, brs), 7.52-7.56 (4H, m), 7.70 (1H, t, J = 7.4 Hz), 7.75 (1H, t, J = 7.5 Hz), 8.04 (1H, d, J = 7.5 Hz) and 8.10 (1H, d, J = 7.5 Hz).
HRMS (EI+) Calcd for C₂₄H₁₆N₂O₃ (M⁺): 380.1161, Found: 380.1162.

### 1.8 Preparation of 12-(4-chlorophenyl)-8-hydroxy-3,4-dihydrobenzo[b]acridine-1,6,11(2H,5H,12H)-trione (28).

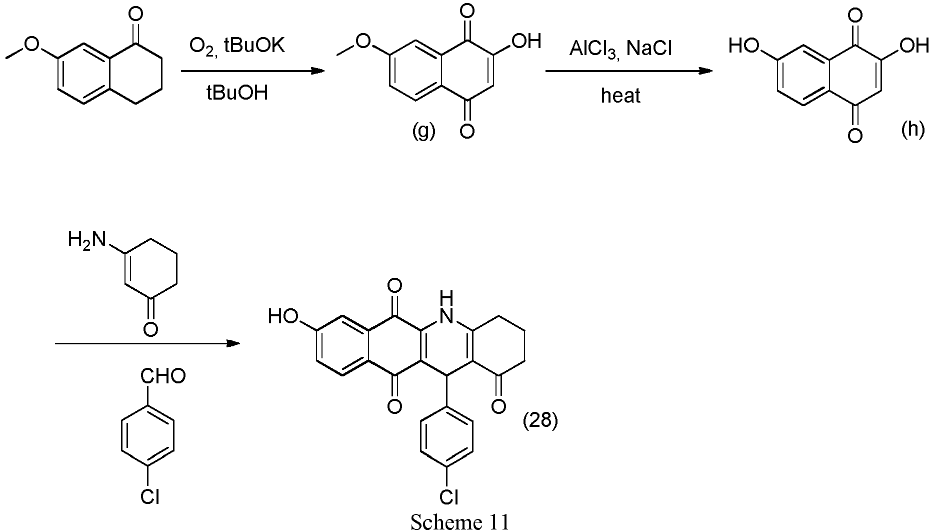

### 1.8.1 Synthesis of 2-hydroxy-7-methoxy-[1,4]-naphthoquinone (g).

Oxygen was passed through a stirring solution of potassium tert-butoxide (5.0 g, 44.5 mmol) in t-butanol (50 mL) at 25-35°C for 30 min. 6-Methoxytretralone (1.0 g, 5.7 mmol) was added and oxygen was passed through the now red mixture until TLC analysis has shown the disappearance of the starting tetralone (typically between 3-6 h). The mixture was cooled to 0°C (ice-bath), quenched with aqueous 1M HCl (20 mL), and extracted with CHCl₃ (6 x 40 mL). The combined organics were extracted with aqueous 1M NaOH (5 x 20 mL). The aqueous phase was then reacidified with concentrate aqueous HCl and extracted with CHCl₃ (5 x 30 mL). The combined organics were dried (Na₂SO₄), filtered and concentrated under reduced pressure to afford the desired product, 2-hydroxy-7-methoxy-[1,4]-naphthoquinone (0.84 g, 72%) as an orange solid. All spectral data we in agreement with those previously published by J.P. Malerich, T.J. Maimore, G. I. Elliot and D. Trauner, J. Am. Chem. Soc., 127(17), 6276-6283, 2005.

### 1.8.2 Synthesis of 2,7-dihydroxy-[1,4]-naphthoquinone (h).

To a stirring melt of AlCl₃ (3.0 g, 22.5 mmol) and NaCl (0.70 g, 12.0 mmol) at 180-190°C was added, in a single portion, finely powdered 2-hydroxy-7-methoxy-[1,4]-naphthoquinone (500 mg, 2.44 mmol). After 5 min the heat was removed and the reaction was allowed to cool to rt. After further cooling to 0°C (ice-bath), the reaction was carefully quenched by the addition of a mixture of crushed ice (50g) and concentrated aqueous HCl (20 mL). The mixture was stirred for 20 mins then extracted with Et₂O (6 x 20 mL). The combined organics were dried (Na₂SO₄), filtered and concentrated under reduced pressure to afford the desired product, 2,7-dihydroxy-[1,4]-naphthoquinone (302 mg, 65%) as a tan solid. All spectral data we in agreement with those previously published by J.P. Malerich, T.J. Maimore, G. I. Elliot and D. Trauner, J. Am. Chem. Soc., 127(17), 6276-6283, 2005.

### 1.8.3 Synthesis of 12-(4-chlorophenyl)-8-hydroxy-3,4-dihydrobenzo[b]acridine-1,6,11(2H,5H,12H)-trione (28).

A stirring solution of 2,7-dihydroxynaphthalene-1,4-dione (91 mg, 0.47 mmol), 4-chlorobenzaldehyde (100 mg, 0.72 mmol) and 3-aminocyclohex-enone (80 mg, 0.72 mmol) in ethanol (5 mL) was heated in a conventional microwave oven (700W) for 5 mins. After cooling to room temperature, the solvent was removed under reduced pressure. Column chromatography (hexanes:EtOAc; 1:1 to 1:2) gave the desired product as an orange solid (46 mg, 24%).
¹H NMR (400 MHz, CD₃OD): delta1.95-2.11 (2H, m), 2.35-2.41 (2H, m), 2.68 (1H, ddd, J= 17.3, 9.8&4.8 Hz), 2.81 (1H, dt, J = 17.5&4.9 Hz), 4.58 (1H, brs), 5.30 (1H, s), 7.05 (1H, dd, J = 8.5 & 2.6 Hz), 7.20 (2H, d, J = 8.5 Hz), 7.30 (1H, d, J = 2.6 Hz), 7.31 (2H, d, J = 8.5 Hz) and 7.96 (1H, d, J = 8.5 Hz).
HRMS (EI+) Calcd for C₂₃H₁₆ClNO₄ (M⁺): 405.0768, Found: 405.0758.

### 1.9 Preparation of 12-(4-chlorophenyl)-5-methyl-3,4-dihydrobenzo[b]acridine-1,6,11(2H,5H,12H)-trione (30).

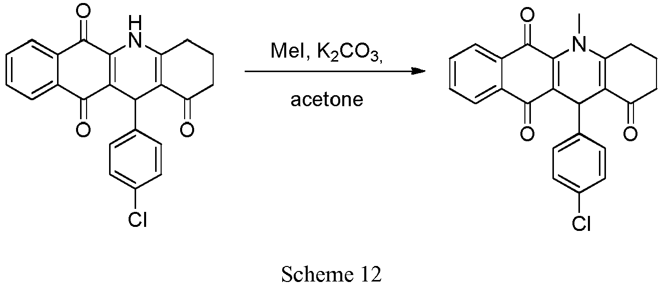

A mixture of 12-(4-chlorophenyl)-3,4-dihydrobenzo[b]acridine-1,6,11(2H,5H,12H)-trione (1) (39 mg, 0.1 mmol), methyl iodide (19 microL mg, 0.3 mmol) and potassium carbonate (138 mg, 1 mmol) in acetone (2 mL) was stirred at room temperature for 72 h.The solvent was removed under reduced pressure and the product was purified by column chromatography (hexanes : EtOAc; 2:1) to afford the desired product as a red solid (19 mg, 47%).
¹H NMR (400 MHz, CDCl₃): delta2.04 -2.20 (2H, m), 2.38-2.45 (2H, m), 2.63 (1H, ddd, J = 17.4, 7.3 & 5.0 Hz), 2.88 (1H, ddd, J = 17.2, 7.0 & 5.3 Hz), 3.63 (3H, s), 5.60 (1H, s), 7.19 (2H, d, J = 8.4 Hz), 7.25 (2H, d, J = 8.4 Hz), 7.66-7.73 (2H, m) and 8.03-8.06 (2H, m).
HRMS (EI+) Calcd for C₂₄H₁₈ClNO₃ (M⁺): 403.0975, Found: 403.0969.

### EXAMPLE 2

Example 2 sets forth a general method for preparing benzo[b]xanthene based fused quinonic systems.

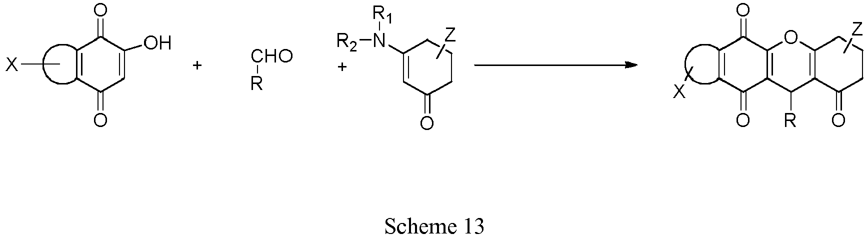

According to scheme 13, a mixture of a 2-hydroxy-1,4-quinone derivative (1 equivalent), an aldehyde (1-2 equivalents) and a 3-bis-subsitituted aminocyclohex-2-enone derivative (1-2 equivalents) was heated at reflux (or in the case of microwave irradiation at a set temperature or time) until TLC analysis indicated that all the 2-hydroxynaphthaquinone had been consumed (typically between 0.1-24 h). Typical solvents include toluene, ethanol, acetonitrile, dioxane, N, N-dimethylformamide and acetic acid. The solvent was removed under reduced pressure, and the desired product was isolated using either column chromatography or preparative TLC (using either DCM, or EtOAc/hexanes as the eluents). The resulting solid could be crystallized from either DCM/hexanes or Et₂O/hexanes.

### 2.1 Preparation of 4-(3,3-dimethyl-1,6,11-trioxo-2,3,4,6,11,12-hexahydro-1H-benzo[b]xanthen-12-yl)benzonitrile (3).

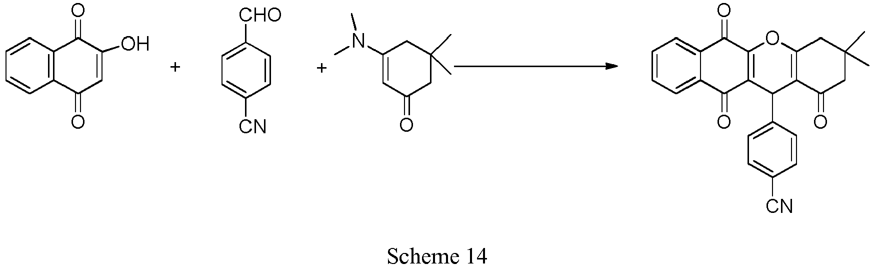

A stirring mixture of 2-hydroxynaphthaquinone (150 mg, 0.86 mmol), 4-formylbenzonitrile (131 mg, 1 mmol) and 3-(dimethylamino)-5,5-dimethylcyclohex-2-enone (167 mg, 1 mmol) in acetonitrile (10 mL) was heated at refluxovernight. After cooling to room temperature, the solvent was removed under reduced pressure and the crude material was purified by column chromatography (hexanes: EtOAc; 3:1 to 1:1). Crystallization from DCM/hexanes gave the desired product as a yellow solid (68 mg, 19%).
¹H NMR (400 MHz, CDCl₃): delta1.06 (3H, s), 1.16 (3H, s), 2.26 (1H, d, J = 16.4 Hz), 2.34 (1H, d, J = 16.4 Hz), 2.67, 2.77 (2H, ABq, J_{AB} = 20.3 Hz), 5.19 (1H, s), 7.53 (2H, d, J = 8.2 Hz), 7.58 (2H, d, J = 8.2 Hz), 7.74-7.77 (2H, m), 8.00-8.03 (1H, m) and 8.15-8.17 (1H, m).
HRMS (EI+) Calcd for C₂₆H₁₉NO₄ (M⁺): 409.1314, Found: 409.1302.

### 2.2 Preparation of 3,3-dimethyl-12-(thiophen-2-yl)-3,4-dihydro-1H-benzo[b]xanthene-1,6,11(2H,12H)-trione (7).

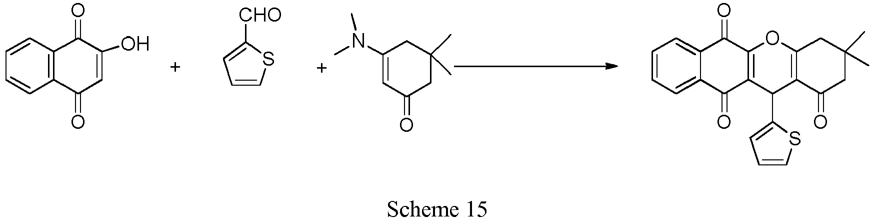

A stirring mixture of 2-hydroxynaphthaquinone (150 mg, 0.86 mmol), thiophene-2-carboxaldehyde (112 mg, 1 mmol) and 3-(dimethylamino)-5,5-dimethylcyclohex-2-enone (167 mg, 1 mmol) in acetonitrile (8 mL) was heated at 100 °C in a sealed tube overnight. After cooling to room temperature, the solvent was removed under reduced pressure and the crude material was purified by column chromatography (hexanes: EtOAc; 3:1). Crystallization from DCM/hexanes gave the desired product as a yellow solid (55 mg, 16%).
¹H NMR (400 MHz, CDCl₃): delta1.16 (3H, s), 1.18 (3H, s), 2.36 (2H, s), 2.66 (1H, dd, J = 17.9 Hz), 2.75 (1H, d, J = 17.9 Hz), 5.53 (1H, s), 6.88 (1H, dd, J = 4.6 & 3.8 Hz), 7.03 (1H, d, J = 3.5 Hz), 7.12 (1H, d, J = 5.1 Hz), 7.73.7.77 (2H, m), 8.09-8.11 (1H, m) and 8.15-8.17 (1H, m). HRMS (EI+) Calcd for C₂₃H₁₈O₄S (M⁺): 390.0926, Found: 390.0912.

### 2.3 Preparation of 12-(benzo[d][1,3]dioxol-5-yl)-3,3-dimethyl-3,4-dihydro-1H-benzo[b]xanthene-1,6,11(2H,12H)-trione (16).

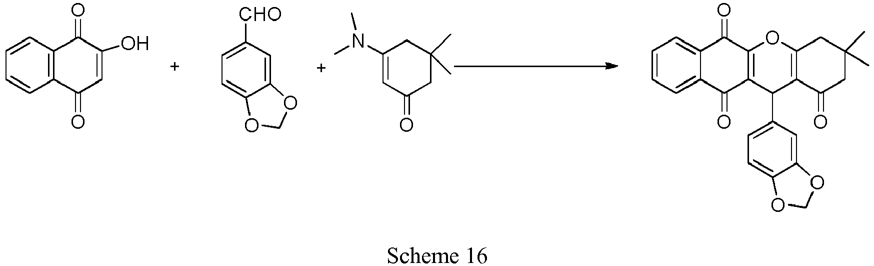

A stirring mixture of 2-hydroxynaphthaquinone (150 mg, 0.86 mmol), benzo[d][1,3]dioxole-5-carbaldehyde (150 mg, 1 mmol) and 3-(dimethylamino)-5,5-dimethylcyclohex-2-enone (167 mg, 1 mmol) in acetonitrile (15 mL) was heated at reflux overnight. After cooling to room temperature, the solvent was removed under reduced pressure and the crude material was purified by column chromatography (hexanes:EtOAc; 3:1 to 1:1). Crystallization from hexanes/EtOAc gave the desired product as a yellow solid (84 mg, 23%).
¹H NMR (400 MHz, CDCl₃): delta1.09 (3H, s), 1.16 (3H, s), 2.29, 2.32 (2H, ABq, J_{AB} = 16.3 Hz), 2.65 (1H, dd, J = 17.8&1.0 Hz), 2.75 (1H, d, J= 17.8 Hz), 5.07 (1H, d, J = 0.8 Hz), 5.90 (2H, dd, J = 5.3& 1.4 Hz), 6.69 (1H, d, J = 8.0 Hz), 6.83 (1H, dd, J = 8.0 & 1.8 Hz), 6.92 (1H, d, J = 1.8 Hz), 7.73-7.75 (2H, m), 8.03-8.05 (1H, m) and 8.14-8.16 (1H, m).
HRMS (EI+) Calcd for C₂₆H₂₀O₆ (M⁺): 428.1260, Found: 428.1266.

### 2.4 Preparation of 12-(furan-2-yl)-3,3-dimethyl-3,4-dihydro-1H-benzo[b]xanthene-1,6,11(2H,12H)-trione (20).

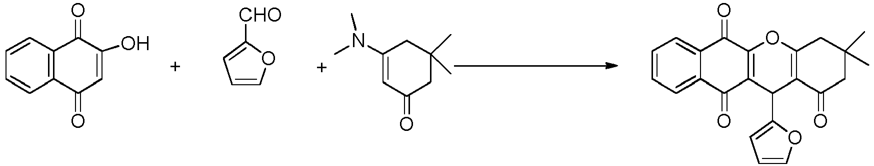

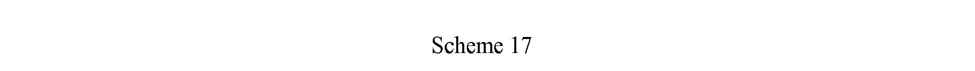

A stirring mixture of 2-hydroxynaphthaquinone (150 mg, 0.86 mmol), furan-2-carbaldehyde (96 mg, 1 mmol) and 3-(dimethylamino)-5,5-dimethylcyclohex-2-enone (167 mg, 1 mmol) in acetonitrile (15 mL) was heated at reflux overnight. After cooling to room temperature, the solvent was removed under reduced pressure and the crude material was purified by column chromatography (hexanes:EtOAc; 3:1). Crystallization from DCM/hexanes gave the desired product as a beige solid (23 mg, 7%).
¹H NMR (400 MHz, CDCl₃): delta1.12 (3H, s), 1.17 (3H, s), 2.35 (2H, s), 2.64 (1H, dd, J = 17.8 & 1.2 Hz), 2.73 (1H, d, J = 17.8 Hz), 5.37 (1H, d, J = 0.8 Hz), 6.27 (1H, dd, J = 3.2 & 1.8 Hz), 6.32 (1H, d, J = 3.0 Hz), 7.21 (1H, dd, J = 1.6&0.6 Hz), 7.75-7.77 (2H, m), 8.09-8.11 (1H, m) and 8.15-8.17 (1H, m).
HRMS (EI+) Calcd for C₂₃H₁₈O₅ (M⁺): 374.1154, Found: 374.1161.

### 2.5 Preparation of 12-(pyridin-2-yl)-3,4-dihydro-1H-benzo[b]xanthene-1,6,11(2H,12H)-trione (11).

### 2.5.1 Synthesis of 3-morpholinocyclohex-2-enone (i).

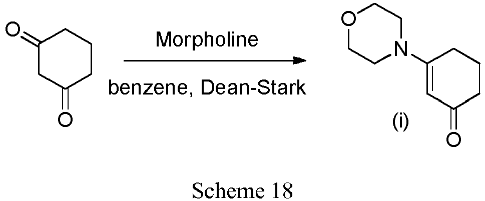

Morpholine (4.35 g, 0.05 mol) was added slowly to a refluxing solution of cyclohexane-1,3-dione (5.6 g, 0.05mol) in benzene (30 mL). The resulting orange solution was maintained at reflux for 5 h during which water was collected from the reaction using a Dean-Stark trap. After cooling to room temperature, the solvent was removed under reduced pressure and the crude material was purified by washing with diethyl ether (4 x 15 mL) and crystallized from EtOAc. The desired product was obtained as an orange solid (8.62 g, 95%). All spectral data were in agreement with those previously published by E.J. Cone, R.H. Garner and A.W. Haynes., J. Org. Chem., 37, 4436-4439, 1972.

### 2.5.2 Synthesis of 12-(pyridin-2-yl)-3,4-dihydro-1H-benzo[b]xanthene-1,6,11(2H,12H)-trione (11).

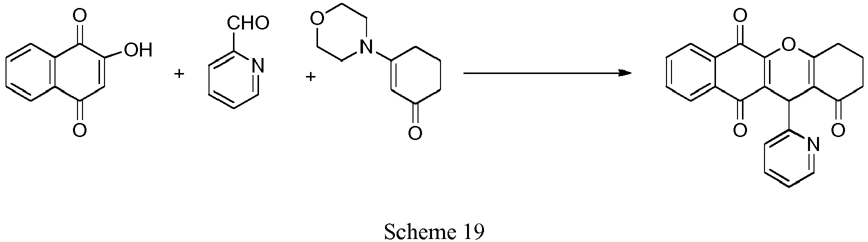

A stirring mixture of 2-hydroxynaphthaquinone (150 mg, 0.86 mmol), picolinaldehyde (107 mg, 1 mmol) and 3-morpholinocyclohex-2-enone (181 mg, 1 mmol) in acetonitrile (10 mL) was heated at reflux overnight. After cooling to room temperature, the solvent was removed under reduced pressure and the crude material was purified by column chromatography (hexanes:EtOAc; 3:1 to 1:1). Crystallization from DCM/hexanes gave the desired product as a yellow solid (53 mg, 17%).
¹H NMR (400 MHz, CDCl₃): delta2.05-2.12 (2H, m), 2.40-2.45 (2H, m), 2.75 (1H, ddd, J = 17.8, 8.2&6.3 Hz), 2.91 (1H, dt, J = 17.9&5.2 Hz), 5.29 (1H, s), 7.05 (1H, dd, J = 7.4 & 4.8 Hz), 7.62 (1H, dt, J = 7.5 & 1.4 Hz), 7.70-7.76 (3H, m), 7.99-8.01 (1H, m), 8.15-8.18 (1H, m) and 8.39-8.41 (1H, m).
HRMS (EI+) Calcd for C₂₂H₁₅NO₄ (M⁺): 357.1001, Found: 357.0989.

### 2.6 Preparation of 4-(1,6,11-trioxo-2,3,4,6,11,12-hexahydro-1H-benzo[b]xanthen-12-yl)benzonitrile (4).

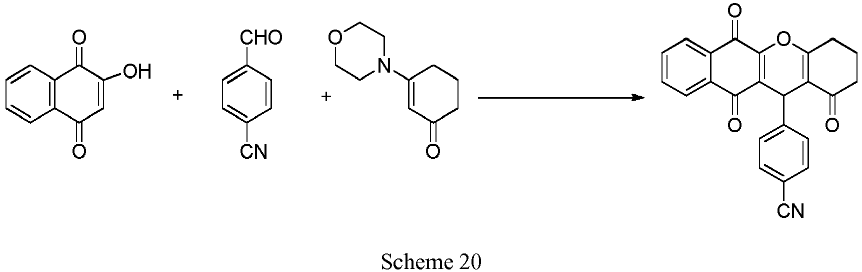

A stirring mixture of 2-hydroxynaphthaquinone (155 mg, 0.9 mmol), 4-formylbenzonitrile (131 mg, 1 mmol) and 3-morpholinocyclohex-2-enone (181 mg, 1 mmol) in acetonitrile (10 mL) was heated at reflux overnight. After cooling to room temperature, the solvent was removed under reduced pressure and the crude material was purified by column chromatography (hexanes:EtOAc; 3:1 to 1:1). Crystallization from DCM/hexanes gave the desired product as a yellow solid (111 mg, 32%).
¹H NMR (400 MHz, CDCl₃): delta2.02-2.19 (2H, m), 2.38-2.49 (2H, m), 2.78 (1H, ddd, J = 17.9, 9.1 & 5.2 Hz), 2.91 (1H, dt, J = 18.1 & 5.2 Hz), 5.31 (1H, d, J = 1.0 Hz), 7.52 (2H, d, J = 8.3 Hz), 7.57 (2H, d, J = 8.0 Hz), 7.74-7.78 (2H, m), 8.00-8.02 (1H, m) and 8.16-8.18 (1H, m).
HRMS (EI+) Calcd for C₂₄H₁₅NO₄ (M⁺): 381.1001, Found: 381.1013.

### EXAMPLE 3

This example illustrates a screening protocol for evaluating putative inhibitors of the JAK and/or STAT pathways for their ability to:
i) impede the phosphorylation of JAK2, or
ii) impede the phosphorylation of STAT5, or
iii) impede the transcription of STAT5, or
iv) reduce the viability of cells possessing a JAKand/or STAT pathway, or
v) induce apoptosis in cells possessing a JAK and/or STAT pathway, or
vi) reduce cell proliferation in a disease mediated by a JAK and/or STAT signaling pathway.

### 3.1 Biological cellular assays.

Cancer cell lines dependent on cytokines and hence JAK-STAT signal transduction, for growth, as well as cell lines expressing consitutively active JAK-STAT pathways can be used to examine the effects of compounds on phosphorylation of JAK kinases or potential downstream substrates such as STAT proteins. These experiments can be performed following an overnight cytokine starvation, followed by a brief preincubation with compound and cytokine stimulation. Proteins are then extracted from cells ans analyzed by techniques familiar to those schooled in the art including Western blotting or ELISAs using antibodies that can differentiate between phosphorylated and total protein. These experiments can investigate the activity of compounds on tumor survival biology or on mediators of inflammatory disease. The ability of compounds to inhibit these cytokine mediated effects can be measured using techniques common to those schooled in the art. Compounds herein can also be tested in cellular models designed to evaluate their potency and activity against mutant JAKs, for example the JAK2V167F mutation found in myeloid proliferative disorders. Endpoints include the effects on cell survival, proliferation, and phosphorylated JAK or STAT proteins.

### 3.1.1 Inhibition of JAK/STAT phosphorylation: cell cultures, total protein extraction and western blot assays.

The Human Erythroleukemia cell line HEL and the human Chronic Myeloid Leukemia cell line K562 were cultured in RPMI 1640 medium containing 10% fetal bovine serum (FBS) and 50U/ml penicillin/50U/ml streptomycin at 37 °C in a 5% CO2 humidified atmosphere to a density of 300.000 viable cells/mL in presence of WP1066 (positive control of phosphorylation inhibition) (Verstovsek et al., 2008) or CM-363 at concentrations of 1, 5 and 10 µM. Cell culture reagents were all obtained from BioWhittaker® (Lonza, Verviers, Belgium). Cell growth and viability was determined by propidium iodide (fluorescent dye) exclusion method using the Accuchip 4X Counting Kit and the ADAM automatic cell counter (Digital-Bio, NanoEntek, Ic., Washington St, MA, USA).As a control of unspecific phosphorylation, some cells in each experiment were exposed to vehicle (negative control of phosphorylation inhibition) (0.1% DMSO). Preliminary observations indicated that the presence of 0.1% DMSO did not affect basal levels of JAK/STAT phosphorylation in both cell lines.

For total protein extraction, cells were scraped with RIPA 1X lysis buffer (25 mM Tris-HCl pH 7.6, 150 mM NaCl, 1% NP-40, 1% sodium deoxycholate, 0.1% SDS, no. 89900, Thermo Scientific, Rockford, IL, USA) supplemented with 1X protease and 1X phosphatase inhibitor cocktails (100X Halt™ Protease Inhibitor Cocktail, no. 87786 and 100X Halt™ Phosphatase Inhibitor Cocktail, no. 78420, both from Thermo Scientific, Rockford, IL, USA). An aliquot of each extract was preserved for protein quantification by bicinchoninic acid assay (Pierce® BCA Protein Assay Kit, no. 23225, Thermo Scientific, Rockford, IL, USA).

Proteins were solubilized in sample buffer containing 0.0625 M Tris-HCl, pH 6.8, 2.3% (wt/vol) SDS, 10% (vol/vol) glycerol, 5% (vol/vol) β-mercaptoethanol, and 0.001% (wt/vol) bromophenol blue and boiled at 95°C for 5 min. Equal amounts (50 µg) of each sample were electrophoresed on 8-10% sodium dodecyl sulfate - polyacrylamide gel electrophoresis (SDS-PAGE) and transferred to nitrocellulose membranes (iBlot® Gel Transfer Stacks, Nitrocellulose, no. IB4010-02, Invitrogen, Barcelona, Spain) as described previously (Fernandez, L, et al., Endocrinology, 13, 1815-1824, 1998; B. Guerra et al., J. Neurochem, 91, 99-109, 2004; B. Guerra et al., J. Appl. Physiol., 102, 1786-1792, 2007; B. Guerra et al., J. Appl. Physiol., 111, 715-725, 2011). The nitrocellulose membranes were then removed from the iBlot® Dry GelTransfer Device (no. IB1001EU, Invitrogen, Barcelona, Spain) and placed in a Ponceau S staining solution (0.5% Ponceau S and 1% glacial acetic acid in water) to verify equal loading of protein in the control and treated samples. After the membranes were stained for 5 min, they were rinsed for 2 min and examined. Equal loading of protein was verified, and the membranes were rinsed for an additional 10 min and immunoscreened. Membranes were blocked with 5% blotting grade blocker nonfat dry milk in Tris Buffered Saline with 0.05% Tween 20 (TBST; blotto-blocking buffer) for at least 1h at room temperature. They were washed thrice in TBS with 0.1% Tween 20 (TBST-0.1). Next, the membranes were incubated over night at 4°C with the appropriate anti-phospho-antibodies (described below) all diluted (1:1000) in 1% bovine serum albumin (BSA)-1% blotto in TBST (BSA-blotto blocking buffer). Antibody-specific labeling was revealed by incubation with a HRP-conjugated goat anti-mouse secondary antibody (1:5000) (no. sc-2031, Santa Cruz Biotechnology, Santa Cruz, CA, USA) or a HRP-conjugated goat anti-rabbit secondary antibody (1:5000) (no. sc-2030, Santa Cruz Biotechnology, Santa Cruz, CA, USA) and visualized with the Immu-Star™ WesternC™ kit (Bio-Rad Laboratories, Hercules, CA, USA). Membranes were stripped of bound antibodies by incubation in stripping buffer (Restore™ Western Blot Stripping Buffer, no. 21059, Thermo Scientific, Rockford, IL, USA) at room temperature for 30min with agitation. Membranes were washed for 3 x 10min in TBST-0.1, blocked with blotto-blocking buffer for at least 1h and re-probed with the corresponding anti-total kinase antibodies (described below) all diluted (1:1000) in blotto-blocking buffer. To check for differences in loading and transfer efficiency across membranes, an antibody directed against β-actin (monoclonal mouse anti-β-actin antibody, no. sc-81178, Santa Cruz Biotechnology, Santa Cruz, CA, USA) was used to hybridize with all membranes previously incubated with the respective phospho and total kinase antibody. Specific bands were visualized using the ChemiDoc XRS System (Bio-Rad Laboratories, Hercules, CA, USA) and analyzed with the image analysis program Quantity One (Bio-Rad Laboratories, Hercules, CA, USA). For immunosignal quantification, band densities were normalized to basal values obtained in vehicle-treated samples. The densitometry analysis was carried out immediately before saturation of immunosignal. Results were normalized by dividing the numerical value of each sample signal by the numerical value of the signal from the corresponding value of the β-actin that served as a control (S. Verstovsek, et al., Clin. Cancer Res., 14, 788-796, 2008)

The following anti-human specific antibodies were used to detect the phosphorylated form of the specified kinases: monoclonal rabbit anti-phospho-Tyr^{1007/1008}-Jak2 antibody (no. 3776, Cell Signaling Technology, Danvers, MA), polyclonal rabbit-anti-phospho-Tyr⁶⁹⁴-Stat5 antibody (no. 9351 Cell Signaling Technology, Danvers, MA), polyclonal rabbit anti-phospho-Tyr⁷⁰⁵-Stat3 antibody (no. 9145, Cell Signaling Technology, Danvers, MA), polyclonal rabbit-anti-phospho-Tyr⁷⁰¹-Stat1 antibody (no. 9171, Cell Signaling Technology, Danvers, MA), polyclonal rabbit-anti-phospho-Tyr⁴¹⁶-Src antibody (no. 2101, Cell Signaling Technology, Danvers, MA), monoclonal mouse anti-phospho-Thr²⁰²/Tyr²⁰⁴-p44/42 MAPK (ERK1/2) antibody (no. 9106, Cell Signaling Technology, Danvers, MA), polyclonal rabbit anti-phospho-Thr¹⁸³/Tyr¹⁸⁵-SAPK/JNK antibody (no. 9251, Cell Signaling Technology, Danvers, MA), monoclonal mouse antiphospho- Thr¹⁸⁰/Tyr¹⁸²-p38MAPK antibody (no. 9216, Cell Signaling Technology, Danvers, MA) and polyclonal rabbit-anti-phospho-Ser⁴⁷³-Akt antibody (no. 9271, Cell Signaling Technology, Danvers, MA).

The following anti-human specific antibodies were used to detect the total form of the specified kinases: monoclonal rabbit anti-Jak2 antibody (no. 3230, Cell Signaling Technology, Danvers, MA), polyclonal rabbit anti-Stat5 antibody (no. sc-836, Santa Cruz Biotechnology, Santa Cruz, CA, USA), monoclonal mouse anti-Stat3 antibody (no. 9139, Cell Signaling Technology, Danvers, MA), polyclonal rabbit anti-Stat1 antibody (no. 9172, Cell Signaling Technology, Danvers, MA), polyclonal rabbit anti-Src antibody (no. 2109, Cell Signaling Technology, Danvers, MA), polyclonal rabbit anti-p44/42 (ERK1/2) antibody (no. 9102, Cell Signaling Technology, Danvers, MA), polyclonal rabbit anti-JNK1/3 (C-17) antibody (no. sc-474, Santa Cruz Biotechnology, Santa Cruz, CA, USA), polyclonal rabbit anti-p38MAPK antibody (no. 9212, Cell Signaling Technology, Danvers, MA) and polyclonal rabbit anti-Akt antibody (no. 9272, Cell Signaling Technology, Danvers, MA).

### Results

Table 4 sets forth potencies of representative compounds of the invention in the JAK2 phosphorylation western blot assay.

**Table 4**

| **Compound #** | **Activity** | **Compound #** | **Activity** | **Compound #** | **Activity** |
|---|---|---|---|---|---|
| 3 | ++ | 10 | ++ | 19 | ++ |
| 4 | ++ | 12 | ++ | 20 | +++ |
| 5 | + | 13 | ++ | 21 | + |
| 6 | + | 15 | ++ | 22 | ++ |
| 7 | +++ | 17 | +++ | 24 | +++ |
| 8 | +++ | 18 | ++ | 27 | + |

| | | | | | |
|---|---|---|---|---|---|
| +++ represents an inhibition at 10microM >75%; ++ represents an inhibition at 10microM >50%; + represents an inhibition at 10microM >30%. | | | | | |

Table 5 sets forth potencies of representative compounds of the invention in the STAT5 phosphorylation western blot assay.

**Table 5**

| **Compound #** | **Activity** | **Compound #** | **Activity** | **Compound #** | **Activity** |
|---|---|---|---|---|---|
| 3 | +++ | 10 | + | 18 | + |
| 4 | +++ | 11 | + | 20 | ++ |
| 5 | + | 12 | ++ | 21 | ++ |
| 6 | ++ | 13 | +++ | 22 | +++ |
| 7 | +++ | 15 | ++ | 24 | + |
| 8 | + | 17 | + | 26 | + |

| | | | | | |
|---|---|---|---|---|---|
| +++ represents an inhibition at 10microM >75%; ++ represents an inhibition at 10microM >50%; + represents an inhibition at 10microM >50%. | | | | | |

### 3.1.2 Inhibition of STAT5 transcription.

Plasmids: The full-length GHR was subcloned into pcDNA1/ampicillin (Invitrogen) as described previously (Ross RJ, Esposito N, Shen XY, et al., Mol Endocrinol 1997, 11:265-73). The pSPI-GLE1 (serine protease inhibitor 2.1 GAS-like element 1)-luc reporter construct was generated by subcloning the Stat5-binding element from serine protease inhibitor gene together with a minimal thymidine kinase promoter into the pGL2 plasmid (Promega), which contains the firefly luciferase cDNA (Wood TJ, Sliva D, Lobie PE, et al. J Biol Chem 1995, 270:9448-53).

Cell culture: HEK-GHR cell line was maintained in Dulbecco's Modified Eagle Medium (DMEM)/F12 supplemented with 10% fetal bovine serum (FBS) and 50U/ml penicillin/50U/ml streptomycin. The cells were maintained in a humidified incubator with 5% CO₂ at 37°C. Cell culture reagents were obtained from PAA laboratories. The HEK-GHR cell line is a stable clone generated and kindly donated by Prof. Richard Ross (Clinical Sciences, Northern General Hospital, Sheffield, UK). Briefly, HEK-GHR cell line was generated in HEK 293 cells (human kidney embryonal cell line) by transfection with the calcium phosphate procedure of the GHR plasmid with pcDNA3/Neo (Invitrogen). Selection was made using G418 (400 ug/ml, Sigma) (Maamra M, Finidori J, Von Laue S, et al., J Biol Chem 1999, 274(21):14791-8).

Luciferase reporter assay: Transcription assays were performed in HEK-GHR. The HEK-GHR cells were co-transfected with Stat5 luciferase (pSPI-GLE1-luc) reporter vector together with a plasmid constitutively expressing beta-galactosidase by using Metafectene Pro as described by the manufacturer (Biontex Laboratories GmbH, Munich, Germany). Following transfection, HEK-GHR cells were serum-starved for 20 h. Cells were then treated with vehicle (0.1 % DMSO) or the indicated compound for 45 minutes prior to the addition of 50 nM recombinant human Growth Hormone (rhGH) (Norditropin, Novo Nordisk) to the medium. Six hours following rhGH addition, cells were harvested with Passive Lysis Buffer (Promega), and levels of luciferase were measured using Luciferase Assay System as described by the manufacturer (Promega). The Galacto-Light Plus System (Applied Biosystems) was used to measure beta-galactosidase activity.

Statistical analysis. The concentration of drug that provokes 50% reduction of luciferase activity (IC50) was determined by using non-linear regression analysis and data were fitted to a log concentration vs normalized response curve in GraphPad software v5 (San Diego, CA).

### Results

Table 6 sets forth potencies of representative compounds of the invention in the HEK-GHR, STAT5 transcription assay.

**Table 6**

| **Compound #** | **Activity** | **Compound #** | **Activity** | **Compound #** | **Activity** |
|---|---|---|---|---|---|
| 1 | ++ | 10 | +++ | 20 | +++ |
| 2 | +++ | 11 | +++ | 21 | +++ |
| 3 | +++ | 12 | +++ | 22 | ++ |
| 4 | +++ | 13 | ++ | 24 | ++ |
| 5 | ++ | 15 | + | 26 | ++ |
| 6 | ++ | 16 | ++ | 27 | ++ |
| 7 | +++ | 17 | ++ | | |
| 8 | ++ | 19 | ++ | | |

| | | | | | |
|---|---|---|---|---|---|
| +++ represents an IC₅₀< 1microM; ++ represents an IC₅₀< 5microM; + represents an IC₅₀< 10microM. | | | | | |

### 3.1.3 MTT Cell viability assay.

Cell viability was assayed by measuring the mitochondrial reduction of the tetrazolium salt 3-(4,5-methyltiazol-2yl)-2,5diphenyl-tetrazolium bromide] (MTT) (Applichen; A2231,0010) (Mosmann T.. J Immunol Methods. 1983 16;65(1-2):55-63; Chuan Y-C et al 2010.. Oncogene. 29 (10), 1531-1542. Cells were seeded in 96-well plates in medium supplemented with 10% FBS (Lonza Cat. N°: 14-502F), 2mM L-Glut (Lonza Cat. N°: BE17-605E) and 100U/ml PEST (Lonza Cat. N°: DE17-605E) at a cell density of 5000-20000 cells/well. Cells were seeded at a density according to their exponential growth phase. Vehicle (0.05% DMSO) or drug were added to culture medium at the indicated concentrations (10 to 0,01microM) during 48 h. Then, MTT (0.3 mg/ml) was added to cells following by incubation for 2-4h at 37°C in the dark. The medium was then discarded and the formazan precipitate was solubilized by addition of 20% SDS (Sigma; L-5750) in 0.02N HCl (Panreac; 131020.1611) for 12-16 h. The optical density was measured at 595 nm with the iMark Microplate Reader (BioRad, USA). Data was expressed as percent growth above the level in controls and the results in figures were plotted as means ± SEM of each test point from 3 replicates.

Statistical analysis. The concentration of drug that provokes 50% reduction of cell viability (IC50) was determined by using non-linear regression analysis and data were fitted to a log concentration vs normalized response curve in GraphPad software v5 (San Diego, CA).

### Results

Table 7 sets forth potencies of representative compounds of the invention in the HEL-based cell viability assay.

**Table 7**

| **Compound #** | **Activity** | **Compound #** | **Activity** | **Compound #** | **Activity** |
|---|---|---|---|---|---|
| 1 | ++ | 10 | ++ | 20 | +++ |
| 2 | ++ | 11 | + | 21 | ++ |
| 3 | +++ | 12 | ++ | 22 | ++ |
| 4 | ++ | 13 | ++ | 24 | ++ |
| 5 | ++ | 15 | +++ | 26 | + |
| 6 | ++ | 16 | ++ | 27 | ++ |
| 7 | + | 17 | ++ | | |
| 8 | ++ | 19 | ++ | | |

| | | | | | |
|---|---|---|---|---|---|
| +++ represents an IC₅₀< 1microM; ++ represents an IC₅₀< 3microM; + represents an IC₅₀< 10microM. | | | | | |

Table 8 sets forth potencies of representative compounds of the invention in the K562-based cell viability assay.

**Table 8**

| **Compound #** | **Activity** | **Compound #** | **Activity** |
|---|---|---|---|
| 1 | + | 16 | ++ |
| 2 | +++ | 18 | + |
| 3 | +++ | 26 | +++ |
| 4 | +++ | 27 | + |
| 13 | ++ | | |

| | | | |
|---|---|---|---|
| +++ represents an IC₅₀< 1microM; ++ represents an IC₅₀< 3microM; + represents an IC₅₀< 10microM. | | | |

### 3.1.4 Inhibition of recombinant human erythropoietin (Epo) induced STAT5 phosphorylation in Human Erythroleukemia (HEL) cells by compound 4.

### Introduction

The glycoprotein hormone Erythropoietin (Epo) is one of the most important stimulatory factors for erythropoiesis, which promotes the proliferation and prevents apoptosis of erythroid precursors allowing differentiation into maturated red blood cells. For a more detailed discussion of the effects of Epo see M. J. Koury, S. T. Sawyer, S. J. Brandt, Curr Opin Hematol 9, 93 Mar, 2002. The Epo receptor (EpoR) belongs to the cytokine receptor superfamily and exerts its effects through receptor homodimerization and phosphorylation of JAK2 on tyrosine residues in the activation loop. Subsequently, JAK2 phosphorylates the signal transducer and activators of transcription (STAT), which translocate to the nucleus to initiate transcription of target genes. For more information about the effects of Epo on the JAK-STAT pathway see T. D. Richmond, M. Chohan, D. L. Barber, Trends in cell biology 15, 146, Mar, 2005.

HEL is a JAK2^{V617F}-dependent cell line. This mutant JAK2 is constitutively active. This results in constitutive activation of STAT5 and a malignant phenotype. This mutation is central to the erythropoietin-independent erythroid proliferation in polycythemia vera (PV). More information about the HEL cell line and the relation between JAK2-V617F and thrombocythemia (ET), and primary myelofibrosis (PMF), can be found in H. Quentmeier, R. A. MacLeod, M. Zaborski, H. G. Drexler, Leukemia 20, 471, 2006 and R. L. Levine, D. G. Gilliland, Blood 112, 2190, 2008.

### Experimental conditions

HEL cells were cultured in RPMI 1640 medium containing 10% fetal bovine serum (FBS), 2 mM L-Glutamine and 50U/ml penicillin/50U/ml streptomycin at 37 °C in a 5% CO₂ humidified atmosphere. Before experiments, HEL cells were serum starved (0.1% FBS) for 18h. Cell culture reagents were all obtained from BioWhittaker® (Lonza, Verviers, Belgium).

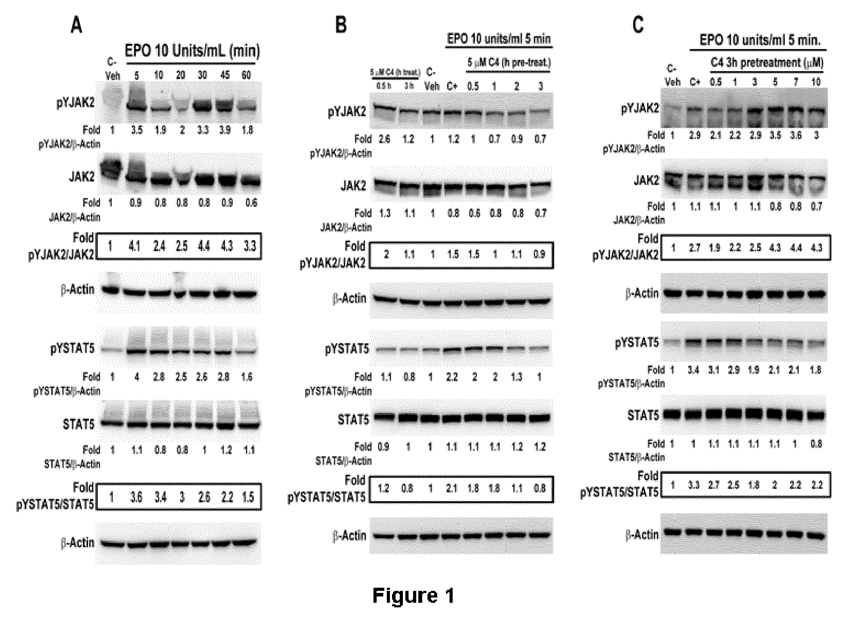

For induced experiments over night serum deprived HEL cells were induced with Epo (10 units/ml) for 5, 10, 20, 30, 45 and 60 min (Figure 1A). For dose-time experiments over night serum deprived HEL cells were pre-incubated with Compound 4 (5 µM) for 0.5, 1, 2 and 3h, and subsequently induced with Epo (10 units/ml) for 5 min (Figure 1B). For dose-response experiments, over night serum deprived HEL cultures were pre-incubated with compound 4 at increasing concentrations (0.5 - 10 µM) for 3h, and subsequently induced with Epo (10 units/ml) for 5 min (Figure 1C). In each experiment, some cells were exposed to vehicle (0.1% DMSO and PBS for Compound 4 and Epo, respectively) as a control of unspecific phosphorylation (C-Vehicle).

For total protein extraction, cells were scraped with RIPA 1X lysis buffer (25 mM Tris-HCl pH 7.6, 150 mM NaCl, 1% NP-40, 1% sodium deoxycholate, 0.1% SDS, no. 89900, Thermo Scientific, Rockford, IL, USA) supplemented with 1X protease and 1X phosphatase inhibitor cocktails (100X Halt™ Protease Inhibitor Cocktail, no. 87786 and 100X Halt™ Phosphatase Inhibitor Cocktail, no. 78420, both from Thermo Scientific, Rockford, IL, USA). An aliquot of each extract was preserved for protein quantification by bicinchoninic acid assay (Pierce® BCA Protein Assay Kit, no. 23225, Thermo Scientific, Rockford, IL, USA).

Proteins were solubilized in sample buffer containing 0.0625 M Tris-HCl, pH 6.8, 2.3% (wt/vol) SDS, 10% (vol/vol) glycerol, 5% (vol/vol) β-mercaptoethanol, and 0.001% (wt/vol) bromophenol blue and boiled at 95°C for 5 min. Equal amounts (80-100 µg) of each sample were electrophoresed on 8-10% sodium dodecyl sulfate - polyacrylamide gel electrophoresis (SDS-PAGE) and transferred to nitrocellulose membranes (iBlot® Gel Transfer Stacks, Nitrocellulose, no. IB4010-02, Invitrogen, Barcelona, Spain) as described previously in B. Guerra, M. Diaz, R. Alonso, R. Marin, J Neurochem 91, 99, 2004. The nitrocellulose membranes were then removed from the iBlot® Dry Gel Transfer Device (no. IB1001EU, Invitrogen, Barcelona, Spain) and placed in a Ponceau S staining solution (0.5% Ponceau S and 1% glacial acetic acid in water) to verify equal loading of protein in the control and treated samples. After the membranes were stained for 5 min, they were rinsed for 2 min and examined. Equal loading of protein was verified, and the membranes were rinsed for an additional 10 min and immunoscreened. Membranes were blocked with 5% blotting grade blocker nonfat dry milk in Tris Buffered Saline with 0.05% Tween 20 (TBST; blotto-blocking buffer) for at least 1h at room temperature. They were washed thrice in TBS with 0.1% Tween 20 (TBST-0.1). Next, the membranes were incubated over night at 4°C with the appropriate anti-phospho-antibodies (described below) all diluted (1:1000) in 1% bovine serum albumin (BSA)-1% blotto in TBST (BSA-blotto blocking buffer). Antibody-specific labeling was revealed by incubation with a HRP-conjugated goat anti-mouse secondary antibody (1:5000) (no. sc-2031, Santa Cruz Biotechnology, Santa Cruz, CA, USA) or a HRP-conjugated goat anti-rabbit secondary antibody (1:5000) (no. sc-2030, Santa Cruz Biotechnology, Santa Cruz, CA, USA) and visualized with the Immu-Star™ WesternC™ kit (Bio-Rad Laboratories, Hercules, CA, USA). Membranes were stripped of bound antibodies by incubation in stripping buffer (Restore™ Western Blot Stripping Buffer, no. 21059, Thermo Scientific, Rockford, IL, USA) at room temperature for 30min with agitation. Membranes were washed for 3 x 10min in TBST-0.1, blocked with blotto-blocking buffer for at least 1h and re-probed with the corresponding anti-total kinase antibodies (described below) all diluted (1:1000) in blotto-blocking buffer. To check for differences in loading and transfer efficiency across membranes, an antibody directed against β-actin (monoclonal mouse anti-β-actin antibody, no. sc-81178, Santa Cruz Biotechnology, Santa Cruz, CA, USA) was used to hybridize with all membranes previously incubated with the respective phospho and total kinase antibody. Specific bands were visualized using the ChemiDoc XRS System (Bio-Rad Laboratories, Hercules, CA, USA) and analyzed with the image analysis program Quantity One (Bio-Rad Laboratories, Hercules, CA, USA). For immunosignal quantification, band densities were normalized to basal values obtained in vehicle-treated samples. The densitometry analysis was carried out immediately before saturation of immunosignal. Results were normalized by dividing the numerical value of each sample signal by the numerical value of the signal from the corresponding value of the β-actin that served as a control. See S. Verstovsek et al., Clin Cancer Res 14, 788, Feb 1, 2008 for more detail.

The following anti-human specific antibodies were used to detect the phosphorylated form of the specified kinases: monoclonal rabbit anti-phospho-Tyr^{1007/1008}-Jak2 antibody (no. 3776, Cell Signaling Technology, Danvers, MA) and polyclonal rabbit-anti-phospho-Tyr⁶⁹⁴-Stat5 antibody (no. 9351 Cell Signaling Technology, Danvers, MA).

The following anti-human specific antibodies were used to detect the total form of the specified kinases: monoclonal rabbit anti-Jak2 antibody (no. 3230, Cell Signaling Technology, Danvers, MA) and polyclonal rabbit anti-Stat5 antibody (no. sc-836, Santa Cruz Biotechnology, Santa Cruz, CA, USA.

### Results

Compound 4 inhibits, in a time/dose dependent manner (Figure 1B and 1C), Epo-induced phosphorylation of STAT5.

### 3.1.5 Inhibition of interleukin-6 (IL-6) induced STAT3 phosphorylation by compound 4 in T47D cells.

### Introduction

Signal transducer and activator of transcription 3 (STAT3) is one of the primary intracellular targets activated after exposure to IL-6 (see P. C. Heinrich, I. Behrmann, G. Muller-Newen, F. Schaper, L. Graeve, The Biochemical journal 334 (Pt 2), 297, Sep 1, 1998). STAT3 activation has been related to enhanced cancer cell growth, survival and immune evasion, and it has been described in nearly 70% of solid (such as breast cancer) and hematological tumors. For more information about STAT3 and cancer see H. Yu, M. Kortylewski, D. Pardoll, Nat Rev Immunol 7, 41, 2007 and M. V. Fagard R, Souissi I, Baran-Marszak F., JAK-STAT 2:e22882, 2013. Upon binding of IL-6 to the heterodimeric IL-6 receptor, recruitment and activation of downstream signaling partners result in phosphorylation of STAT3 on tyrosine residue 705 (pY⁷⁰⁵). Once phosphorylated, STAT3 forms homodimers, which enter the nucleus and activate multiple pro-growth and pro-survival genes.

### Experimental conditions

T47D cells were routinely maintained in 75-cm² culture flasks with vented caps at 37°C, 5% CO₂ in RPMI 1640 growth media without phenol red supplemented with 10% FBS, 2mM L-glutamine, 10mM HEPES, 50U/ml penicillin/50U/ml streptomycin, and 0.3 mg/ml gentamicin. Two days prior the assays, cells were placed in withdrawal media (to remove all estrogens from the culture) consisting of RPMI supplemented with 10% dextran-coated charcoal-stripped FBS (DCC-FBS RPMI), 2mM L-glutamine, 10mM HEPES, 50U/ml penicillin/50U/ml streptomycin, and 0.3 mg/ml gentamicin. After 24h incubation in low estrogen media, T47D cells were serum deprived over night in 0.5% DCC-FBS RPMI. Cells were treated with vehicle, IL-6, compound 4 (C4) or both, the following day. Cell culture reagents were all obtained from BioWhittaker® (Lonza, Verviers, Belgium).

For induced experiments overnight serum-deprived T47D cells were induced with IL-6 (25 ng/ml) for 3, 5, 10, 20, 30, 45 and 60 min (Figure 2A). For dose-time experiments serum deprived T47D cells were pre-incubated with 5 µM Compound 4 for 0.5, 1, 2 and 3h or 5 µM WP1066 for 3h, and subsequently induced with IL-6 (25 ng/ml) for 30 min (Figure 2B). For dose-response experiments, overnight serum deprived T47D cultures were pre-incubated with compound 4 at increasing concentrations (0.5 - 10 µM) for 1h, and subsequently induced with IL-6 (25 ng/ml) for 30 min (Figure 2C). In each experiment, some cells were exposed to vehicle (0.1% DMSO and PBS for Compound 4 and Il-6, respectively) as a control of unspecific phosphorylation (C- Vehicle).

Total protein extraction and Western immunoblotting procedures were performed as described above.

The following anti-human specific antibodies were used to detect the phosphorylated form of the specified kinases: monoclonal rabbit anti-phospho-Tyr^{1007/1008}-Jak2 antibody (no. 3776, Cell Signaling Technology, Danvers, MA) and polyclonal rabbit anti-phospho-Tyr⁷⁰⁵-Stat3 antibody (no. 9145, Cell Signaling Technology, Danvers, MA).

The following anti-human specific antibodies were used to detect the total form of the specified kinases: monoclonal rabbit anti-Jak2 antibody (no. 3230, Cell Signaling Technology, Danvers, MA) and monoclonal mouse anti-Stat3 antibody (no. 9139, Cell Signaling Technology, Danvers, MA).

### Results

IL-6 (25 ng/ml) stimulates JAK2 and STAT3 phosphorylation in overnight-serum deprived T47D cells with a maximal induction observed after 30 min of treatment (Figure 2A). Compound 4 (C4) inhibits, in a time/dose dependent manner (Figures 2B and 2C), the IL-6-induced STAT3 phosphorylation.

### 3.1.6 Induction of apoptosis in HEL cells by compound 4.

### Introduction

Human erythroleukemia (HEL) cells, at a density of 1.5 x 10⁵ cells / ml, were treated with compound 4 at the indicated concentrations for 24 or 48 hours. The cells were incubated with propidium iodide and annexin-V-FITC and the quantity of phosphatidylserine externalized was determined using flow cytometry. Using this methodology one can determine the quantity of a) Viable cells: they do not bind annexin V and exclude propidium iodide, b) early apoptotic cells: they bind annexin V and exclude propidium iodide, c) late apoptotic cells: they bind annexin V and incorporate iodide propidium d) necrotic cells: they bind annexin V and propidium iodide incorporated or incorporated only propidium iodide.

### Result

Compound 4 induced, in a dose and time dependent manner, apoptosis of HEL cells (Figure 3).

### 3.1.7. In vivo efficacy study: Effect of compound 4 on tumor xenografs induced in mice using K562 cells.

### Introduction

K-562 is an erythroleukemia cell line derived from a chronic myeloid leukemia patient in blast crisis. These cell contain the BCR/ABL chimeric oncogene, a constitutively active tyrosine kinase, which is generated from the Philadelphia chromosome translocation (t(9;22)(q34;q11)). Studies have shown that in growing cultures of K562 cells, both STAT3 and STAT5 are constitutively activated and that STAT5 in particular is necessary for their growth in soft agar. Furthermore treatment of these cells with the new specific JAK3 inhibtor WHI-P131 (30-100 µM) caused a reduction in tyrosine phosphorylation of STAT5. It also caused a dose-dependent decrease in G1 and S phases and an increase in G2/M phases in flow cytometry experiments. These studies indicate that STAT5 plays a significant role in the proliferation of K562 leukemia cells.

### Experimental conditions

6 weeks old female athymic mice (NOD-SCID), were purchased from Charles River, and were housed in a specifically designed pathogen-free isolation facility. The mice were subcutaneously inoculated into the right flank with 2.5x10⁶ K562 cells in 100 µL of RPMI-1640 medium and 100 µL of Matrigel. When tumours became palpable, mice were randomized into three groups (n = 8 per group); the control group (receiving vehicle alone- DMSO: PEG-400: sterile water at 5:25:70 (w/v)), the group receiving compound 4 10 mg/Kg or the group receiving the positive control Imatinib 40 mg/kg. The vehicle and compound 4 treatments were administered *via* intraperitoneal injection (i.p.) every day except Saturdays and Sundays. While Imatinib was administered orally using the same schedule. The study was conducted over a 25 day period beginning on a Monday. Caliper measurements of the tumor diameters were performed twice per week, and the tumor volume was estimated as the volume of an ellipse using the following formula: V= 4/3π x (a/2) x (b/2)²; Where *a* and b correspond to the longest and shortest diameter, respectively. Mice were sacrificed when their tumor diameters reached 2 cm, or when they became moribund. Statistical analysis of the tumor volume was done using the Mann-Whitney test.

### Results

As shown in Figure 4 the volume of the tumors established in the animals in the control group (administration of vehicle alone) increased in a consistent manner throughout the 25 day study period. As expected the administration of the positive control compound Imatinib (40 mg/kg, orally) produced a pronounced and statistically significant reduction (P = 0.039 day 4 and P = 0.008 day 25, compared to control group) in tumor growth. Administration of compound 4(10 mg/kg, i.p) also produced a pronounced, and statistically significant reduction, in tumor growth (P = 0.004 day 4 and P = 0.007 day 25 compared to control group).

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein, including the priority document (European Application No. EP 12177591), are hereby referred to in their entirety for all purposes.

## Claims

1. A compound having the formula (Ia): 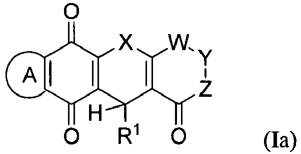 or a pharmaceutically acceptable salt thereof, wherein:
(i) Ring A is independently selected from the group consisting of substituted or unsubstituted (C₃-C₈)cycloalkyl, substituted or unsubstituted aryl, substituted or unsubstituted heteroaryl, and substituted or unsubstituted heterocyclyl having up to 3 heteroatoms selected from N, O, S, SO and SO₂;
(ii) X is independently selected from the group consisting of O, NR³, S and SO₂; wherein
R³ is independently selected from the group consisting of H, CN, SO₂R⁴, C(=O)OR⁴, C(=O)R⁴, C(=O)NR⁵R⁶, C(=NR⁷)NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂, wherein;
R⁴ is independently selected from the group consisting of substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂;
R⁵ and R⁶ are independently selected from the group consisting of H, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂;
or R⁵ and R⁶, when both connected to the same N, form a 4-, 5-, 6- or 7-membered heterocycloalkyl group or heteroaryl group, each optionally substituted with 0, 1, 2 or 3 substitutents independently selected from OH, CN, halogen, CF₃, (C₁-C₆)alkyl, (C₂-C₆)alkenyl, (C₂-C₆)alkynyl, (C₁-C₆)alkoxy, (C₃-C₈)cycloalkyl, aryl or heteroaryl;
R⁷ is independently selected from the group consisting of H, CN, substituted or unsubstituted (C₁-C₆)alkyl, substituted or unsubstituted (C₁-C₆)alkoxy, substituted or unsubstituted (C₃-C₈)cycloalkyl, substituted or unsubstituted aryl, substituted or unsubstituted heteroaryl;
(iii) W and Z are independently selected from the group consisting of (CR⁸R⁹)ₙ, O and NR³;
(iv) Y is independently selected from the group consisting of (CR⁸R⁹)ₙ and C(=O), wherein;
n is an integer from 0 to 2; and
R⁸ and R⁹ are independently selected from the group consisting of H, CN, substituted or unsubstituted (C₁-C₆)alkyl, substituted or unsubstituted (C₂-C₆)alkenyl, substituted or unsubstituted (C₂-C₆)alkynyl, substituted or unsubstituted C₁-C₆alkoxy, substituted or unsubstituted (C₃-C₈)cycloalkyl, substituted or unsubstituted aryl, substituted or unsubstituted heteroaryl;
(v) R¹ is independently selected from the group consisting of unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted aryl, substituted or unsubstituted heteroaryl, substituted or unsubstituted heterocyclyl having up to 3 heteroatoms selected from N, O, S, SO and SO₂;
with the provisos that when ring A is unsubstituted phenyl, X = O, W and Z = CH₂, and Y = C(CH₃)₂ then R¹ is other than phenyl, 4-nitrophenyl, 4-chlorophenyl, 3-chlorophenyl, 3-bromophenyl, 3-nitrophenyl, 2-chlorophenyl, 3-fluorophenyl, benzo[d][1,3]dioxol-5-yl, 3,4-dimethylphenyl, 2-bromophenyl, 4-fluorophenyl, 3-hydroxyphenyl, 2,3 dichlorophenyl, 4-bromophenyl, 4-methylphenyl, 3-methoxyphenyl or 3,4-dimethoxyphenyl, and
when ring A is unsubstituted phenyl, X = O, and W, Y and Z = CH₂, then R¹ is other than benzo[d][1,3]dioxol-5-yl, 4-fluorophenyl, 3-hydroxyphenyl, 4-chlorophenyl, 2-methoxyphenyl, 3 methoxyphenyl or 2-thienyl.

2. A compound according to any of the preceding claims in which ring A is a member selected from:
- a substituted or unsubstituted aryl, preferably a substituted or unsubstituted phenyl;
- a substituted or unsubstituted heteroaryl, preferably a substituted or unsubstituted 5- or 6-membered heteroaryl, more preferably a substituted or unsubstituted 6-membered heteroaryl; or
- a 5-, 6- or 7-membered substituted or unsubstituted heterocyclyl having up to 3 heteroatoms selected from N, O, S, SO and SO₂;

3. The compound according to any of the preceding claims having the formula (Ib): 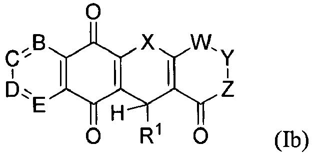 wherein:
(a) B, C, D and E are independently selected from N or CR¹⁰, with the proviso that B, C, D and E cannot all be N
wherein R¹⁰ is independently selected from the group consisting of H, CN, NO₂, C(=O)OR⁴, COR⁴, CONR⁵R⁶, NR⁵COR⁴, NR⁵CONR⁵R⁶, C(=NR⁷)NR⁵R⁶, SO₂R⁴, SO₂NR⁵R⁶, halogen, CF₃, OR⁵, SR⁵, NR⁵R⁶, substituted or unsubstituted (C₁-C₆)alkyl, substituted or unsubstituted (C₂-C₆)alkenyl, substituted or unsubstituted (C₂-C₆)alkynyl, substituted or unsubstituted (C₁-C₆)alkoxy, substituted or unsubstituted (C₃-C₇)cycloalkyl, substituted or unsubstituted aryl, substituted or unsubstituted heteroaryl, substituted or unsubstituted heterocyclyl having up to 3 heteroatoms selected from N, O, S, SO and SO₂;
(b) W, X, Y, Z, R¹, R³, R⁴, R⁵, R⁶, R⁷, R⁸, R⁹ and n are as previously defined.

4. The compound according to claim 3, wherein:
a) B and D are CR¹⁰ and C and E are N;
b) C and E are CR¹⁰ and B and D are N;
c) B, C and E are CR¹⁰ and D is N;
d) B, D and E are CR¹⁰ and C is N; or
e) E, C and D are CR¹⁰ and B is N.

5. The compound according to any of the preceding claims, wherein;
- X is independently selected from the group consisting of O or NR³, preferably independently selected from the group consisting of O or NH; and
- W, Y and Z are independently selected from the group consisting of (CR⁸R⁹)ₙ.

6. A composition comprising a compound according to any of claims 1 to 5 and at least one pharmaceutically acceptable carrier.

7. A compound having the formula (Ia) according to any of claims 1 to 5 or a composition according to claim 6, for use as medicament.

8. A compound or a composition according to claim 7, for use as medicament in the treatment of a disease mediated by a JAK, a STAT or a JAK-STAT pathway.

9. The compound or the composition according to any of claims 7 or 8, for use as a medicament in the treatment of an immunological or inflammatory disease, a hyperproliferative disease or a myeloproliferative disorder (MPD).

10. Use of a compound according to any of claims 1 to 5 or of a composition according to claim 6, for the manufacture of a medicament for treatment of a disease mediated by a JAK, a STAT or a JAK-STAT pathway.

11. Use of a compound or a composition according to claim 10, for the manufacture of a medicament for treatment of an immunological or inflammatory disease, a hyperproliferative disease or a myeloproliferative disorder (MPD).

## Patentansprüche

1. Eine Verbindung der Formel (Ia): 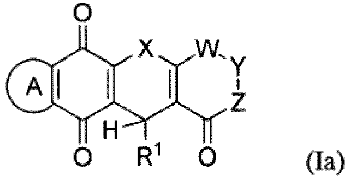 oder ein pharmazeutisch verträgliches Salz derselben, wobei:
i. Ring A unabhängig aus der Gruppe bestehend aus substituiertem oder nicht substituiertem (C₃-C₈)-Cycloalkyl, substituiertem oder nicht substituiertem Aryl, substituiertem oder nicht substituiertem Heteroaryl und substituiertem oder nicht substituiertem Heterozyclyl ausgewählt wird und bis zu 3 aus N, O, S, SO und SO₂ ausgewählte Heteroatome besitzt;
ii. X unabhängig aus der Gruppe bestehend aus O, NR³ und SO₂ ausgewählt wird, wobei
R³ unabhängig aus der Gruppe bestehend aus H, CN, SO₂R⁴, C(=O)OR⁴, C(=O)R⁴, C(O)NR⁵R⁶, C(=NR⁷)NR⁵R⁶, substituiertem oder nicht substituiertem (C₂-C₆)-Alkenyl, substituiertem oder nicht substituiertem (C₂-C₆)-Alkinyl, substituiertem oder nicht substituiertem (C₁-C₆)-Alkoxy, substituiertem oder nicht substituiertem (C₃-C₇)-Cykloalkyl, substituiertem oder nicht substituiertem Aryl, substituiertem oder nicht substituiertem Heteroaryl und substituiertem oder nicht substituiertem Heterozyclyl ausgewählt wird und bis zu 3 aus N, O, S, SO und SO₂ ausgewählte Heteroatome besitzt; wobei
R⁴ unabhängig aus der Gruppe bestehend aus substituiertem oder nicht substituiertem (C₁-C₆)-Alkyl, substituiertem oder nicht substituiertem (C₂-C₆)-Alkenyl, substituiertem oder nicht substituiertem (C₂-C₆)-Alkinyl, substituiertem oder nicht substituiertem (C₃-C₈)-Cykloalkyl, substituiertem oder nicht substituiertem Aryl, substituiertem oder nicht substituiertem Heteroaryl und substituiertem oder nicht substituiertem Heterozyclyl ausgewählt wird und bis zu 3 aus N, O, S, SO und SO₂ ausgewählte Heteroatome besitzt;
R⁵ und R⁶ unabhängig aus der Gruppe bestehend aus H, substituiertem oder nicht substituiertem (C₁-C₆)-Alkyl, substituiertem oder nicht substituiertem (C₂-C₆)-Alkenyl, substituiertem oder nicht substituiertem (C₂-C₆)-Alkinyl, substituiertem oder nicht substituiertem (C₃-C₈)-Cykloalkyl, substituiertem oder nicht substituiertem Aryl, substituiertem oder nicht substituiertem Heteroaryl und substituiertem oder nicht substituiertem Heterozyclyl ausgewählt werden und bis zu 3 aus N, O, S, SO und SO₂ ausgewählte Heteroatome besitzen;
oder R⁵ und R⁶, wenn beide mit demselben N verbunden sind und eine 4-, 5-, 6- oder 7-gliedrige Heterocycloalkylgruppe oder Heteroarylgruppe bilden, jeweils optional mit 0, 1, 2 oder 3 Substituenten ausgewählt aus OH, CN, Halogen, CF3, (C₁-C₆)-Alkyl, (C₂-C₆)-Alkenyl, (C₂-C₆)-Alkinyl, (C₁-C₆)-Alkoxy, (C₃-C₈)-Cykloalkyl, Aryl oder Heteroaryl substituiert werden;
R⁷ unabhängig aus der Gruppe bestehend aus H, CN, substituiertem oder nicht substituiertem (C₁-C₆)-Alkyl, substituiertem oder nicht substituiertem (C₁-C₆)-Alkoxy, substituiertem oder nicht substituiertem (C₃-C₈)-Cykloalkyl, substituiertem oder nicht substituiertem Aryl, substituiertem oder nicht substituiertem Heteroaryl ausgewählt wird;
iii. W und Z unabhängig aus der Gruppe bestehend aus (CR⁸R⁹)ₙ, O und NR³ ausgewählt werden;
iv. Y unabhängig aus der Gruppe bestehend aus (CR⁸R⁹)ₙ und C(=O) ausgewählt wird, wobei
N eine ganze Zahl von 0 bis 2 ist; und
R⁸ und R⁹ unabhängig aus der Gruppe bestehend aus H, CN, substituiertem oder nicht substituiertem (C₁-C₆)-Alkyl, substituiertem oder nicht substituiertem (C₂-C₆)-Alkenyl, substituiertem oder nicht substituiertem (C₂-C₆)-Alkinyl, substituiertem oder nicht substituiertem (C₃-C₈)-Cykloalkyl, substituiertem oder nicht substituiertem Aryl, substituiertem oder nicht substituiertem Heteroaryl ausgewählt werden;
v. R¹ unabhängig aus der Gruppe bestehend aus nicht substituiertem (C₁-C₆)-Alkyl, substituiertem oder nicht substituiertem (C₂-C₆)-Alkenyl, substituiertem oder nicht substituiertem (C₂-C₆)-Alkinyl, substituiertem oder nicht substituiertem (C₃-C₈)-Cykloalkyl, substituiertem oder nicht substituiertem Aryl, substituiertem oder nicht substituiertem Heteroaryl und substituiertem oder nicht substituiertem Heterozyclyl ausgewählt wird und bis zu 3 aus N, O, S, SO und SO₂ ausgewählte Heteroatome besitzt;
mit der Maßgabe, dass, falls der Ring A nicht substituiertes Phenyl, X = 0, W und Z = CH₂ und Y = C(CH₃)₂ sind, R¹ etwas anderes als Phenyl, 4-Nitrophenyl, 4-Chlorophenyl, 3-Chlorophenyl, 3-Bromophenyl, 3-Nitrophenyl, 2-Chlorophenyl, 3-Fluorophenyl, Benzo[d][1,3]dioxol-5-yl, 3,4-Dimethylphenyl, 2-Bromophenyl, 4-Fluorophenyl, 3-Hydroxyphenyl, 2,3-Dichlorophenyl, 4-Bromophenyl, 4-Methylphenyl, 3-Methoxyphenyl oder 3,-Dimethoxyphenyl ist, und dass, wenn der Ring A nicht substituiertes Phenyl, X = O und W, Y und Z = CH₂ sind, R¹ etwas anderes als Benzo[d][1,3]dioxol-5-yl, 4-Fluorophenyl, 3-Hydroxyphenyl, 4-Chlorophenyl, 2-Methoxyphenyl, 3-Methoxyphenyl oder 2-Thienyl ist.

2. Eine Verbindung nach einem der vorstehenden Ansprüche, wobei der Ring A ein aus Folgendem ausgewähltes Mitglied ist:
- ein substituiertes oder nicht substituiertes Aryl, vorzugsweise ein substituiertes oder nicht substituiertes Phenyl;
- ein substituiertes oder nicht substituiertes Heteroaryl, vorzugsweise ein substituiertes oder nicht substituiertes 5- oder 6-gliedriges Heteroaryl, am liebsten ein substituiertes oder nicht substituiertes 6-gliedriges Heteroaryl; oder
- ein 5-, 6- oder 7-gliedriges substituiertes oder nicht substituiertes Heterozyclyl, das bis zu 3 aus N, O, S. SO und SO₂ ausgewählte Heteroatome besitzt.

3. Die Verbindung nach einem der vorstehenden Ansprüche mit der Formel (Ib): 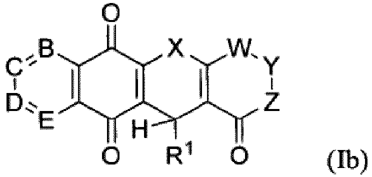 wobei:
a) B, C, D und E unabhängig aus N oder CR¹⁰ ausgewählt werden, mit der Maßgabe, dass B, C, D und E nicht alle N sein können, wobei R¹⁰ unabhängig aus der Gruppe bestehend aus H, CN, NO₂, C(=O)OR⁴, COR⁴, CONR⁵R⁶, NR⁵COR⁴, NR⁵CONR⁵R⁶, C(=NR⁷)NR⁵R⁶, SO₂R⁴, SO₂NR⁵R⁶, Halogen, CF₃, OR⁵, SR⁵, NR⁵R⁶, substituiertem oder nicht substituiertem (C₁-C₆)-Alkyl, substituiertem oder nicht substituiertem (C₂-C₆)-Alkenyl, substituiertem oder nicht substituiertem (C₂-C₆)-Alkinyl, substituiertem oder nicht substituiertem (C₁-C₆)-Alkoxy, substituiertem oder nicht substituiertem (C₃-C₇)-Cykloalkyl, substituiertem oder nicht substituiertem Aryl, substituiertem oder nicht substituiertem Heteroaryl und substituiertem oder nicht substituiertem Heterozyclyl ausgewählt wird und bis zu 3 aus N, O, S, SO und SO2 ausgewählte Heteroatome besitzt;
b) W, X, Y Z, R¹, R³, R⁴, R⁵, R⁶, R⁷, R⁸, R⁹ und n wie vorstehend definiert sind.

4. Die Verbindung nach Anspruch 3, wobei:
a) B und D = CR¹⁰ sind und C und E = N sind;
b) E und E = CR¹⁰ sind und B und D = N sind;
c) B, C und E = CR¹⁰ sind und D = N ist;
d) B,D und E = CR¹⁰ sind und C = N ist; oder
e) E, C und D = CR¹⁰ sind und B = N ist.

5. Die Verbindung nach einem der vorstehenden Ansprüche, wobei:
- X unabhängig aus der Gruppe bestehend aus O oder NR³ ausgewählt wird, vorzugsweise unabhängig aus der Gruppe bestehend aus O oder NH; und
- W, Y und Z unabhängig aus der Gruppe bestehend aus (CR⁸R⁹)ₙ ausgewählt werden.

6. Eine Zusammensetzung, welche eine Verbindung nach einem der Ansprüche 1 bis 5 und mindestens einen pharmazeutisch verträglichen Träger umfasst.

7. Eine Verbindung der Formel (Ia) nach einem der Ansprüche 1 bis 5 oder eine Zusammensetzung nach Anspruch 6, die als Medikament verwendet wird.

8. Eine Verbindung oder Zusammensetzung nach Anspruch 7 zur Verwendung als Medikament bei der Behandlung einer Krankheit, die mit einem JAK-, einem STAT- oder einem JAK-STAT-Signalweg vermittelt wird.

9. Die Verbindung oder die Zusammensetzung nach einem der Ansprüche 7 oder 8 zur Verwendung als Medikament bei der Behandlung einer immunologischen oder entzündlichen Krankheit, einer hyperproliferativen Krankheit oder einer myeloproliferativen Erkrankung (MPD).

10. Verwendung einer Verbindung nach einem der Ansprüche 1 bis 5 oder einer Zusammensetzung nach Anspruch 6 zur Herstellung eines Medikaments für die Behandlung einer Krankheit, die mit einem JAK-, einem STAT- oder einem JAK-STAT-Signalweg vermittelt wird.

11. Verwendung der Verbindung oder Zusammensetzung nach Anspruch 10 zur Herstellung eines Medikaments für die Behandlung einer immunologischen oder entzündlichen Krankheit, einer hyperproliferativen Krankheit oder einer myeloproliferativen Erkrankung (MPD).

## Revendications

1. Un Composé de formule (Ia) : 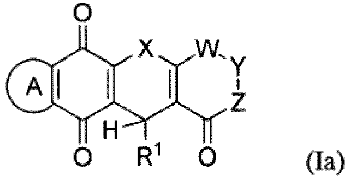 ou un de ses sels de qualité pharmaceutique, dans lequel :
(i) L'anneau A est sélectionné de manière indépendante dans le groupe composé de (C₃-C₈)cycloalkyle substitué ou non substitué, aryle substitué ou non substitué, hétéroaryle substitué ou non substitué, et hétérocyclyle substitué ou non substitué possédant jusqu'à 3 hétéroatomes sélectionnés parmi N, O, S, SO et SO₂ ;
(ii) X est sélectionné de manière indépendante dans le groupe composé de O, NR³, S et SO₂ ; dans lequel
R³ est sélectionné de manière indépendante dans le groupe composé de H, CN, SO₂R⁴, C(=O)OR⁴, C(=O)R⁴, C(=O)NR⁵R⁶, C(=NR⁷)NR⁵R⁶, (C₁-C₆)alkyle substitué ou non substitué, (C₂-C₆)alkényle substitué ou non substitué, (C₂-C₆)alkynyle substitué ou non substitué, (C₁-C₆)alkoxy substitué ou non substitué, (C₃-C₇)cycloalkyle substitué ou non substitué, aryle substitué ou non substitué, hétéroaryle substitué ou non substitué, hétérocyclyle substitué ou non substitué possédant jusqu'à 3 hétéroatomes sélectionnés parmi N, O, S, SO et SO₂, dans lequel ;
R⁴ est sélectionné de manière indépendante dans le groupe composé de (C₁-C₆)alkyle substitué ou non substitué, (C₂-C₆)alkényle substitué ou non substitué, (C₂-C₆)alkynyle substitué ou non substitué, (C₃-C₈)cycloalkyle substitué ou non substitué, aryle substitué ou non substitué, hétéroaryle substitué ou non substitué, hétérocyclyle substitué ou non substitué possédant jusqu'à 3 hétéroatomes sélectionnés parmi N, O, S, SO et SO₂ ;
R⁵ et R⁶ sont sélectionnés de manière indépendante dans le groupe composé de H, (C₁-C₆)alkyle substitué ou non substitué, (C₂-C₆)alkényle substitué ou non substitué, (C₂-C₆)alkynyle substitué ou non substitué, (C₃-C₈)cycloalkyle substitué ou non substitué, aryle substitué ou non substitué, hétéroaryle substitué ou non substitué, hétérocyclyle substitué ou non substitué possédant jusqu'à 3 hétéroatomes sélectionnés parmi N, O, S, SO et SO₂ ;
ou R⁵ et R⁶, lorsqu'ils sont tous deux liés au même N, forment un groupe hétérocycloalkyle ou un groupe hétéroaryle à 4, 5, 6 ou 7-chaînons, chacun éventuellement substitué par 0, 1, 2 ou 3 substituants sélectionnés de manière indépendante parmi OH, CN, halogène, CF₃, (C₁-C₆)alkyle, (C₂-C₆)alkényle, (C₂-C₆)alkynyle, (C₁-C₆)alkoxy, (C₃-C₈)cycloalkyle, aryle ou hétéroaryle ;
R⁷ est sélectionné de manière indépendante dans le groupe composé de H, CN, (C₁-C₆)alkyle substitué ou non substitué, (C₁-C₆)alkoxy substitué ou non substitué, (C₃-C₈)cycloalkyle substitué ou non substitué, aryle substitué ou non substitué, hétéroaryle substitué ou non substitué ;
(iii) W et Z sont sélectionnés de manière indépendante dans le groupe composé de (CR⁸R⁹)n, O et NR³ ;
(iv) Y est sélectionné de manière indépendante dans le groupe composé de (CR⁸R⁹)n et C(=O), dans lequel ;
n est un nombre entier de 0 à 2 ; et
R⁸ et R⁹ sont sélectionnés de manière indépendante dans le groupe composé de H, CN, (C₁-C₆)alkyle substitué ou non substitué, (C₂-C₆)alkényle substitué ou non substitué, (C₂-C₆)alkynyle substitué ou non substitué, (C₁-C₆)alkoxy substitué ou non substitué, (C₃-C₈)cycloalkyle substitué ou non substitué, aryle substitué ou non substitué, hétéroaryle substitué ou non substitué ;
(v) R¹ est sélectionné de manière indépendante dans le groupe composé de (C₁-C₆)alkyle non substitué, (C₂-C₆)alkényle substitué ou non substitué, (C₂-C₆)alkynyle substitué ou non substitué, (C₃-C₈)cycloalkyle substitué ou non substitué, aryle substitué ou non substitué, hétéroaryle substitué ou non substitué, hétérocyclyle substitué ou non substitué possédant jusqu'à 3 hétéroatomes sélectionnés parmi N, O, S, SO et SO₂ ;
à condition que lorsque l'anneau A est un phényle non substitué, X = O, W et Z = CH₂, et Y = C(CH₃)₂ alors R¹ est autre que phényle, 4-nitrophényle, 4-chlorophényle, 3-chlorophényle, 3-bromophényle, 3-nitophényle, 2-chlorophényle, 3-fluorophényle, benzo[d][1,3]dioxol-5-yl, 3,4-diméthylphényle, 2-bromophényle, 4-fluorophényle, 3-hydroxyphényle, 2,3 dichlorophényle, 4-bromophényle, 4-méthylphényle, 3-méthoxyphényle ou 3,4-diméthoxyphényle, et
lorsque l'anneau A est un phényle non substitué, X = O, et W, Y et Z = CH₂, alors R¹ est autre que benzo[d][1,3]dioxol-5-yl, 4-fluorophényle, 3-hydroxyphényle, 4-chlorophényle, 2-méthoxyphényle, 3-méthoxyphényle ou 2-thionyle.

2. Un composé selon n'importe laquelle des revendications précédentes dans lequel l'anneau A est un membre sélectionné parmi :
- un aryle substitué ou non substitué, de préférence un phényle substitué ou non substitué ;
- un hétéroaryle substitué ou non substitué, de préférence un hétéroaryle à 5- ou 6- chaînons substitué ou non substitué, de préférence encore un hétéroaryle à 6- chaînons substitué ou non substitué ; ou
- un hétérocyclyle à 5-, 6- ou 7- chaînons substitué ou non substitué possédant jusqu'à 3 hétéroatomes sélectionnés parmi N, O, S, SO et SO₂ ;

3. Le composé selon n'importe laquelle des revendications précédentes de formule (Ib) : 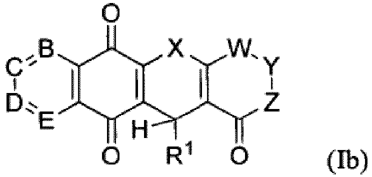 dans lequel :
(a) B, C, D et E sont sélectionnés de manière indépendante parmi N ou CR¹⁰, à condition que B, C, D et E ne puissent pas tous être N dans lequel R¹⁰ est sélectionné de manière indépendante dans le groupe composé de H, CN, NO₂, C(=O)OR⁴, COR⁴, CONR⁵R⁶, NR⁵COR⁴, NR⁵CONR⁵R⁶, C(=NR⁷)NR⁵R⁶, SO₂R⁴, SO₂NR⁵R⁶, halogène, CF₃, OR⁵, SR⁵, NR⁵R⁶, (C₁-C₆)alkyle substitué ou non substitué, (C₂-C₆)alkényle substitué ou non substitué, (C₂-C₆)alkynyle substitué ou non substitué, (C₁-C₆)alkoxy substitué ou non substitué, (C₃-C₇)cycloalkyle substitué ou non substitué, aryle substitué ou non substitué, hétéroaryle substitué ou non substitué, hétérocyclyle substitué ou non substitué possédant jusqu'à 3 hétéroatomes sélectionnés parmi N, O, S, SO et SO₂ ;
(b) W, X, Y, Z, R¹, R³, R⁴, R⁵, R⁶, R⁷, R⁸, R⁹ et n sont tels que définis précédemment.

4. Le composé selon la revendication 3, dans lequel :
(a) B et D sont CR¹⁰ et C et E sont N ;
(b) C et E sont CR¹⁰ et B et D sont N ;
(c) B, C et E sont CR¹⁰ et D est N ;
(d) B, D et E sont CR¹⁰ et C est N ; ou
(e) E, C et D sont CR¹⁰ et B est N.

5. Le composé selon n'importe laquelle des revendications précédentes dans lequel :
- X est sélectionné de manière indépendante dans le groupe composé de O ou NR³, de préférence sélectionné de manière indépendante dans le groupe composé de O ou NH ; et
- W, Y et Z sont sélectionnés de manière indépendante dans le groupe composé de (CR⁸R⁹)ₙ.

6. Une composition comprenant un composé selon n'importe laquelle des revendications 1 à 5 et au moins un vecteur de qualité pharmaceutique.

7. Un composé de formule (Ia) selon n'importe laquelle des revendications 1 à 5 ou une composition selon la revendication 6, à utiliser comme médicament.

8. Un composé ou une composition selon la revendication 7, à utiliser comme médicament dans le traitement d'une maladie dont la médiation est faite par une voie JAK, une voie STAT ou une voie JAK-STAT.

9. Le composé ou la composition selon n'importe laquelle des revendications 7 ou 8, à utiliser comme médicament dans le traitement d'une maladie immunologique ou inflammatoire, d'une maladie hyperproliférative ou d'un syndrome myéloprolifératif (MPD).

10. Utilisation d'un composé selon n'importe laquelle des revendications 1 à 5 ou d'une composition selon la revendication 6, pour la fabrication d'un médicament destiné au traitement d'une maladie dont la médiation est faite par une voie JAK, une voie STAT ou une voie JAK-STAT.

11. Utilisation d'un composé ou d'une composition selon la revendication 10, pour la fabrication d'un médicament destiné au traitement d'une maladie immunologique ou inflammatoire, d'une maladie hyperproliférative ou d'un syndrome myéloprolifératif (MPD).
